# EUROPEAN PATENT SPECIFICATION

(11) **EP 3 541 783 B1**
(45) Date of publication and mention of the grant of the patent: **27.09.2023**
(21) Application number: 17837894.9
(22) Date of filing: 17.11.2017
(51) Int. Cl.: C07C 403/02, C07C 403/10, C07C 403/20, C07C 403/24, A61K 31/07, A61P 25/00, A61P 37/00, A61P 17/00

(54) **PRECURSOR COMPOUNDS FOR PROVIDING RETINOIDS OF THE VITAMIN A5 PATHWAY AND USES THEREOF**
VORLÄUFERVERBINDUNGEN ZUR BEREITSTELLUNG VON RETINOIDEN DES VITAMIN-A5-SIGNALWEGS UND VERWENDUNGEN DAVON
COMPOSÉS PRÉCURSEURS POUR OBTENIR DES RÉTINOÏDES DE LA VOIE DE LA VITAMINE A5 ET LEURS UTILISATIONS

(30) Priority: 17.11.2016 HU 1600629; 05.05.2017 HU 1700196
(43) Date of publication of application: 25.09.2019
(73) Proprietor: University of Debrecen, 4032 Debrecen (HU); Université de Strasbourg, 67081 Strasbourg cedex (FR); Universidade de Vigo, 36310 Vigo (Pontevedra) (ES)
(72) Inventor: KREZEL, Wojciech, 67081 Strasbourg Cedex (FR); RÜHL, Ralph, 4002 Debrecen (HU); DE LERA, Angel R, 36310 Vigo Pontevedra (ES)
(74) Representative: Svingor, Adam
(86) International application number: PCT/HU2017/050047
(87) International publication number: WO 2018/091937

(56) References cited:
- WO-A1-2016/181288
- ÁNGEL R. DE LERA ET AL: "An Endogenous Mammalian Retinoid?X Receptor Ligand, At Last!", CHEMMEDCHEM, vol. 11, no. 10, 6 May 2016 (2016-05-06), pages 1027-1037, XP055462620, ISSN: 1860-7179, DOI: 10.1002/cmdc.201600105 cited in the application

## Description

### FIELD OF THE INVENTION

The invention relates to the field of retinoid X receptor (RXR) signalling and a novel vitamin A pathway called Vitamin A5 pathway. Compounds which are useful to provide (R) 9-cis-13,14-dihydro-retinoic acid an endogenous RXR ligand are claimed as well as their uses and method for preparation thereof. The compounds of the invention are useful for pharmaceutical and nutritional uses.

More particularly, precursors of 9cDHRA are among others 9-cis-13,14-dihydroretinol (9CDHROL, vitamin A5) and 9-cis-13,14-dihydro-beta carotene (9CDHBC, pro-vitamin A5), which are respectively novel types of retinoid and carotenoid RXR ligand precursors. It has been surprisingly found herein that such precursors might be directly or indirectly metabolized to 9CDHRA. The invention also relates to preventive / pharmaceutical usage of those compounds and in particular in treatment of depressive-like behaviors in chronic stress animal model of depression and other various diseases where RXR-mediated signalling is affected or was proposed as therapeutic target. Such diseases include neurodegenerative and metabolic diseases, skin- and immunological dysfunctions (including inflammation) as well as cardio-vascular diseases and life-style applications like memory enhancing effects.

### BACKGROUND ART

Vitamin A, in parallel with vitamin C and D, was among the first group of compounds that were associated to deficiency symptoms. This active lipid was later named "vitamin A". In 1931 Karrer et al. identified this fat-soluble nutrient derivative in cod liver oil (Karrer et al., 1931a; Karrer et al., 1931b). Paul Karrer, who elucidated the structure of retinol (i.e. vitamin A1) was awarded the Nobel Prize in Chemistry, for basic vitamin A research in 1937. In parallel, Edisbury et al. (Edisbury et al., 1937) and Gilliam et al. (Gillam et al., 1938) found in 1937-38 a food factor mainly present in marine fish. They used the term vitamin A2 to name this second category of vitamin A, as it displays different absorption spectra than retinol due to the presence of an additional double bound at the ring C3-C4 positions.

In the 1980's the molecular action of vitamin A was further expanded mainly by the groups of Pierre Chambon and Ronald Evans through the identification of all-trans-retinoic acid (ATRA) as the bioactive mediator of a large array of vitamin A effects. They identified ATRA as a nutrient derived lipid hormone and thereby the association of ATRA with RAR (retinoic acid receptors) for mediating transcriptional activity (Giguere et al., 1987) and the RARs themselves as new members of the nuclear hormone receptor superfamily (Petkovich et al., 1987). Besides RARs, also the retinoid-X receptors (RXRs) (Kliewer et al., 1992; Leid et al., 1992; Mangelsdorf et al., 1990; Evans 2014) identified as mediators of important function and were established as the obligatory heterodimer-binding partner for a large variety of nuclear hormone receptors. In 1992 9-cis-retinoic acid, 9CRA, was identified as the putative "endogenous" ligand for the RXRs (Heyman et al., 1992; Levin et al., 1992). In parallel the vitamin A2 derivative all-*trans*-3,4-didehydro-retinoic acid (ATDDRA; Vitamin A2-acid) was identified endogenously in humans (Vahlquist et al., 1982) and later shown to display similar activity as ATRA in activating RAR-mediated gene transcription (Torma et al., 1994). However, 9CRA, although a potent RXR ligand, has only been rigorously identified endogenously either after high pharmacological (toxicological) retinoid administration or after artificial nutritional interventions with food that is rich in vitamin A (Arnhold et al., 1996; Schmidt et al., 2002; Ulven et al., 2001) (?). In addition "vitamin A" derivatives were found in invertebrates, namely 3-hydroxyretinal ("Vitamin A3") in arthropods and 4-hydroxyretinal ("Vitamin A4") in some crustaceans (Babino et al., 2016).

Retinol (Vitamin A1) plays an essential role in vision, particularly night vision, normal bone and tooth development, reproduction, and the health of skin and mucous membranes (the mucus-secreting layer that lines body regions such as the respiratory tract). Vitamin A also acts in the body as an antioxidant, a protective chemical that may reduce the risk of certain cancers.

As to medical uses of these compounds, all-*trans*-retinoic acid (ATRA, also called Tretinoin) is the active agent in several medicinal formulations and is used, among others, in cosmetic and topical applications against e.g acne and in acute promyelocytic leukemia.

Isomer 9-cis-retinoic acid (9CRA) is also used as a medicament under the name of Alitretinoin. The oral formulation of 9CRA (Alitretinoin) is marketed under the trade name Toctino.

The primary indication for isotretinoin (the active agent being 13-cis-retinoic acid) is the treatment of severe cystic acne vulgaris, and is also indicated for the treatment of skin lesions in AIDS-related Kaposi's sarcoma.

Under the trade name Toctino the compound has been granted prescription rights in the UK for oral use in chronic hand eczema; guidance suggests, however, to prescribe it in severe cases only.

Thus, somewhat contrary to the multiple role of retinoids only a few of them are used as medicaments in a relatively few specific diseases.

All these variants present in medicaments are present in the acid form and comprise a 13,14 double bond.

The Palczewski group reported the endogenous presence 13,14-dihydroretinoids, wherein the 13,14 double bond is hydrogenated, and identified all-trans-13,14-dihydroretinoic acid (ATDHRA) as a low affinity ligand for RARs and a weaker activator than ATRA of RAR-controlled genes in cell-based assays (Moise et al., 2004; Moise et al., 2005, Moise et al., 2009).

Besides RARs, a further class of receptors, retinoid X receptors forming heterodimers with RARs play an important role in nuclear receptor signalling [D. A. Mangelsdorf, R. M. Evans, Cell 1995, 83, 841-850.]. As mentioned above 9-cis-retinoic acid (9CRA) is a potent activator of RXRs, mediators of important function and obligatory heterodimer-binding partners for a large variety of nuclear hormone receptors.

Besides 9CRA a second class of derivatives that have been found to activate RXR-mediated signaling are various fatty acids like phytanic acid (PHYA), docosahexaenoic acid (DHA) and oleic acid (de Urquiza et al., 2000; Goldstein et al., 2003; Kitareewan et al., 1996). Several findings indicate, however, that the endogenous levels of these derivatives are too low to bind RXR and elicit transcriptional activation.

Recently the endogenous presence of 9-*cis*-13,14-dihydroretinoic acid (9CDHRA) together with all-*trans* isomer has been confirmed in several organs (liver, serum, brain) from mice through a combined LC-MS-MS and UV analytical set-up and comparison with synthetic standard samples. The quantities measured were considered sufficient to maintain RXR-dependent activities. In fact, 9CDHRA was found to display biological activities similar to those of synthetic RXR agonists and coordinated the transcriptional activities of several nuclear receptor-signaling pathways, possibly through the corresponding permissive heterodimers (Riihl et al., 2015).

However, metabolic pathways leading to this compound are still unclear and further work is required to characterize this ligand and to determine its presumably multiple roles in biological systems (de Lera et al., 2016).As a summary, vitamin A research has thus far established fundamental principles for connecting diet with vitamin A and lipid hormone receptor activation and further mediated signaling resulting in regulation of various (patho)-physiological pathways. Unfortunately, the endogenous presence and nutritional relevance of the RXR ligand and especially the status of 9CRA in that regard has proven highly controversial.

The present inventors have now identified a series of compounds including members of an independent new vitamin A pathway (Vitamin A5), most notably 9-cis-13,14-dihydroretinol (9cDHROL), and which are precursors of the actual endogenous RXR ligand. They have also surprisingly found that these precursors produce an unexpectedly high increase of 9cDHRA in the brain (e.g. preferential as to the liver), and are useful vectors to target brain signaling of RXRs, whereas also demonstrated systemic production thereof. Moreover, starting from 9cDHROL extremely high levels of 9cDHRA have been generated in the tissues and among others in the brain.

These findings render the compounds of the invention good candidates both as medicaments and as nutraceuticals.

### BRIEF DESCRIPTION OF THE INVENTION

The invention relates to a compound of general formula (I) 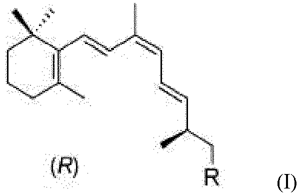 wherein R is -CH₂OR₂ wherein
R₂ is H or an acyl group -C(O)R₃ wherein R₃ is a group which is removed by hydrolysis in a mammalian tissue or organ to result in (*R*)-9-cis-13,14-dihydroretinol and a biologically acceptable tolerable compound
said compound being converted into (*R*) -9-cis-13,14-dihydroretinoic acid in a mammalian tissue or organ or cells, once administered.

Preferably, in the compound of general formula (I) 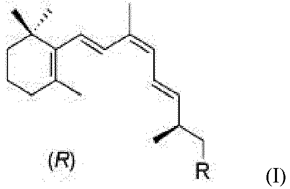
R is CH₂OR₂, wherein
R₂ is H or an acyl group C(O)R₃ wherein R₃ is a C₁₋₂₅ alkyl or a C₂₋₂₅ alkenyl,
wherein said compound is converted into 9-*cis*-13,14-dihydroretinol in mammalian tissue or organ or cells, and then into (*R*)-9-*cis*-13,14-dihydroretinoic acid in a mammalian tissue or organ, once administered.

Preferably the compound of the invention is a compound of general formula (3) 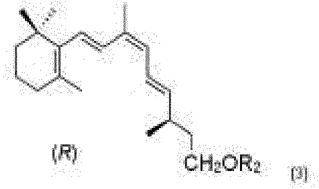 wherein R₂ is H or an acyl group C(O)R₃ wherein R₃ is selected from a
C₁₋₂₃ alkyl, preferably a C₁₋₆ alkyl and a C₉₋₂₃ alkyl, more preferably C₁₋₄ alkyl and a C₁₁₋₂₁ alkyl and a C₂₋₂₅ alkenyl, preferably a C₂₋₆ alkenyl and a C₁₋₂₃ alkenyl, more preferably a C₁₃₋₂₃ alkenyl.

In a further preferred embodiment the compound of the invention is a compound of general formula (4) 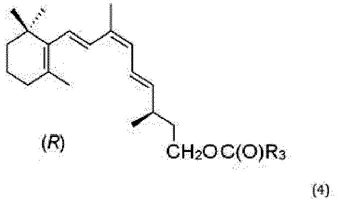 wherein R₃ is selected from
- a C₁₋₄ alkyl, preferably methyl, ethyl, propyl or isopropyl,
- a C₁₁₋₂₁ alkyl, preferably C₁₃₋₁₉ alkyl and
- a C₁₁₋₂₃ alkenyl, preferably a polyunsaturated C₁₃₋₂₃ alkenyl.

The invention also relates to a compound of general formula (I) for use in therapy in a mammalian subject 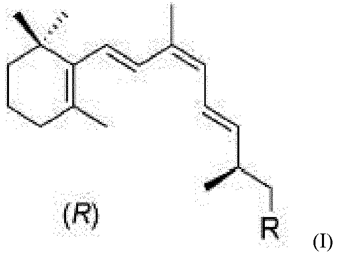
wherein R is selected from COOR₁ and CH₂OR₂ and a group of general formula (A) wherein
R₁ is a group which is removed by hydrolysis in a mammalian tissue or organ to result in (R) 9-cis-13,14-dihydroretinoate and a biologically acceptable tolerable compound and/or
R₂ is H or an acyl group C(O)R₃ wherein C(O)R₃ is a group which is removed by hydrolysis in a mammalian tissue or organ to result in (R) 9-cis-13,14-dihydro-retinol and a biologically acceptable tolerable compound, and/or
R is a group of general formula A 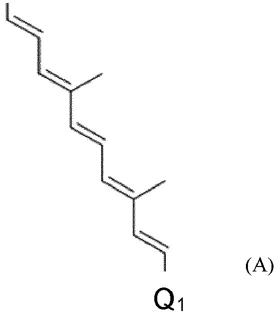
wherein Q₁ is a substituted or unsubstituted C₆₋₁₀ alkenyl or cycloalkenyl, preferably a substituted or unsubstituted trimethyl-cycloalkenyl or more preferably a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl. If the trimethylcyclohexenyl, e.g. 2,6,6-trimethylcyclohexenyl group is substituted it is preferably hydroxyl-substituted or oxosubstituted, preferably oxosubstituted;
wherein said compound is converted into 9-cis-13,14-dihydro-retinol in mammalian tissue or organ or cells, once administered.

Highly preferably the group of general formula A is a group of formula a 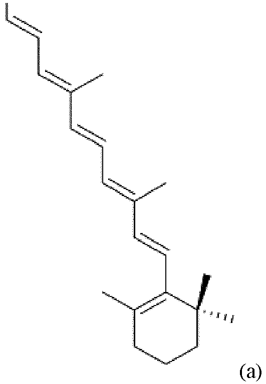 wherein said compound is a 9-cis-13,14-dihydro-beta-beta-carotene (9CDHBC).

Said compound is converted into R configuration 9-cis-13,14-dihydroretinoic acid in a mammalian tissue or organ or cells, once administered. Mammalian tissue or organ or cells is understood herein as at least one or at least one type of tissue or organ or cell culture or population of cells or multiple tissue or organ or cell culture or population of cells including the case when different steps of the conversion take place in different tissue or organ or cell types.

Preferably said compound is for use in therapy in a human subject.

Preferably said compound is for use in therapy of a disease wherein a retinoid X receptor related disease is treated.

Optionally, in particular wherein the invention relates to the compounds themselves R₁ is different from ethyl in particular in cases defined above.

The enantiomeric configuration of the compound is indicated in the formula. Preferably the compound or any composition comprising it is enriched in this enantiomer (normally indicated as (R)) or preferably enantiopure, as defined herein.

The invention also relates to a compound of general formula (I) 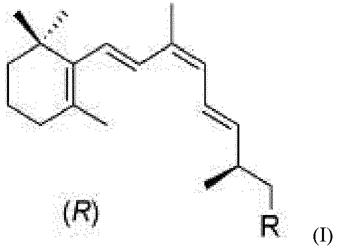
wherein R is selected from COOR₁ and CH₂OR₂ and a group of formula (A), wherein
R₁ is a C₁₋₂₅ alkyl or a C₂₋₂₅ alkenyl and/or
R₂ is H or an acyl group C(O)R₃ wherein R₃ is a C₁₋₂₅ alkyl or a C₂₋₂₅ alkenyl and/or
R is a group of general formula A 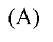 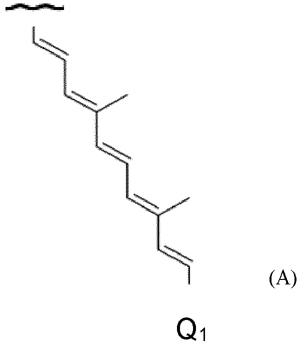
wherein Q₁ is a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl; if the trimethylcyclohexenyl, e.g. 2,6,6-trimethylcyclohexenyl group is substituted it is preferably hydroxyl-substituted or oxosubstituted, preferably oxosubstituted;
wherein said compound is converted into 9-cis-13,14-dihydro-retinol in mammalian tissue or organ or cells, once administered.

Highly preferably the group of general formula A is a group of formula a 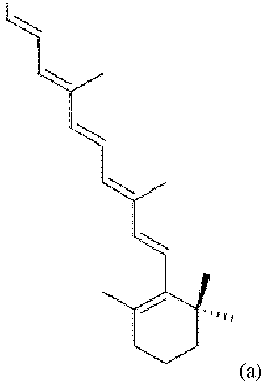 wherein said compound is a 9-cis-13,14-dihydro-beta-beta-carotene (9CDHBC).

Said compound is capable of converting into R configuration 9-cis-13,14-dihydroretinoic acid in a mammalian tissue or organ, once administered.

In a preferred embodiment in the compound of the invention of general formula I R₁ or R₂ is as defined above or the compound is a 9-cis-carotenoid compound which is a 9-cis-13,14-dihydro-beta-carotene derivative or a 9-cis-13,14-dihydro-beta-carotene, preferably a 9-cis-13,14-dihydro-beta-beta-carotene, as a precursor compound, wherein said compound is converted into R configuration 9-cis-13,14-dihydroretinoic acid in a mammalian tissue or organ or cells, once administered.

Preferred options for the length of alkyl or alkenyl chain in case of R₁ or R₂ are as defined herein.

Preferably said compound is for use in therapy, as defined herein, in a mammalian, preferably in a human subject.

Optionally, in particular wherein the invention relates to the compounds themselves R₁ is different from ethyl. The "invention relates to the compounds themselves" means that the compounds are claimed as products and are not limited by a medical or diagnostic use or purpose carried out on human or animal body. Moreover, it means that the compound is not claimed as a part of a use or a method in a use or method claim.

The enantiomeric configuration of the compound is indicated in the formula. Preferably the compound or any composition comprising it is enriched in this enantiomer (normally indicated as (R)) or preferably enantiopure, as defined herein.

In a first aspect the compound is a compound is of general formula (3) 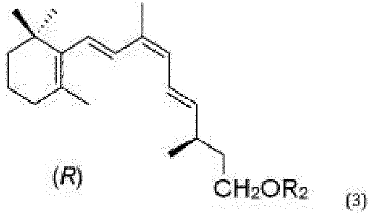
wherein R₂ is H or an acyl group C(O)R₃ wherein R₃ is selected from a
C₁₋₂₅ alkyl or a C₁₋₂₃ alkyl, preferably a C₁₋₈ alkyl or a C₁₋₆ alkyl and a C₉₋₂₃ alkyl, more preferably C₁₋₄ alkyl and a C₁₁₋₂₁ alkyl and a
C₂₋₂₅ alkenyl, preferably a C₂₋₈ alkenyl or C₂₋₆ alkenyl and a C₂₋₂₃ alkenyl, more preferably a C₁₃₋₂₃ alkenyl.

In a preferred embodiment R₃ is a C₁₋₈ alkyl, preferably a C₁₋₆ alkyl, more preferably a C₁₋₄ alkyl.

In a preferred embodiment R₃ is a C₉₋₂₃ alkyl, preferably a C₁₁₋₂₁ alkyl, more preferably a C₁₃₋₁₉ alkyl. In a preferred embodiment R₃ is C₂₋₈ alkenyl preferably C₂₋₆ alkenyl more preferably C₂₋₄ alkenyl.

In a preferred embodiment R₃ is C₉₋₂₅ alkenyl, preferably a C₁₁₋₂₃ alkenyl, more preferably a C₁₃₋₂₃ alkenyl.

In an embodiment R₂ is H and the compound is (R) (R) 9-*cis*-13,14-dihydro-retinol.

In a further embodiment R₂ is an acyl group C(O)R₃ and R₃ is a compound as defined above and C(O)R₃ is a group which is removed by hydrolysis of the ester in a mammalian tissue or organ to result in the corresponding alcohol (R) 9-cis-13,14-dihydro-retinol. Thus, the ester is converted to an alcohol and a biologically tolerable and/or acceptable compound. Said alcohol compound is converted into R configuration 9-cis-13,14-dihydroretinoic acid in a mammalian tissue or organ, once administered. This means that the compound has this capability, i.e. "is" also means "being capable of" depending on whether the compound is actually administered.

Preferably said compound is for use in therapy in a mammalian subject, preferably in a human subject.

The enantiomeric configuration of the compound is indicated in the formula. Preferably the compound or any composition comprising it is enriched in this enantiomer (normally indicated as (R)) or preferably enantiopure, as defined herein.

In a preferred embodiment of this aspect of the invention said compound is of general formula (4) 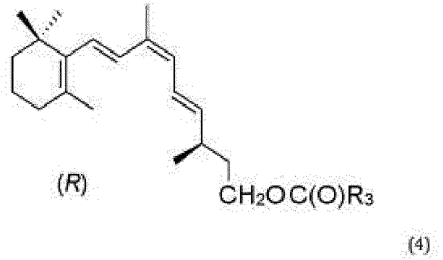 wherein R₃ is as defined in case general formula (3) or R₃ is selected from
- a C₁₋₄ alkyl, preferably methyl, ethyl, propyl or isopropyl,
- a C₁₁₋₂₁ alkyl, preferably C₁₃₋₁₉ alkyl and
- a C₁₁₋₂₃ alkenyl.

Preferably alkenyl is a polyunsaturated C₁₃₋₂₃ alkenyl.

In a preferred embodiment said compound being converted into R configuration 9-cis-13,14-dihydroretinol in a mammalian tissue or organ, once administered.

More preferably the mammalian tissue is a nervous tissue or the tissue or organ is that of the central nervous system or of the peripheral nervous system. The mammalian cells are preferably nerve cells (neurocytes), preferably or in particular oligodendrocytes.

In a further preferred embodiment the mammalian tissue is blood. In a further preferred embodiment the mammalian tissue is liver.

In a further aspect of the invention said compound is of general formula (2) 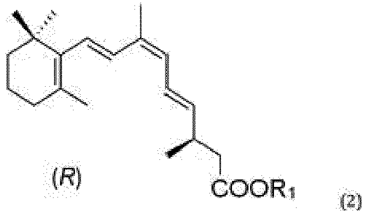 wherein R₁ is selected from a
C₁₋₂₅ alkyl or C₁₋₂₃ alkyl, preferably a C₁₋₆ alkyl and a C₉₋₂₃ alkyl, more preferably C₁₋₄ alkyl and a C₁₁₋₂₁ alkyl and a C₂₋₂₅ alkenyl or C₂₋₂₄ alkenyl, preferably a C₂₋₆ alkenyl and a C₉₋₂₃ alkenyl, more preferably a C₁₃₋₂₃ alkenyl.

Optionally, in particular wherein the invention relates to the compounds themselves R₁ is different from ethyl.

The enantiomeric configuration of the compound is indicated in the formula. Preferably the compound or any composition comprising it is enriched in this enantiomer (normally indicated as (R)) or preferably enantiopure, as defined herein.

In a preferred embodiment R₁ is selected from a C₁₋₈ alkyl and a C₉₋₂₃ alkyl, more preferably C₁₋₄ alkyl and a C₁₁₋₂₁ alkyl and a
C₂₋₂₅ alkenyl or C₂₋₂₄ alkenyl, preferably a C₂₋₆ alkenyl and a C₉₋₂₃ alkenyl, more preferably a C₁₃₋₂₃ alkenyl.

In a preferred embodiment R₁ is a C₁₋₈ alkyl, preferably a C₁₋₆ alkyl, more preferably a C₁₋₄ alkyl.

In a preferred embodiment R₁ is C₂₋₈ alkenyl preferably C₂₋₆ alkenyl more preferably C₂₋₄ alkenyl.

In a preferred embodiment R₁ is selected from methyl, ethyl, propyl or isopropyl optionally methyl, propyl or isopropyl.

In a further aspect the invention also relates to a compound of general formula (5) 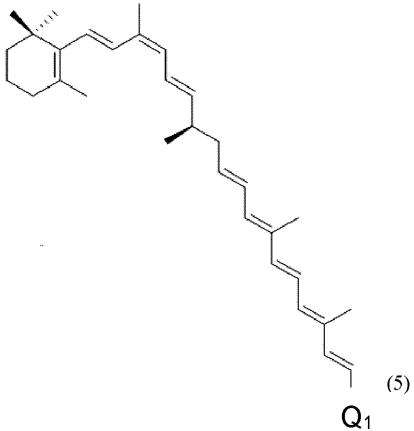
wherein Q₁ is a substituted or unsubstituted C₆₋₁₀ alkenyl or cycloalkenyl, preferably a substituted or unsubstituted trimethyl-cycloalkenyl or more preferably a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl, wherein if the trimethylcyclohexenyl, e.g. 2,6,6-trimethylcyclohexenyl group is substituted it is preferably hydroxyl-substituted or oxosubstituted, preferably oxosubstituted;
wherein said compound is converted into 9-cis-13,14-dihydro-retinol in mammalian tissue or organ or cells, once administered.

Highly preferably the group of general formula A is a group of formula a

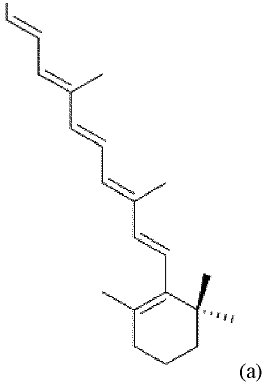

In a preferred embodiment the invention also relates to a 9-cis-carotenoid compound which is a 9-cis-13,14-dihydro-beta-carotene derivative or a 9-cis-13,14-dihydro-beta-carotene for example a 9-cis-13,14-dihydro-beta-beta-carotene or a 9-cis-13,14-dihydro-beta-alpha-carotene, preferably a 9-cis-13,14-dihydro-beta-beta-carotene, as a precursor compound, wherein said compound is converted into R configuration 9-cis-13,14-dihydroretinoic acid in a mammalian tissue or organ or cells, once administered. In particular the 9-cis-carotenoid compound is a biologically acceptable tolerable compound.

In a further aspect the invention relates to a pharmaceutical composition comprising the compound as defined above for a compound of general formula (3) said composition also comprising one or more pharmaceutically acceptable carriers, e.g. additive(s) and/or excipient(s).

Preferably, the invention relates to a pharmaceutical composition comprising the compound as defined above for a compound of general formula (4) said composition also comprising one or more pharmaceutically acceptable carriers, e.g. additive(s) and/or excipient(s).

Preferably, the invention relates to a pharmaceutical composition comprising the compound as defined above for the 9-cis-carotenoid and/or a compound of general formula (5), in particular a 9-cis-13,14-dihydro-beta-carotene as a precursor compound said composition also comprising one or more pharmaceutically acceptable carriers, e.g. additive(s) and/or excipient(s).

The invention also relates to a nutraceutical composition comprising the compound as defined above for a compound of general formula (3) or preferably for a compound of general formula (4), or of general formula (5) or the 9-cis-carotenoid compound, preferably 9-cis-13,14-dihydro-beta-carotene as a precursor compound said composition also comprising one or more nutraceutically acceptable additive(s) and/or excipient(s). Preferably, said composition being a dietary supplement, a functional food, a medical food or a food with a health claim. Preferably said nutraceutical composition comprises said compound in an amount or concentration higher than it is present in the natural food matrix and/or comprises additional amount of the compound, preferably additional isolated or artificially prepared compound.

The invention discloses a novel chemical synthesis of 9-cis-13,14-dihydroretinol and ester forms thereof via alkyl 9-cis-13,14-dihydroretinoate. The method comprises
i) reduction of alkyl 9-cis-13,14-dihydroretinoate into 9-cis-13,14-dihydroretinol and optionally
ii) alkylation of 9-cis-13,14-dihydroretinol into the corresponding ester form. The esters are exemplified hereinbelow.

In a preferred embodiment reduction is carried out by a hydride catalyst, like diisobutylaluminium hydride (DIBAL-H) reduction in an organic solvent, preferably a small heterocycle like THF.

Esterification of alcohols are preferably carried out by alkylic andhidride reagents. Preferably basic nitrogen containing single ring heterocycle is applied like pyridine and/or derivatives. In a highly preferred embodiment the reaction is carried out in the presence of dimethylaminopyridine (DMAP).

Preferred organic solvent applied are e.g. CH₂Cl₂, chloroform, CCl₄ etc.

Other esters of 9-cis-13,14-dihydroretinoic acid and 9-cis-13,14-dihydroretinol (such as palmitate and others) can be easily prepared using the same sequence.

In a further embodiment the invention also relates to a novel chemical synthesis of a compound of general formula (5) as defined above, preferably a 9-cis-carotenoid compound which is a 9-cis-13,14-dihydro-beta-carotene derivatives preferably a 9-cis-13,14-dihydro-beta-beta-carotene, said method comprising
- conversion of a compound of general formula (3) 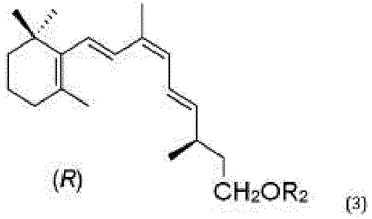 wherein R₂ is H or an OH-protecting group;
   into the corresponding aldehyde of formula (6) as an intermediate 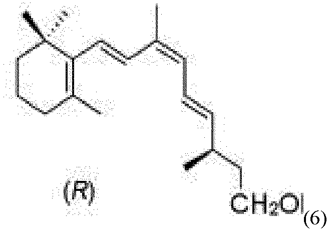
- reacting said aldehide intermediate with Wittig-reagent compound (7) 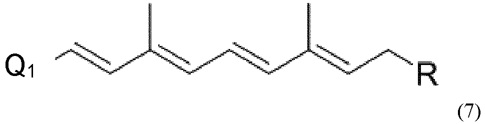
   wherein Q₁ is a substituted or unsubstituted C₆₋₁₀ alkenyl or cycloalkenyl, preferably a substituted or unsubstituted trimethyl-cycloalkenyl or more preferably a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl. If the trimethylcyclohexenyl, e.g. 2,6,6-trimethylcyclohexenyl group is substituted it is preferably hydroxyl-substituted or oxosubstituted, preferably oxosubstituted;
   and R is a group forming a phosphorous ylide, preferably a phosphonium group, preferably a triphenylphosphonium group, to give the desired compound according to formula (5).

In a preferred embodiment 9-cis-13,14-dihydro-beta-beta-carotene is prepared wherein general formula (7) is a compound of general formula (8) 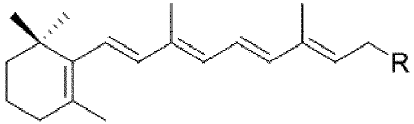 R is a group forming a phosphorous ylide, preferably a phosphonium group, preferably a triphenyl-phosphonium group, to give 9-cis-13,14-dihydro-beta-beta-carotene.

In a further aspect the invention also relates to a use of a compound as defined above for a compound of general formula (3) or preferably for a compound of general formula (4) or for a compound of general formula (5) or the 9-cis-carotenoid or the particular options as a food ingredient or food supplement. Preferably, the food ingredient is ingredient of a functional food or a medical food.

In an embodiment of the invention the compound as defined above for a compound of general formula (3) or preferably for a compound of general formula (4) or a compound of general formula (5) or the 9-cis-carotenoid or the particular options is for use in therapy in a mammalian subject.

In a preferred embodiment the compound is in the form of a pharmaceutical composition.

In a further preferred embodiment the compound is in the form of a nutraceutical composition which requires an authorization to be marketed for the indication as provided herein.

In a further aspect the invention relates to a pharmaceutical composition or use in therapy of a mammalian subject, said composition comprising the compound as defined above for general compound (2) said composition also comprising one or more pharmaceutically acceptable carriers, e.g. additive(s) and/or excipient(s).

The invention also relates to a nutraceutical composition comprising the compound as defined above for general compound (2) said composition also comprising one or more nutraceutically acceptable additive(s) and/or excipient(s). Preferably, said composition being a dietary supplement, a functional food, a medical food or a food with a health claim.

In a further aspect the invention also relates to a use of a compound as defined above for general compound (2) as a food ingredient. Preferably, the food ingredient is ingredient of a functional food or a medical food.

In an embodiment of the invention the compound as defined above for general compound (2) is for use in therapy in a mammalian subject.

In a preferred embodiment the compound is in the form of a pharmaceutical composition.

In a further preferred embodiment the compound is in the form of a nutraceutical composition which requires an authorization to be marketed for the indication as provided herein.

In a preferred embodiment the compound or the pharmaceutical composition or the nutraceutical composition is for use in the prevention and/or treatment of an RXR-mediated signaling dysfunction.

In a preferred embodiment the compound or the pharmaceutical composition or the nutraceutical composition is for use in the prevention and/or treatment of a disease related to or due to impaired retinoid X receptor signaling. Said disease preferably can be treated or prevented or alleviated by a selective. retinoid X receptor ligand.

In a preferred embodiment the compound or the pharmaceutical composition or the nutraceutical composition is for use in the prevention and/or treatment of a disease selected from central nervous system related diseases and peripheral nervous system related diseases.

In a preferred embodiment the compound or the pharmaceutical composition or the nutraceutical composition is for use in the prevention and/or treatment of mental diseases.

In a preferred embodiment the compound or the pharmaceutical composition or the nutraceutical composition is for use in the prevention and/or treatment of memory impairment or for use in enhancing memory performance, wherein preferably said memory is working memory.

In a preferred embodiment the compound or the pharmaceutical composition or the nutraceutical composition is for use in the prevention and/or treatment of impaired cognitive functions or impaired learning.

In a preferred embodiment the compound or the pharmaceutical composition or the nutraceutical composition is for use in the prevention and/or treatment of depression.

In a preferred embodiment the compound or the pharmaceutical composition or the nutraceutical composition is for use in the prevention and/or treatment of a neurodegenerative disorder.

In a preferred embodiment the compound or the pharmaceutical composition or the nutraceutical composition is for use in the prevention and/or treatment of a neurodegenerative disorder selected from Alzheimer's disease, Parkinson's disease, Mild Cognitive Impairment (MCI), Parkinson's disease with MCI, Huntington's disease, Dementia with Lewy bodies (DLB), Amyotrophic lateral sclerosis (ALS), and other neurodegenerative related dementias due to changes in the brain caused by ageing, disease or trauma; or spinal cord injury and ataxias, disseminated sclerosis and multiple sclerosis or other neurological conditions, preferably from Alzheimer's disease and Parkinson's disease.

In the above medical indication embodiments the compound is preferably selected from compounds as defined above for general compound (2), (3) or (4). In the above medical indication embodiments the compound is preferably selected from compounds as defined above for general compound (3) or preferably (4).

The invention also teaches the use of any of the above compounds in the preparation of a pharmaceutical preparation or a medicament as defined herein or above.

The invention also teaches the use of any of the above compounds in the preparation of a nutraceutical preparation, preferably a medical food or a food with a health claim as defined herein or above.

The invention also relates to the compound of the invention or a pharmaceutical composition of the invention or a nutraceutical composition for use in a method for treatment of a disease as defined above, wherein said compound or composition is administered to a mammalian subject preferably a human in need thereof in an effective dose. In a preferred embodiment administration is regular, e.g. daily administration.

The invention also relates to the compounds as defined herein for use in maintaining health, preferably as a food ingredient, a dietary supplement, e.g. as a vitamin or precursor thereof or as a nutraceutical ingredient. In a preferred embodiment the invention relates to a use to maintain health to prevent a condition as defined herein. In an embodiment said use is for maintenance of normal vision, maintenance of normal skin and mucous membranes, and maintenance of normal hair.

More preferably said use is for maintenance of normal brain function or maintenance of mental health or maintenance of mental performance or maintenance normal psychological function or cognitive function or for contribution of the functioning of the nervous system. In an embodiment these uses are defined in a health claim.

The invention also relates to the compounds or compositions of the invention for use in a method for maintaining a healthy condition of a subject as defined above wherein a compound of the invention or a nutraceutical composition of the invention is administered to a mammalian subject preferably a human in need thereof as a part of a diet. In a preferred embodiment administration is regular, e.g. daily administration.

The invention also relates to compounds according to any of general formulae (3), (4) and/or (5) as defined above for use as a dietary supplement as a vitamin A5 (9CDHROL) or precursor thereof. In a particular embodiment the precursor is 9CDHBC. In a further particular embodiment the precursor is selected from 9CDHROL-esters.

The invention also relates to compounds according to any of general formulae (3), (4) and/or (5) as defined above for use as a dietary supplement as a 9CDHRA precursor, and RXR-ligand. In a particular embodiment the 9CDHRA precursor is selected from 9CDHBC, 9CDHROL, 9CDHROL-esters and 9CDHRA-esters.

Preferably the precursors are administered in an intravenous, topical or oral route. The precursors can be administered systemically or locally.

These precursors can be given for selective metabolic conversion to 9CDHRA, the endogenous RXR-ligand for enabling RXR-mediated signaling.

In a particular embodiment the invention relates to the use of 9CDHBC to prevent, treat or ameliorate any condition as defined above. In a preferred embodiment the condition is selected from the group consisting of: (a) affective abnormalities in stress associated disorders including (but not limited to) psychiatric disorders such as depression, schizophrenia (b) memory deficits associated with dementia, and in particular Alzheimer-disease.

In a particular embodiment 9CDHBC is for use in memory improvement.

In a particular embodiment the invention relates to 9CDHROL or any ester thereof as defined herein for use in a method to prevent, treat or ameliorate any condition as defined above. In a preferred embodiment the condition is selected from the group consisting of: (a) affective abnormalities in stress associated disorders including (but not limited to) psychiatric disorders such as depression, schizophrenia (b) memory deficits associated with dementia, and in particular Alzheimer-disease.

In a particular embodiment 9CDHROL or any ester thereof is used for memory improvement.

In a particular embodiment the invention relates to 9CBC for use as a carotenoid precursof of 9CDHBC.

In a particular embodiment the invention relates to 9CBC for use as a food supplement to increase the level of 9CDHBC in the human body in a non-therapeutic application.

### BRIEF DESCRIPTION OF THE FIGURES

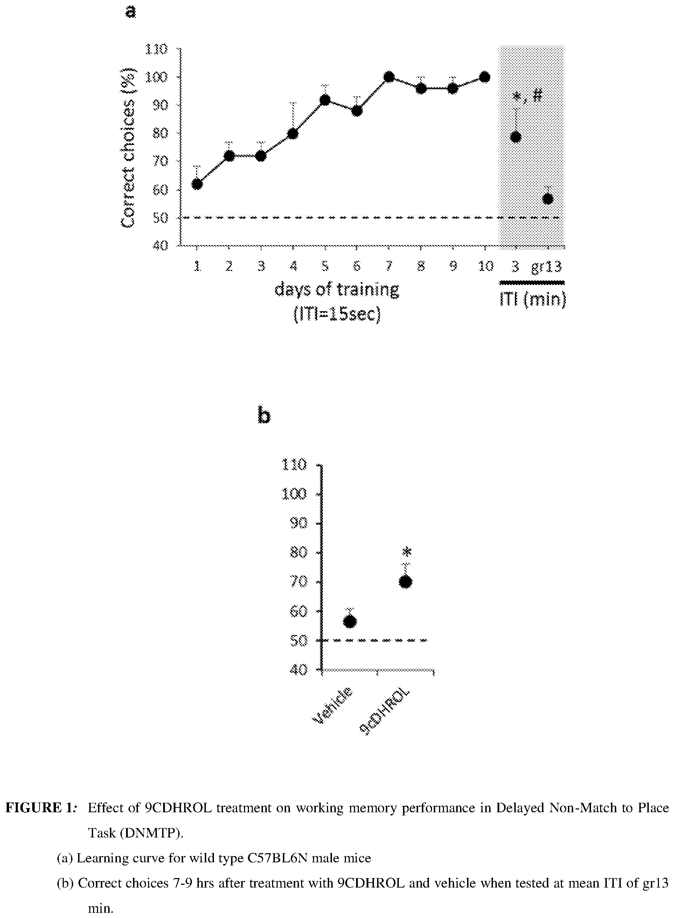

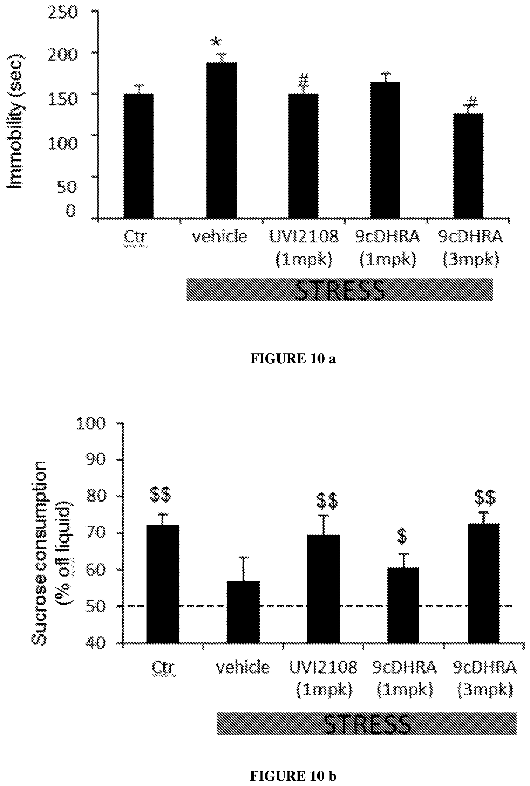

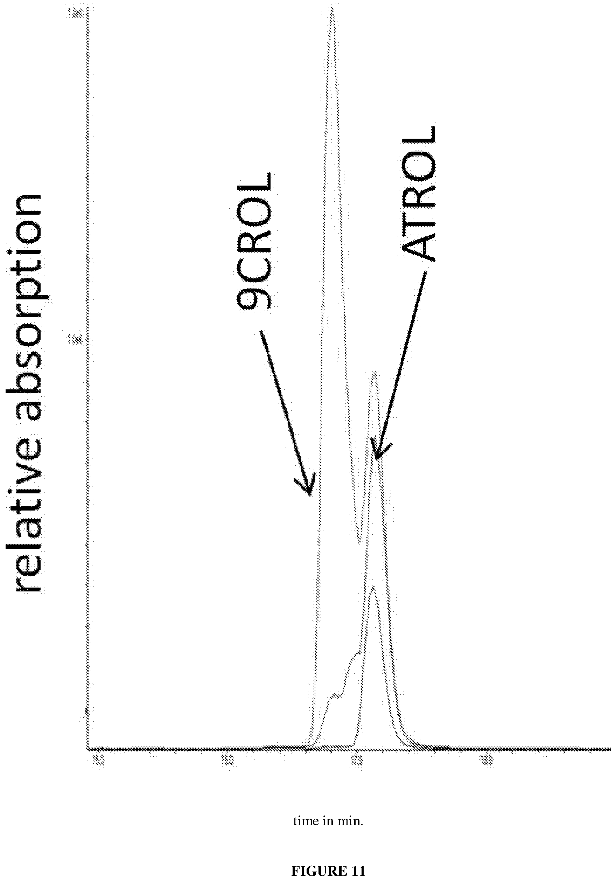

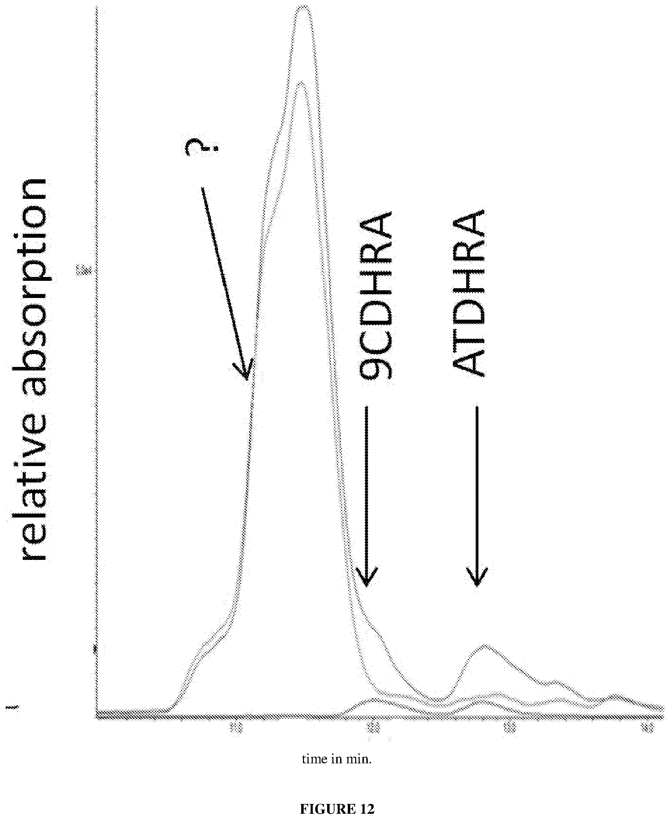

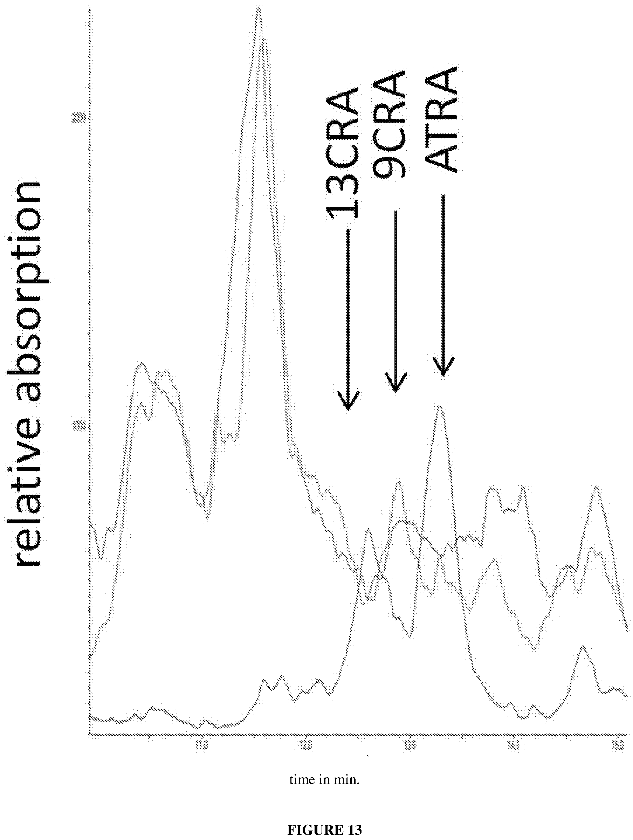

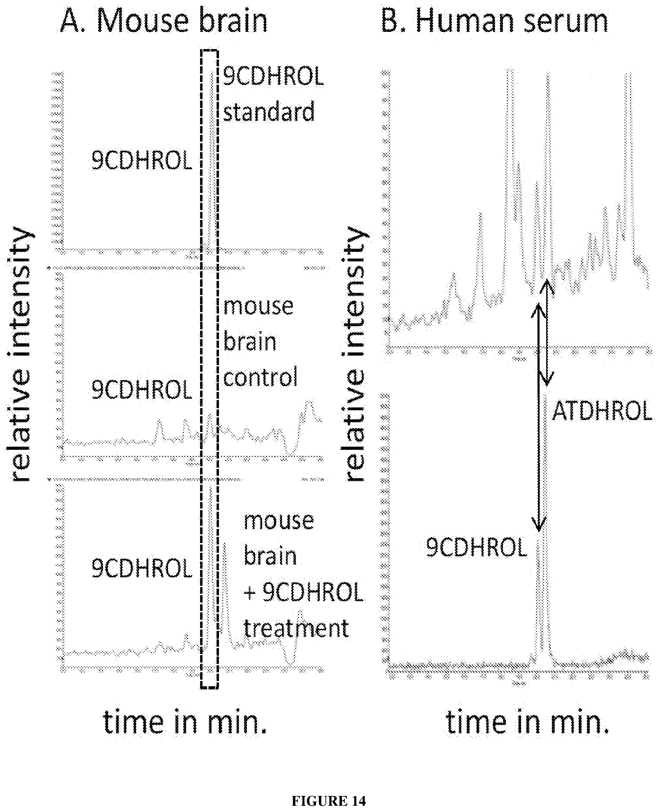

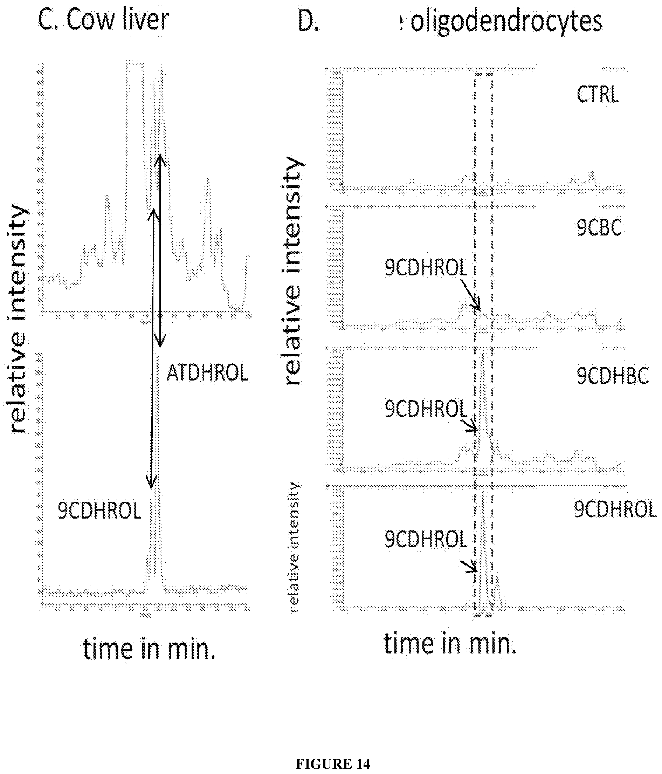

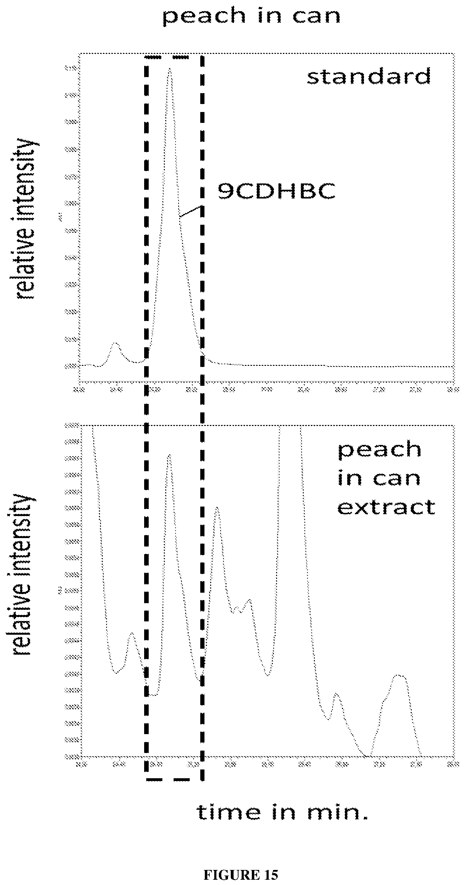

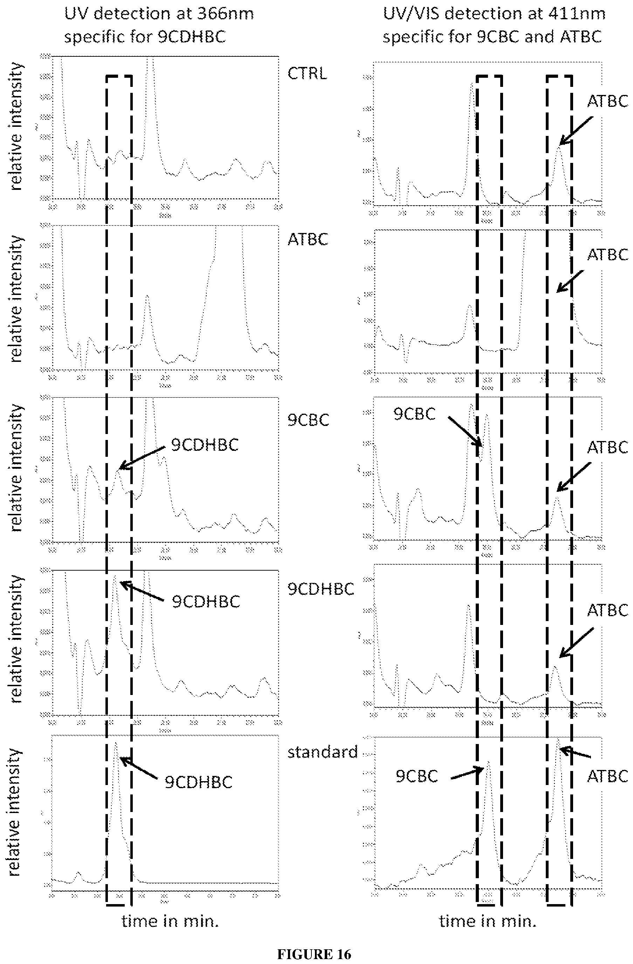

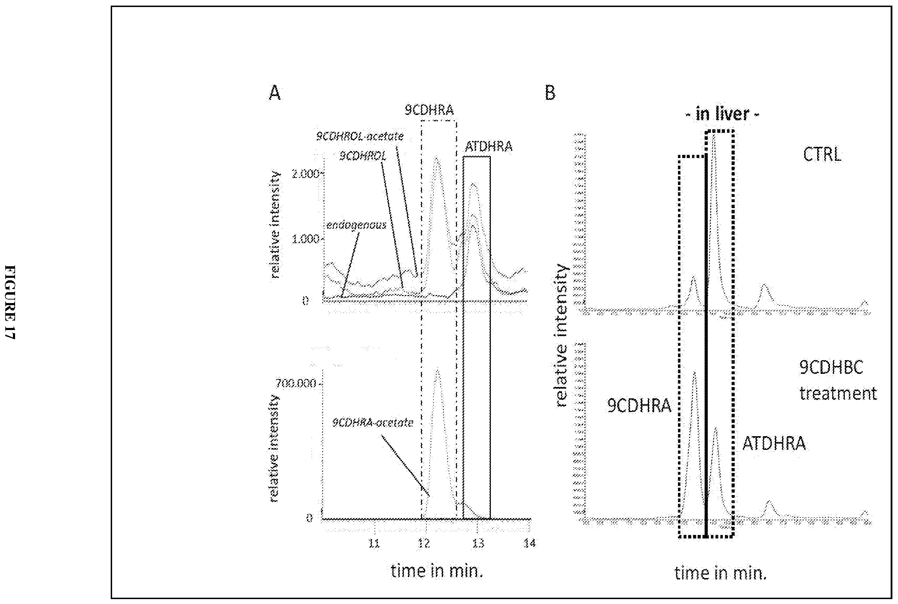

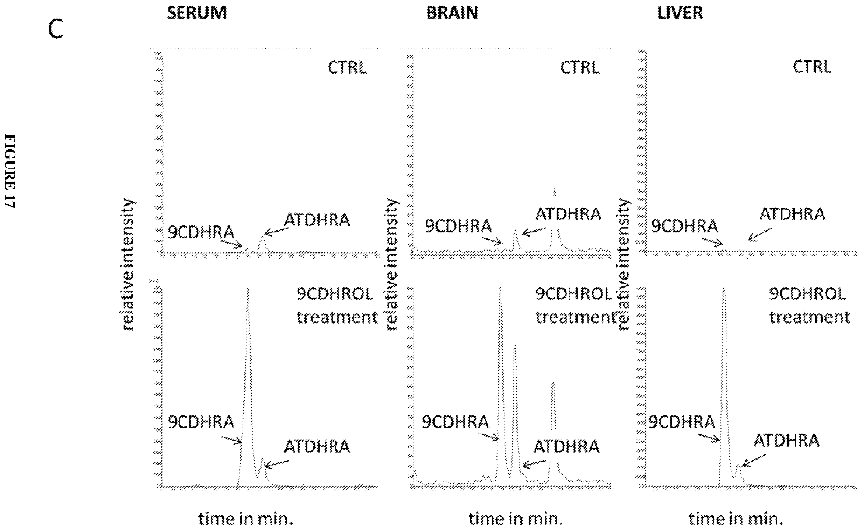

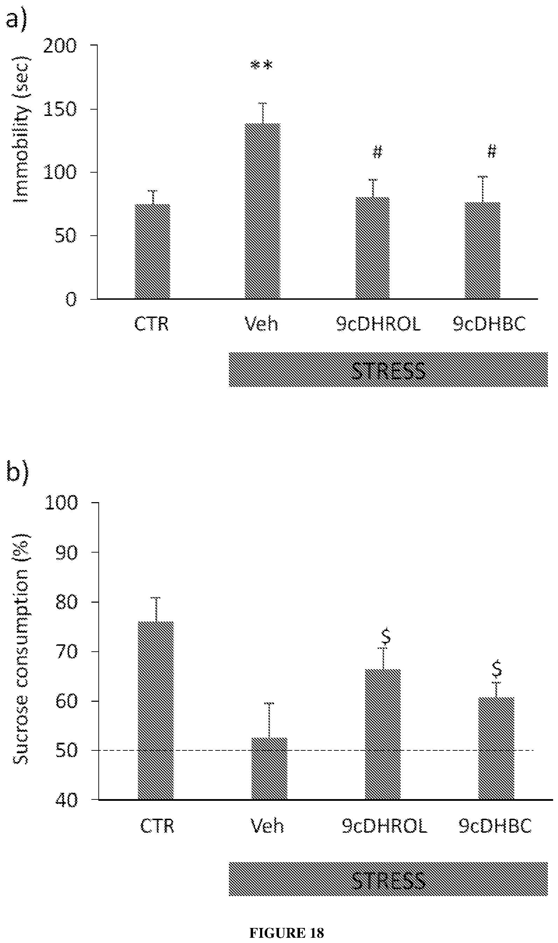

**Figure 1****:** 9CDHROL treatment improves working memory performance in Delayed Non-Match to Place Task (DNMTP). (a) an increasing learning curve for wild type C57BL6N male mice (n=5) illustrates acquisition of the working memory task. Memory performance scored for increased inter-trial intervals (ITI) is shown in grey field and corresponds to ITI of 3min and a mean ITI at which mice displayed memory deficit and which for the whole group attained mean value of 13min (indicated as gr13). (b) 9CDHROL, but not vehicle treatment improved working memory performance 7-9hrs after treatment when tested at mean ITI of gr13 min. Statistical differences identified with one or two group t-test were indicated respectively as: ^{∗}, p<0.05 when compared to 50% performance at chance level and #, p<0.05, for comparison with the performance on the last day of acquisition phase (day 10).
**Figure 2****:** 9CDHROL standard mixture (top chromatogram) and endogenous levels in the liver of mice after vehicle treatment (bottom chromatogram).
**Figure 3****:** 9CDHROL in mice after vehicle treatment representing endogenous levels (blue) and after 9CDHROL-treatment (red) in mice.
**Figure 4****:** Levels of 9CDHRA after vehicle treatment representing endogenous levels (blue) and after 9CDHROL-treatments (red) in mice.
**Figure 5****:** Levels of 9CDHRA after vehicle treatment representing endogenous levels (blue) and after 9CDHROL-treatment (red) in mouse liver. Top figure: Normal range (set on maximum); bottom figure: extended range for the maximum range of endogenous retinoids.
**Figure 6****:** 9CDHRA control-treatment (endogenous levels) and after supplementations of retinoids to oligodendrocyte cells. Up: endogenous (blue), 9CDHROL (orange) and 9CDHROL-acetate (pink) and bottom: 9CDHRA-acetate (pink)
**Figure 7****:** Summary of putative nutritional precursors of the new vitamin A5 cluster precursors. Retinoids not yet identified as endogenous retinoids are marked with blue letters. Abbreviations: 9-*cis*-13,14-dihydro-retinol (9CDHROL), 9-*cis*-13,14-dihydro-retinal (9CDHRAL) and 9-*cis*-13,14-dihydro-retinoic acid (9CDHRA), all-*trans*-13,14-dihydro-retinal (ATDHRAL), all-*trans*-13,14-dihydro-retinol (ATDHROL), all-*trans*-13,14-dihydro-retinoic acid (ATDHRA).
**Figure 8****:** Increasing doses of (*R*)-9CDHRA reversed working memory deficits in Rbp1-/- mice and showed pro-mnemonic activity in WT mice (n=8/group) in DNMTP task when tested at minimal ITI, at which mice performed at chance level (50%) and which was 6min for the Rbp1-/- or 12min for WT mice. ttt: ATRA at concentration 10-5 M was cytotoxic.^{∗}, p<0.05. #, p<0.05; ##, p<0.01 as compared to vehicle treatment in the same group; $, p<0.05; $$, p<0.01; one group student t-test for comparison with performance at chance level of 50%. All the error bars represent S.E.M.
**Figure 9****:** R-9CDHRA displays RXR agonist-like activities in vivo. Reverse of behavioural deficits after treatments with UVI2108 and R-9CDHRA.
   Increasing doses of R-9CDHRA (0.1, 1, 2 mg/kg) reduce despair behaviour in Rbp1-/- mice in the forced swim test (n=26 for vehicle groups and n=6-8 for each remaining group); Statistical differences revealed by PLSD Fisher test were indicated as: ^{∗∗∗}, p<0.001 for comparison with vehicle treated WT controls in respective group. All the error bars represent S.E.M.
**Figure 10****:** R-9CDHRA displays antidepressant effects in chronic social defeat stress model.
Figure 10a: Social defeat stress significantly increased immobility time in the forced swim test in mice receiving (vehicle; n=12) as compared to non-stressed control (ctr; n=17) mice. 9cDHRA treatments decreased such immobility in stressed mice in a dose dependently manner (n=8 for 1mg/kg and n=6 for 3 mg/kg of 9cDHRA) and to a similar extent as synthetic RXR agonists UVI2108 (n=12).
Figure 10b: Sucrose preference deficit induced by social defeat stress was prevented by UVI2108 or by 9cDHRA treatments. Statistical differences revealed by PLSD Fisher test were indicated as : ^{∗}, p<0.1 when compared to control mice; #, p<0.1 as compared to vehicle treated stressed mice; $$, p<0.01, $, p<0.1 as compared to absence of sucrose preference corresponding to the value of 50% of sucrose consumption. All the error bars represent S.E.M.
**Figure 11**: Measurement of Retinol in cell culture after retinol application: ROL standard (black line), ATROL applied (blue line), 9CROL applied (red line).
**Figure 12****:** Measurement of DH-RA in cell culture after retinol application: ROL standard (black line), ATROL applied (blue line), 9CROL applied (red line).
**Figure 13**: RA measurement in cell culture after retinol application: ROL standard (black line), ATROL applied (blue line), 9CROL applied (red line).
**Figure 14****:** A. 9CDHROL standard mixture (top chromatogram) and endogenous levels in mouse brain (middle chromatogram) and after 9CDHROL-treatmenst (bottom chromatogram), both y-axis scales of the brain samples were identical, while the y-axis of the standard are not similar and was fit on the maximum high of the relevant peaks. B. 9CDHROL in human serum (top chromatogram) and 9CDHROL / ATDHROL standard mixture (bottom chromatogram), both y-axis scales are not similar and were fit on the maximum high of the relevant peaks. C. 9CDHROL in human food chain / cow liver (top chromatogram) and 9CDHROL / ATDHROL standard mixture (bottom chromatogram), both y-axis scales are not similar and were fit on the maximum high of the relevant peaks. D. Conversion of 9CDHBC and 9CBC to 9CDHROL in human oligodendrocyte cell line in vitro after CTRL, 9CBC, 9CDHBC and 9CDHROL administration. Y-axis scales of the top three chromatograms are similar and were fit on the maximum high of the relevant peaks, while the bottom chromatogram has a much larger scale.
**Figure 15****:** 9CDHBC in the human food chain, standard 9CDHBC eluting at 25.0 min (upper figure) and extracted peach from a can (bottom figure) with a peak co-eluting at 25,0 min and comparable UV /VIS spectra (data not shown).
**Figure 16****:** Intra-conversion of carotenoids: A. Conversion of administered 9CBC to 9CDHBC in human oligodendrocyte cell line *in vitro.* Peak with a retention time of 25,1 min is present after 9CDHBC treatment as well in lower levels after 9CBC treatment. B. No conversion of ATBC to either 9CBC or 9CDHBC in oligodendrocyte human cell line *in vitro.* ATBC is eluting at 27,2 min (detectable at 411 nm, while having a UVmax of 450 nm) and present in very high levels after ATBC-treatment (second figure from the top at the right panel). 9CBC is present as a major shoulder peak after 9CBC-treatmenst while non-detectable after CTRL or alternative treatments.
**Figure 17****:** A. Conversion of 9CDHROL, 9CDHROL-ester and 9CDHRA-ester to 9CDHRA in human oligodendrocyte cell line *in vitro.* A slightly different retention time of the derivatives was observed due to a different HPLC-system and extraction procedure used as explained in the materials and methods section, retinoid gradient. B. Conversion of 9CDHBC to 9CDHRA: 9CDHRA and ATDHRA standard levels after control-treatment / endogenous (top chromatogram) and 9CDHBC-supplementation (bottom chromatograms) in mouse liver using the same y-axis scale dimension. C. Conversion of 9CDHROL to 9CDHRA: 9CDHRA and ATDHRA standard levels in serum, brain and liver after control-treatment / endogenous (top chromatograms) and after 9CDHROL-supplementation (bottom chromatograms) in mice using the same y-axis scale dimension per relevant organ (serum, brain, liver).
**Figure 18****:** Antidepressant activities of 9CDHROL and 9CDHBC in chronic social defeat stress model. (a) Social defeat stress significantly increased immobility time in the forced swim test in mice receiving (veh; n=6) as compared to non-stressed control (CTR; n=6) mice. 9CDHROL treatment (n=5) decreased such immobility in stressed mice similarly to 9CDHBC (n=4). (b) Sucrose preference deficit induced by social defeat stress was prevented by 9CDHROL or 9CDHBC treatments. Statistical differences revealed by student t-test were indicated as: ^{∗∗}, p<0.01 when compared to control mice; #, p<0.05 as compared to vehicle treated stressed mice; $, p<0.05 as compared to the value of 50% of sucrose consumption corresponding to absence of sucrose preference. All the error bars represent S.E.M.

### DEFINITIONS

It is to be understood that this invention is not limited to the specific examples and embodiments provided herein and alternatives which are within the skills of a person skilled in the art are to be included. It is also to be understood that the terminology used herein is for the purpose of describing particular embodiments only, and is not intended to be limiting.

As used in this specification and the appended claims, the singular forms "a", "an" and "the" include plural references unless the context clearly dictates otherwise.

Typically the compounds according to the invention relate to enantiomers (pure enantiomers or mixture provided that they are obtainable by the method of the invention), hydrates and solvates of same, solid forms of same, as well as mixtures of said forms.

An "enantiomer" is either one of a pair of optical isomer compounds that are different compound and are mirror images of each other.

"Enantiomer" is preferably understood herein as a compound of the invention as obtainable by an enantioselective method preferably an enantioselective preparation method, highly preferably as an enantiopure or enantiomerically pure compound. "Enantiopure" or "enantiomerically pure" is a compound wherein the molecules have (to the extent obtainable by the method of the invention) the same chirality. Preferably enantiopure or enantiomerically pure means optically pure, more preferably having at least 90%, preferably at least 95%, more preferably at least 98% or 99% optical purity. Highly preferably the molecules of the "enantiopure" or "enantiomerically pure" have the same chirality within limits of detection.

"Alkyl" refers to herein a straight or branched hydrocarbon chain radical consisting solely of carbon and hydrogen atoms, which is saturated, having up to 25 (preferably 23 or 21 or 19) carbon atoms. In certain embodiments an alkyl may comprise 1 to 25 carbon atoms (referred to as C₁₋₂₅ alkyl), or preferably it can be a C₁₃₋₂₅ alkyl, a C₁₃₋₂₃ alkyl a C₁₃₋₂₁ alkyl, a C₁₃₋₁₉ alkyl or a C₁₃₋₁₇ alkyl; alternatively, it can be a short chain alkyl, e.g. a C₁₋₃ alkyl, a C₁₋₄ alkyl, a C₁₋₅ alkyl, a C₁₋₆ alkyl, a C₁₋₇ alkyl or a C₁₋₈ alkyl; still alternatively in some embodiment it can be a C₈₋₁₇ alkyl, a C₈₋₁₅ alkyl or a C₈₋₁₃ alkyl. The alkyl is attached to the rest of the molecule by a single covalent bond. The alkyl is preferably a non-branched, straight hydrocarbon chain. Alternatively it may comprise a branched hydrocarbon chain, however, the branched side chains are typically methyl, ethyl or propyl groups, preferably methyl or ethyl groups. The alkyl chain may be optionally substituted by one or more of the following substituents: halo (including -F, Br, Cl or I), cyano, nitro, oxo, C₁₋₃ alkoxyl or hydroxyl. Preferably the alkyl is unsubstituted.

"Alkenyl" refers to herein a straight or branched hydrocarbon chain radical consisting solely of carbon and hydrogen atoms, which is unsaturated, i.e. containing at least one double bond (i.e. C=C), having at least 2 and up to 25 (preferably 23 or 21 or 19) carbon atoms. In certain embodiments an alkyl may comprise 2 to 25 carbon atoms (referred to as C₂₋₂₅ alkyl); mutatis mutandis the chain length can be the same as with alkyls, however the shortest chain comprises at least 2 carbon atoms. In respect of branching and substitution the same applies as in case of alkyl chain.

The esters of (R) 9-cis-13,14-dihydro-retinol of the present invention may be fatty acid esters. Here, the term "fatty acid" refers to a carboxylic acid having a long aliphatic (non-aromatic) chain which can be saturated (alkyl) or unsaturated (alkenyl). Preferably the alkyl chains in fatty acids used in the invention are open chain compounds which are either straight or branched. Typically, fatty acid alkyl chains contain at least 11 and at most 25, preferably 23, 22 or 21 carbons.

The compounds of the invention have pharmaceutical (medicinal) and nutritional uses as well.

A "pharmaceutical composition" relates to a composition for use in treatment of human or animal body to restore or maintain health, said composition comprising an (one or more) active agent and one or more additional substance useful as carrier. The term "carrier" refers to a diluent, adjuvant, filler, excipient, stabilizer, or vehicle with which the agent is formulated for administration.

"Nutraceutical" refers to a foodstuff that provides health benefits in addition to its basic nutritional value. A nutraceutical has a physiological benefit or provide protection against physiological disorder or discomfort. A neutraceutical composition comprises a composition of the invention and at least an additional substance, e.g. a neutraceutical carrier or a food component.

The term "dietary supplement" refers to a neutraceutical e.g. a neutraceutical composition intended to provide nutrients that may otherwise not be consumed in sufficient quantities.

"Functional food" is also a neutraceutical e.g. a neutraceutical composition and refers to any modified food or food ingredient that may provide a benefit or provide protection against physiological disorder or discomfort; beyond the traditional nutrients it contains.

A "health claim" defines a health benefit for a neutraceutical and is subject to regulatory approval (analogous to an indication in case of a medicament) in accordance with a national or equivalent law. A "health claim" is to be as food labels and in food marketing.

The word "comprising" as used herein means contain i.e. is allows the presence of other entities or members. The term may be limited to "consisting essentially of" meaning comprising the listed essential components or ingredients and optionally other non-essential components or ingredients; or to "consisting of" which means that any additional component is excluded.

"Therapy" may include prevention and/or treatment.

"Preventing" or "prevention" of the development of a disease or condition refers at least the reduction of likelihood of the risk of or susceptibility to acquiring a disease or disorder, or preferably causing at least one of the clinical symptoms of the disease or disorder not to develop in a patient that may be exposed to or predisposed to the disease but does not yet experience or display symptoms of the disease.

"Treating" or "treatment" of any disease or disorder refers, in some embodiments, to amelioration of at least one disease, disorder or condition or preferably reducing the development of the disease or disorder or at least one of the clinical symptoms thereof. In certain embodiments "treating" or "treatment" refers to ameliorating at least one physical parameter of the disease. In certain embodiments "treating" or "treatment" refers to inhibiting the disease or disorder or condition, either physically or physiologically. In certain embodiments, "treating" or "treatment" refers to preventing or delaying the onset of the disease or disorder.

Specifically in case of diseases of the central or peripheral nervous system treatment and in particular in case of degenerative disorders or in case of mental disorders, as appropriate, may include slowing in the rate of degeneration or decline; making the degeneration less debilitating; improving a subject's physical or mental well being; improving or preserving memory and/or cognitive functions; restoring and/ or improving alertness and ability to concentrate or, in some situations, preventing the onset of dementia.

Thus, the terms "prevention" and "treatment" may overlap as follows from the above definition as far as and when the latter includes preventing or delaying the onset of the disease or disorder.

A subject is any animal subject or a human subject, in particular a vertebrate subject, more particularly a warm-blooded subject or a mammalian subject.

A "mammalian subject" as used herein may be any mammal, preferably a laboratory animal, a pet, a livestock animal or a domestic animal. A mammalian subject may be, as example, a rodent, a primate, an ape or a human. Preferably mammals are any vertebrates within the class Mammalia having neocortex (a region of the brain), hair and mammary glands.

The compounds and the pharmaceutical compositions according to the invention may be used in a therapeutic method.

The compounds and the pharmaceutical compositions according to the invention are use in a therapeutic method for the treatment of disease involving retinoic acid receptors (RAR) and retinoid X receptors (RXR). Preferably the disease involves retinoid X receptors and the ligand of the invention is selective thereto. Such disease may be a disease due to impairment of RXR signalling.

The compounds and the pharmaceutical compositions according to the invention are used in a therapeutic method, in particular for the treatment of a mental disorder/disease.

The term "central nervous system related diseases" are diseases or disorders affecting the central nervous system (CNS) i.e. either the brain or the spinal cord, resulting in neurological or mental disorders, preferably which are linked to the RXR complex with RAR or other nuclear receptors. Causes of CNS diseases may be e.g. trauma, infections, degeneration, autoimmune disorders, structural defects, tumors, and stroke. Here we focus on neurodegenerative diseases, mood disorders, schizophrenia, and autism.

The term "peripheral nervous system related diseases" are diseases affecting the peripheral nervous system, preferably which are linked to the RXR complex with RAR or other nuclear receptors. However the ligand of the invention is preferably selective to RXR.

The term "mental disorder" include among others obsessive-compulsive disorder, post-traumatic stress disorder, anxiety, panic attacks, schizophrenia, schizoaffective disorders, depression, mania, manic-depression (bipolar disorder), apathy, delirium, phobias, amnesia, eating disorders (e.g., bulimia, anorexia), and the like. In one embodiment, the mental disorders include obsessive-compulsive disorder, post-traumatic stress disorder, panic attacks, schizophrenia, schizoaffective disorders, depression, mania, manic-depression (bipolar disorder), apathy, delirium, phobias, amnesia, and eating disorders (e.g., bulimia, anorexia). In another embodiment, the psychiatric disorders include obsessive-compulsive disorder, schizophrenia, schizoaffective disorders, depression, mania, manic-depression (bipolar disorder), apathy, delirium, and phobias. In another embodiment, the psychiatric disorders include obsessive-compulsive disorder, schizophrenia, schizoaffective disorders, depression, mania, and manic-depression (bipolar disorder). Preferably or in particular the term "psychiatric disorder" as used herein refer to and will be understood by the skilled person as "mental disorders" as described in sections F06-F50 of WHO International Statistical Classification of Diseases and Related Health Problems 10^{th} Revision. In an embodiment neurodegenerative disorders are excluded from the scope of mental disorders or mental disorders.

"Working memory or synonym short-term memory" characterizes the acquisition, storage, retention and recall of the information for a short interval of time spanning from some minutes to several hours, optionally days and used to modify behavior of the subject.

"Memory loss" refers to a reduction in the ability to acquire, store, retain and/or recall information including past experiences, knowledge and thoughts.

The term "depression" or "depressive disorder" or "mood disorder" refers to a medical field that can be understood by the skilled practitioner. A "mood disorder" refers to disruption of feeling tone or emotional state experienced by an individual for an extensive period of time. Mood disorders include major depression disorder (i.e., unipolar disorder), mania, dysphoria, bipolar disorder, dysthymia, cyclothymia and many others. See, e.g., Diagnostic and Statistical Manual of Mental Disorders, Fourth Edition, (DSM IV). A "Major depression disorder," "major depressive disorder," or "unipolar disorder" refers to a mood disorder involving any of the following symptoms: persistent sad, anxious, or "empty" mood; feelings of hopelessness or pessimism; feelings of guilt, worthlessness, or helplessness; loss of interest or pleasure in hobbies and activities that were once enjoyed, including sex; decreased energy, fatigue, being "slowed down"; difficulty concentrating, remembering, or making decisions; insomnia, early-morning awakening, or oversleeping; appetite and/or weight loss or overeating and weight gain; thoughts of death or suicide or suicide attempts; restlessness or irritability; or persistent physical symptoms that do not respond to treatment, such as headaches, digestive disorders, and chronic pain. Various subtypes of depression are described in, e.g., DSM IV.

The term "neurodegenerative disease or disorder" as used herein refers to a disease or disorder characterized by progressive nervous system dysfunction or by progressive loss of structure or function of neural tissue, preferably of neurons, including death of neurons. The "neurodegenerative disease or disorder" is preferably selected from the group consisting of Parkinson's disease, Alzheimer's disease, Huntington's disease, frontotemporal lobar degeneration associated with protein TDP-43 (FTLD-TDP, Dementia with Lewy bodies (DLB), vascular dementia, Amyotrophic lateral sclerosis (ALS), Mild Cognitive Impairment (MCI), Parkinson's disease with MCI, and other neurodegenerative related dementias due to changes in the brain caused by ageing, disease or trauma; or spinal cord injury and ataxias, disseminated sclerosis and multiple sclerosis, or other neurological conditions.

The compounds and the pharmaceutical compositions according to the invention are used in a therapeutic method, in particular for the treatment of Alzheimer disease.

The term "Alzheimer's disease" refers to the most common form of dementia, which is well recognized clinically. Memory loss also plays an important part in this disease.

### DETAILED DESCRIPTION OF THE INVENTION

Vitamin A is a substance that can fully reverse vitamin A deficiency syndrome (IUPAC-IUB, 1982; Moore, 1929; Rühl, 2007). Vitamin A1, used herein as a term applied to the retinol, retinyl esters and retinal, and pro-vitamin A derivatives like β-carotene, α-carotene and β-cryptoxanthin and comparable vitamin A2 derivatives (3,4-didehydro-retinoids and the carotenoid anhydro-lutein), which mainly occur in avian and fish species (Cama et al., 1952; Moise et al., 2007), belong to this category. The human relevance of visual pigments from the vitamin A3 and A4 cluster, which thus far have not been linked to vertebrates may also be considered critically (Babino et al., 2016).

In adult animals RXR-heterodimers, mainly RXR-VDR, RXR-PPAR, RXR-FXR and RXR-LXR heterodimers along with RXR-RAR, regulate homeostatic lipid metabolism and inflammation (reviewed in (Chawla et al., 2001; Desvergne, 2007; Evans and Mangelsdorf, 2014; Mangelsdorf and Evans, 1995; Mangelsdorf et al., 1995; Shulman and Mangelsdorf, 2005)). Changes in this receptor-mediated signaling result in severe metabolic and immunological diseases (reviewed in ((Szanto et al., 2004a) and (Desvergne, 2007)). Several of these physiological effects are dependent on RXR-mediated processes like inflammatory response and lipid signaling and they are thereby linked to various patho-physiological effects identified in adult vitamin A-deficient animals (Nunez et al., 2010; Stephensen et al., 2007; Wan et al., 2003).

An endogenous RXR ligand serves as the major switch to enable RXR-heterodimer-mediated signaling in the mammalian organism. Various effects observed in vitamin A-deficient animals were comparable to effects found in RXR-KO animals (Kastner et al., 1997a; Kastner et al., 1997b). The present inventors believe that, besides non-nuclear hormone receptor-mediated effects in vision, RAR- and especially RXR-mediated pathways are the major pathways for vitamin A-activity and that these are dependent on endogenous RAR and/or RXR ligands.

It is widely accepted that ATRA is the endogenous relevant RAR ligand but other ligands have also been described (Moise et al., 2005; Riihl et al., 2015). 9-*cis*-Retinoids can be converted to all-*trans*-retinoids and serve as additional precursors for the endogenous RAR-ligand ATRA.

Shirley M A et al. in 1996 (Shirley M A et al. 1996) have found that in rats 9-cis-RA is reduced to 13,14-dihydro-9-cis-RA and the latter is conjugated with taurine to form a novel metabolite, considered this, nevertheless, as an initial step leading to beta-oxidation.

Recently 9-*cis*-13,14-dihydroretinoic acid (9CDHRA) has been identified as an endogenous retinoid in mice. It has been concluded by the present inventors that 9CDHRA is the endogenous relevant ligand for the retinoid X receptor (RXR), a nuclear hormone receptor involved in the regulation of various physiological relevant pathways in the brain, lipid metabolism, inflammation, differentiation, proliferation and cell cycle. The RXR-mediated signaling has been reported to be dysregulated in various diseases ranging from neurological dysfunctions, cardiovascular diseases and skin / immune-related diseases where an RXR-signalling related dysregulation of lipid homeostasis and inflammation are involved. The inventors have further identified the endogenous RXR ligand, 9CDHRA, as a signaling lipid molecule possibly related to the dysfunctions of RXR due to reduced levels of the ligand. Thereby a new vitamin A pathway called vitamin 5 pathway has been identified.

The present invention relates to physiological as well as nutritional precursors of 9CDHRA. The major precursor is claimed to be 9-*cis*-13,14-dihydroretinol (9CDHROL), i.e. vitamin A5 and its esters, 9-*cis*-13,14-di-hydroretinyl esters (9CDHROL-esters) as well as 9-*cis*-13,14-dihydroretinoic acid esters (9CDHRA-esters). In addition to these molecules novel carotenoids as pro-vitamin A(5) precursors like 9-*cis*-13,14-dihydro-β-carotene (9CDHBC). Further upstream precursors of 9CDHBC (like 9-cis-β-carotene (9CBC)) are contemplated.

The aim of the present study was to find and synthesize by organic chemical synthesis these additional derivatives and test representative examples of these precursors in animal models for depression in mouse models.

In the set of experiments described in the Examples the present inventors have developed a new chemical synthesis of 9-cis-13,14-dihydroretinol and 9-cis-13,14-dihydroretinyl-acetate via ethyl 9-cis-13,14-dihydroretinoate. Other esters of 9-cis-13,14-dihydroretinoic acid and 9-cis-13,14-dihydroretinol (such as palmitate and others) can be easily prepared using basically the same method.

New synthesis methods for 9-cis-13,14-dihydro-β-carotene and synthesized 9-cis-β-carotene have also been developed.

Alternatively, chemoenzymatic synthesis of long-chain retinyl esters is also an option. A part of these syntheses utilize retinol as the starting material [O'Connor et. al. Ausl..1. Chem. 1992, 45, 641; Maugard et. al. Bio-lechnol. Prog. 2002, 18, 424.). Certain authors have utilized retinyl esters such as retinyl acetate as the starting material for a biocatalytic preparation of long-chain retinyl esters (see e.g. unexamined Japanese Patent Application JP 62-248495, 1987). US7566795B2 describes an improved method for the preparation of long-chain esters of retinol via chemoenzymatic processing from short-chain retinyl esters and an appropriate long-chain acid or ester in the presence of an enzyme and organic solvent and optionally in the presence of at least one molecular sieve and/or at least one ion exchange resin to form the retinyl ester.

Several patent applications have been directed to various vitamin A form precursors and variants thereof.

As to esters of acid forms, WO 95/04018 A1 relates to the preparation of 9-cis retinoic acid esters. The compounds comprise double bonds between carbons 11 and 12, as well as between carbon atoms 13 and 14 and can have either *cis* or *trans* configurations. While methods can be utilized herein, the compounds necessarily cannot be 13,14 dihydro derivatives. WO 95/32946 A1 also relates to the preparation of 9-cis-retinoic acid esters. The compounds must comprise double bonds between carbons 11-12 and 13-14, which may be both cis and trans isomers.

As to esters of retinol, WO2013134867 A1 describes the treatment of retinitis pigmentosa using 9-cis or 11-cis retinyl esters (see claim 17). The description mentions 9-cis-13,14-dihydroretinoic acid as a metabolite, but there is no suggestion to 9-cis-13,14-dihydroretinol derivatives. ("Pharmacokinetic analyses showed the predominant metabolites to be either 9-cis-retinyl oleate or 9-cis-retinyl palmitate and 13,14-dihydro-9-cis-retinoic acid. At 4 hours after dosing, the concentration of these compounds was higher than that of 9-cis-retinyl acetate and 9-cis-retinol." See page 70, penultimate paragraph.)

WO 2011/034551 A2 describes pharmaceutical formulations comprising one or more 9-cis-retinyl esters (acetate, palmitate, etc.) in a lipid vehicle and their uses for ophthalmic purposes. The description does not mention the use of 9-cis-13,14-dihydroretinol derivatives.

Thus, apparently no documents appears to describe the medical use or, in particular, the nutritional use of 9-cis-13,14-dihydroretinol or its ester derivatives. Moreover, we have not found data relating to the ester derivatives of 9-cis-13,14-dihydroretinol.

RXR-ligand binding is a highly important mechanism responsible for vitamin A-mediated effects.

The RXR-precursor pathway is different from the RAR-precursor pathway leading to ATRA and selective RAR-mediated signaling. Alternatively pro-vitamin A1 carotenoids like proven for ATBC cannot be converted in the human organism to 9-cis-derivatives and are thereby no precursors for RXR-selective ligands. Vitamin A1 alcohol all-trans-retinol is weakly and non-isomer-selectively converted to 9CDHROL and is thereby a weak precursor and a no selective precursors for RXR-selective ligands. ATROL mainly may serves as multifunctional precursors which is stored as retinyl-ester and tightly controlled by binding proteins in serum and cells for general homeostasis and enabling RAR- and RXR-ligand synthesis and further RAR- and RXR-mediated signaling without RXR-selectivity.

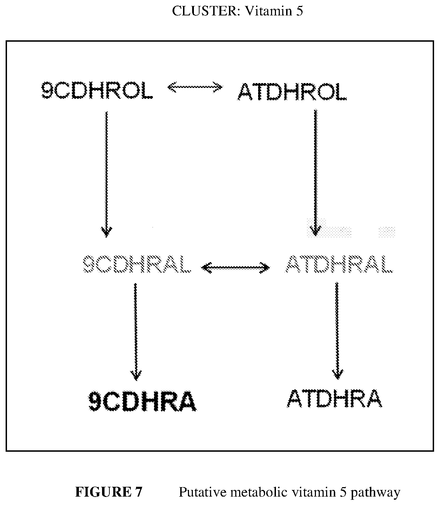

It is contemplated herein that 9-*cis*-13,14-dihydroretinoids can be considered as specific and/or selective precursors of RXR ligands. Based on the information outlined above, potential RXR-ligands originating from vitamin A1 and A2 pathways seem to have no or very limited endogenous relevance. The present inventors have shown, however, that this new signaling pathway, which is relevant for RXR-activation, should also be included as a crucial criterion for general vitamin A functions (Figure 7). In the figure the presence of 9CDHRAL and ATDHRAL are not yet identified. This route appears to be specific to the vitamin A pathway as analogous alcoholic forms 9CROL and ATROL essentially do not convert into the corresponding acids in cell cultures e.g. in oligodendrocytes. In summary, because the non-endogenously relevant 9CRA originating from vitamin A1 pathway should be excluded as an endogenous relevant RXR-activator, then 9-*cis*-13,14-dihydroretinoids and their nutritional precursors represent a novel vitamin A signaling pathway which is called the vitamin A5 pathway.

The present inventors have elucidate the metabolic conversion of compounds of this vitamin A5 pathway in supplementation experiments in the mammalian organism in *in vitro* cellular systems, identify them in mouse and human organism as endogenous derivatives and identify these derivatives directly or indirectly within the human food chain. In addition, it has been investigated and surprisingly found that 9CDHROL and 9CDHBC display antidepressant activities in social stress defeat protocol as an RXR-mediated process. Therefore the invention relates to novel treatment methods of depression by administration of RXR-ligands or vitamin A5 (9CDHROL) as well aas RXR-ligand pre-precursors or vitamin A5 precursors (pro-vitamin A5 derivatives) like 9CDHBC.

As an example to show RXR signalling effect, among RXR-signalling dysregulated diseases, depression has been chosen as a heterogeneous group of psychiatric disorder with non-clearly identified aetiology. It is characterised by a number of core, affective symptoms including anhedonia and feelings of despair as well as less specific, cognitive symptoms such as deficits of working memory. It has been shown that reduced bioavailability of 9CDHRA in mice carrying null mutation for cellular retinol binding protein 1 (Rbp1) leads to depressive-like behaviours, whereas pharmacological treatment with 9CDHRA restores normal behaviour in these mice. For depression the administration of selective RAR-ligands does not lead to any improvement and this dysfunction and it seems to be a selective RXR-signalling related dysfunction. The treatments with 9CDHRA precursors as disclosed herein are of direct relevance for research into depression as 9CDHRA and 9CDHRA-precursors, similarly to synthetic RXR ligands was effective in preventing depressive-like behaviours in chronic stress animal model of depressive behaviours.

Specifically the present inventors have determined that the 9CDHRA precursor 9CDHROL, when given orally to mice can also induce positive effects on memory performance in mice. Moreover they have determined that 9CDHROL is a precursor of 9CDHRA when given orally to mice.

This is a surprising finding as analogues 9-cis-retinol (9CROL) and all-trans-retinol (ATROL) are not or just weakly converted to 9-cis-retinoic acid (9CRA) and all-trans-retinoic acid (ATRA) in *in vitro* experiments (see Figure 13) therefore they are not good precursor substrates for the corresponding acid forms.

Similarly, analogues 9-cis-retinol (9CROL) and all-trans-retinol (ATROL) are not or just weakly converted to 9-cis-13,14-dihydroretinoic acid (9CDHRA) and all-trans-13,14-dihydroretinoic acid (ATDHRA) in *in vitro* experiments (see Figure 13) therefore they are not good precursor substrates for active RAR- and RXR ligands, either.

Thus the present inventors have found an unexpected novel vitamin A pathway by showing that 9CDHRA precursor 9CDHROL as well as esters thereof are special precursors of the active RAR- and RXR ligands.

The present results allow the conclusion that 9CDHROL is a novel endogenous retinoid in mice and derivatives which, once administered, result in 9CDHROL in the animal or human body, can be considered as a source of this new type of vitamin A.

The present inventors have also determined that 9CDHROL-ester and 9CDHRA-ester are alternative to 9CDHROL as precursors for 9CDHRA.

Thus, 9CDHROL and 9CDHROL-ester and 9CDHRA-ester are physiological and nutritional precursors for 9CDHRA and can be used as alternative treatments to reach higher 9CDHRA-levels in (specific) tissues.

In additional animal studies in a set of models of mental diseases further evidence has been provided that R-9CDHRA displays RXR agonist-like activities in vivo and reverses behavioural deficits in mice.

Together with evidence that 9CDHRA-esters and 9CDHROL as well as its esters increase the 9CDHRA-levels in a surprising manner in the brain and in oligodendrocytes, showing reversal of behavioural deficits in Rbp1 - /- mice, improvement of cognitive performance in wild type mice reversal of antidepressant effects in chronic social defeat stress model further supports the utility of the compounds of the invention in a broad range of diseases of the central and peripheral nervous system. Rbp1-/- mice were used beacuse they show the same type of deficits as RXRg-/- suggesting that RXRg signaling is down-regulated. Normalise their memory is a proof that these mice are missing an RXR ligad and not the receptor itself (or receptor functionality). The use of RXRg-/- mice is a negative control to show that 9cDHRA acts at RXRg to improve memory - in consequence in absence of RXRg it cannot improve memory. The results were confirmed in wild type animals.

RXR-liganding by 9CDHRA is resulting in selective RXR-activation pathways and RXR-LXR, RXR-NR4A, RXR-VDR, RXR-FXR and RXR-PPAR mediated signaling. These RXR-mediated signalling pathways can be modified by an RXR-ligand only and not by an RAR-ligand.

The present inventors have found the endogenous RXR ligand 9CDHRA and its effective precursor 9CDHROL (vitamin A5) as well as upstream precursors. This Vitamin A5 / pro-vitamin A5 pathway involves pharmaceutical or nutritional or derma topical applications of our claimed selective RXR-precursors.

Prevention and usage of these Vitamin A5 / pro-vitamin A5 compounds for diseases where RXR-mediated signaling is augmented like for various neurodegenerative diseases and further diseases of the skin and cardiovascular system involving RXR-mediated altered lipid homeostasis and immune-regulation like atherosclerosis, obesity and diabetes. These diseases with a dysfunctional RXR-mediated signaling can be prevented and / or treated by our RXR-selective precursors.

As an example used in this study, depression was treated as an RXR-mediated signaling dysfunction and 9CDHRA as well as 9CDHROL and 9CDHBC as 9CDHRA precursors as an active treatment / prevention strategy. Many other diseases were reported, however, in the art where an RXR-KO phenotype exists ranging mainly from neurodegenerative diseases and dysfunctions in the cardio-vascular system like obesity, diabetes, atherosclerosis and appetite regulation.

We claim that we have found an important physiological switch-mechanism in the human organisms which can be selectively switched on by a) a selective physiological ligand (9CDHRA) or b) selectively by nutritional precursors present in food, which can also be given as supplements / pharmaceuticals, 9CDHROL and 9CDHBC. This switch can enable RXR-mediated signaling and thereby prevent RXR-dependent dysfunctions and diseases.

It has been also surprisingly found that the selective 9CDHRA-precursor 9CDHBC is a pro-vitamin A5 carotenoid for enabling RXR-mediated signaling.

As shown here just 9CDHBC and not 9CBC is an excellent precursor of 9CDHROL (vitamin A5). The carotenoid 9CDHBC is a good precursors for 9CDHRA in mouse serum, liver and brain, when given orally to mouse in *in vivo* supplementation trials In humans carotenoids are transported (while not in mice) and stored within the body, while this happens not in mice. It has been shown that it is 9CDHBC and not 9CBC and especially not ATBC, is an excellent precursors of 9CDHROL and also 9CDHRA.

9CBC (already known) and 9CDHBC are both present in the human food chain (shown for peach and additional for broccoli) as well as an overall precursor in the food chain present in algae. Plancton, mainly containing algae, is the starting material of all food of our planet: starting from plankton / algae, fishes, larger aquatic and later terrestrial animals and finally humans, as the end of the global food chain with relevance selectively evaluated from the human perspective.

According to the present invention functional food is claimed which comprises added isolated vitamin A5 or any precursor thereof like 9CDHBC or other compounds or compounds for use according to the invention. Preferably the functional food according to the invention is a processed food and preferably a preserved food.

The selective RXR precursor ligands of the invention are surprisingly more effective than non-selective compounds. For example, the known endogenous retinoid vitamin A1 alcohol, ATROL, is just a weak and non-isomer selective precursor of 9CDHROL and no precursor of 9CDHRA in human oligodendrocyte cell culture *in vitro.* In supplemented mice it is just weakly and partly non significantly converted to 9CDHRA: factor 2 in serum (non-significantly), factor 2,7 in liver (significantly) and factor 7 in mouse brain (significantly) examined in mouse after oral supplementation we determine thereby no or just inefficient precursor substrate potential for the usage as a selective RXR-ligand precursor. For comparison 9CDHROL, as our novel claimed Vitamin A5 alcohol, is converted to 9CDHRA in human oligodendrocyte cell culture *in vitro* excellently and highly efficiently (factor 98 in serum and factor 76 in liver and factor 1294 in mouse brain, all significantly), when given the same amount of these retinoids supplemented to mice. As shown here, 9CDHROL has been confirmed to be present in human serum as well as in the human food chain examined in commercially available beef liver.

9CDHROL is thereby an excellent physiological and nutritional precursor for 9CDHRA and can be used as alternative treatments to selectively reach higher 9CDHRA-levels and selective RXR-mediated signaling proven in mice behavioral studies. 9CDHROL is very efficiently converted to 9CDHRA, as shown in *in vitro* as well as *in vivo* supplementation trials. Esters of 9CDHROL (9CDHROL-ester) and 9CDHRA (9CDHRA-ester) are also excellent and even more stable derivatives and can be given alternatively and yielding even higher conversion to 9CDHRA. We claim that 9CDHROL belongs to a novel selective vitamin A family functioning as selective RXR-ligand precursors, named to be Vitamin A5 family.

9CDHBC is a novel endogenous derivative identified in high amounts in the human food chain at various levels. We claim therefore that 9CDHBC is a novel selective pro-vitamin, named pro-vitamin A5 as a selective precursor for the selective RXR-ligand 9CDHRA.

We also claim that 9CBC is a pre-precursor of an RXR-ligand and a pro-pro-vitamin A5. 9CBC can be used alternatively as a food ingredient or nutraceutical derivatives with a very weak and non-selective derivative for preventing VA5-deficiency and for general VA5-supplementation to prevent RXR-dependent dysfunctions of our organisms, here shown representatively by depression as a selective RXR-ligand / RXR-mediated neurodegenerative disease.

13,14-Dihydro-carotenoids have not been reported to date except for the case of acyclic carotenoids such as lycopene, phytoene and phytofluene. The 13,14-double bond formal hydrogenation might result from the action of retinol-saturase (RETSAT) on the unsaturated precursor retinol or from an alternative metabolism of classical known retinoids (Moise et al., 2004). All-trans-13,14-dihydro-retinoic acid have been identified by research groups of the present inventors in high concentration in serum, liver and brain of young wildtype non-vitamin A supplemented mice (respectively, 96 ng/ml; 352 ng/mg; 38 ng/g).

In summary, the pathway from 9-cis-dihydro-carotenoids, preferably 9-cis-13,14-dihydro-carotenoids has a physiological relevance for endogenous 9CDHRA synthesis starting from dietary carotenoids. Thus, it is contemplated that 9CDHROL and 9CDHBC are nutritional and endogenous relevant precursors of the endogenous RXR-ligand 9CDHRA.

Thus, the invention also relates to the use of 9-cis-dihydro-carotenoids (9CDHBC) for the purposes as disclosed herein.

The experiments show that the compounds of the invention are capable of providing 9cDHRA and could work to maintain health, in particular health of the central or peripheral nervous system and/or maintain health against the diseases mentioned herein or prevent or treat said diseases practically in anybody. In particular the disease is a central or peripheral nervous system disease as listed herein.

Certain aspects and embodiment of the invention are detailed below, then the invention is illustrated by Examples which are part of the invention; however, the skilled person will understand that based on the specific and general teaching provided herein other variants and embodiments are at hand and can be carried out.

### Preparation of the compounds of the invention

The starting compounds may be obtained commercially or may be synthesized according to standard methods.

### Preparation of 9-cis-13,14-dihydroretinoic acic and its esters

In the embodiment presented in the Examples the preparation of (*R*)-**4** ethyl ester of (*R*)-9-cis-13,14-dihydroretinoic acid [(*R*)-**1**] was based on the Suzuki coupling of enantiopure trienyliodide **3** and boronic acid **2** (See Scheme 1 in Example 1). The synthesis of **3** started with (*Z*)-stannyldienol **5** which was transformed into the benzothiazolyl allyl sulfide **6** by Mitsunobu reaction with the corresponding thiol and subsequently oxidized to sulfone **7** with H₂O₂ and a peroxymolybdate (VI) reagent. The Julia-Kocienski olefination was performed using a slight excess of base and in the excess of enantiopure aldehyde (*R*)-**8** which is an ethyl-ester. As anticipated from previous findings on the stereochemical outcome of the reactions of allylsulfones and aldehydes, the newly formed olefin of trienyl ester (*R*)-**9** is of *Z*-geometry. Treatment of the precursor stannane with a solution of iodine in CH₂Cl₂ produced the iodide (*R*)-**3** via Sn-I exchange and iodine-promoted isomerization of the 9*Z*,11*Z* diene to the desired 9*Z*,11*E* geometric isomer. Geometric isomers can be confirmed by NOE experiments.

The Suzuki reaction of freshly prepared boronic acid **2** and dienyl iodide (*R*)-**3**, followed by immediate work-up resulted in ethyl (*R*)-9-*cis*-13,14-dihydroretinoate (*R*)-**4**. Saponification of (*R*)-**4** provided the desired carboxylic acid (*R*)-**1** without detectable loss of stereochemical integrity. (See reaction Scheme 1)

Following the general scheme, enantiomer (*S*)-**1** was also prepared with similar efficiency.

The preparation of the compounds of the invention is described in more detail in Example 1.

Alternative 9-*cis*-13,14-dihydroretinoic acid esters can be prepared with the corresponding ester variants of the (*R*)-**8** compound.

In addition, the carboxylic acid 9-*cis*-13,14-dihydroretinoic acid can be esterified with other alkyl or alkenyl-containing alcohols using standard procedures, such as the treatment of a mixture of both compound with dicyclohexylcarbodiimide (DCC) and dimethylaminopyridine (DMAP).

Moreover further esters could be prepared starting from the corresponding aldehyde (*R*)-**8** with a different ester than ethyl.

### Preparation of 9-cis-13,14-dihydroretinol and its 9-cis-13,14-dihydroretinyl esters

9-*cis*-13,14-dihydroretinol has been prepared as described herein by DIBAL-H (Diisobutylaluminium hydride) reduction of Ethyl 13,14-dihydroretinoate.

Ester reduction can also be accomplished with lithium aluminum hydride (LiAlH₄) in tetrahydrofuran (THF) or diethyl ether, or with lithium borohydride and an alcohol (EtOH, MeOH) in THF/diethyl ether as solvent.

9-*cis*-13,14-dihydroretinyl acetate was prepared from 9-*cis*-13,14-dihydroretinol by adding excess Ac₂O and pyridine in the presence of DMAP. The obtained ester was isolated and purified.

The synthesis method maintained the configuration optical purity of the enantiomers.

By this method alternative esters can be prepared with a carboxylic acid anhydride reagent R₂O, being R a carboxylic acid, or using the corresponding chlorides derived from the carboxylic acids, or using the activated carboxylic acid prepared, for example, with DCC and DMAP.

Esters of 9-*cis*-13,14-dihydroretinol can be prepared by transesterification reactions of esters thereby the acyl group can be replaced.

Alternatively, the retinyl esters can be prepared from 9-cis-13,14-dihydroretinol using appropriate esterifying agents.

A further method may be selective enzymatic reactions.

For example lecithin:retinol acyltransferase (LRAT) catalyzes a transesterification, transferring long chain fatty acyl moieties (primarily palmitic, stearic, oleic and linoleic acids) present at the sn-1 position of membrane bilayer phosphatidylcholine to retinol, forming retinyl esters (O'Byme, Sheila M. et al. 2013). Lipases form a further group of enzymes suitable for synthesis of esters, like fatty acid ester (Kai Z J 2011, Yin Chunhua 2006) Alternatively, a combined chemo-enzymatic method can be used (Liu ZQ 2015)

Such enzymes may be prepared by recombinant techniques and used to selectively prepare the desired ester. Alternatively the enzyme can be cloned into appropriate host cell, overexpressed in active form and this recombinant cell can be used to prepare the desired ester.

### Certain preferred esters of the invention

### Preferred 9-cis-13,14-dihydroretinoic acid esters

Preferred 9-*cis*-13,14-dihydroretinoic acid esters are typically lower alkyl esters like methyl, ethyl or propyl esters of 9-*cis*-13,14-dihydroretinoic acid. Esters of 9-*cis*-13,14-dihydroretinoic acid with long chain fatty alcohols are less preferred than esters of long chain fatty acids of 9-*cis*-13,14-dihydroretinol.

### Further preferred 9-cis-13,14-dihydroretinyl ester compounds

Further preferred 9-*cis*-13,14-dihydroretinyl ester compounds include formate (methanoate), ethanoate and propionate esters, as well as, though less preferred, butyrate and valerate esters.

Long chain fatty acids may also form esters with 9-cis-13,14-dihydroretinol.

Long chain fatty acids (LCFA) include fatty acids with an aliphatic tails from 13 to 21 carbons.

Long chain fatty acids can be selected from saturated and unsaturated fatty acids the latter including monounsaturated and polyunsaturated fatty acids.

### Saturated fatty acids

Typical esters with saturated fatty acids can be formed e.g. with the following long chain fatty acids:

| | | |
|---|---|---|
| Myristic acid | CH₃(CH₂)₁₂COOH | 14:0 |
| Palmitic acid | CH₃(CH₂)₁₄COOH | 16:0 |
| Stearic acid | CH₃(CH₂)₁₆COOH | 18:0 |
| Arachidic acid | CH₃(CH₂)₁₈COOH | 20:0 |

While saturated fatty acids are often stated to be unadvantageous in diet, they still may be useful components of the esters of the invention as precursors of 9CDHROL in the human tissues.

### Monounsaturated fatty acids (MUFAs)

Monounsaturated fatty acids have one carbon-carbon double bond, which can occur in different positions. Themost common monoenes have a chain length of 16-22 and a double bond with the cis configuration which is preferred.

Preferred MUFAs which are applicable in the present invention are oleic acid CH₃(CH₂)₇(HC=CH)(CH₂)₇COOH and palmitoleic acid CH₃(CH₂)₅(HC=CH)(CH₂)₇COOH. .

### Polyunsaturated fatty acids (PUFAs)

In polyunsaturated fatty acids (PUFAs) the first double bond may be found between the third and the fourth carbon atom from the first carboxylic acid carbon; these are called ω-3 fatty acids. If the first double bond is between the sixth and
seventh carbon atom, then they are called ω-6 fatty acids.

Preferred PUFAs which are applicable in the present invention are for example ω-3 and ω-6 fatty acids.

Among PUFAs arachidonic acid (ARA) and docohexanoic acid (DHA) are useful in the treatment of Alzheimer's disease (see e.g. EP1419780B1).

In this case, PUFAs, once hydrolysed, also can exert their advantageous effect in the organ in question.

In an embodiment, essential fatty acid esters are preferred. Two essential fatty acids are linoleic acid (LA) and alpha-linolenic acid (ALA). The human body has a limited ability to convert ALA into the longer-chain omega-3 fatty acids - eicosapentaenoic acid (EPA) and docosahexaenoic acid (DHA), which are also preferred.

### Branched chain fatty acids (BCFA)

Branched-chain fatty acids (BCFA) are bioactive food components [Ran-Ressler RR et al. "Branched-chain fatty acid content of foods and estimated intake in the USA." Br J Nutr. 2014 Aug 28;112(4):565-72.], which are present in milk and soy and in some cases (like phytanic acid) in ruminant animal fats, and certain fish. Naturally occurring branched chain fatty acids demonstrate potential health properties and are preferred components of the esters of the invention.

Such preferred BCFAs are e.g. the naturally occurring 13-methyltetradecanoic acid, 15-methylpalmitic acid and phytanic acid (3,7,11,15-tetramethyl hexadecanoic acid).

Fatty acids and their role in biology can be found e.g. in the following books:
(Ching Kuang Chow "Fatty Acids in Foods and their Health Implications" 2007; Arild C et al. Fatty Acids: Structures and Properties ENCYCLOPEDIA OF LIFE SCIENCES 2005)
The compounds and/or the pharmaceutical compositions according to the invention may be used in a therapeutic method. A preferred therapy relates to diseases of the nervous system.

### The compounds of the invention in the nervous system and nervous tissue

The compounds of the invention can be converted into their active form in nervous tissue or in the nervous system. Preferably if the compounds are administered to an animal or human subject this conversion in the nervous system or in nervous tissue (in a preferred embodiment in the brain) is preferential.

In the preferred embodiment of the invention the compounds are converted by hydrolysis into 9CDHROL. Alternatively, the compound is 9CDHROL. In the nervous system 9CDHROL is further converted into 9CDHRA which exerts its RXR agonist effect.

The nervous system as used herein consists of the central nervous system (CNS) and the peripheral nervous system (PNS). The central nervous system (CNS) is comprised of the brain and spinal cord.

The peripheral nervous system (PNS) includes all (branching) peripheral nerves, and regulates and controls bodily functions and activity.

According to the invention 9CDHRA formed in situ exerts its RXR agonist effect in the nervous tissue to prevent or treat diseases of the CNS or PNS (as detailed herein wherein such diseases are discussed).

Nervous tissue (or nerve tissue) is the main tissue component of the above-mentioned two parts of the nervous system: the CNS and the PNS. It is composed of neurons, or nerve cells, which receive and transmit impulses, and neuroglia, also known as (neuro)glial cells (glia), which assist the propagation of the nerve impulse as well as providing nutrients to the neuron.

### Neuroglial cells are e.g. the following cells:

### Microglial cells, astrocytes, oligodendrocytes, NG2 glia, Schwann cells, satellite glial cell, enteric glia

To mention a few in somewhat more detail, oligodendrocytes are central nervous system structures that form myelin sheaths on the axons of a neuron, which are lipid-based insulation that promotes an efficient conduction of nerve impulses down the axon. NG2 glia are CNS cells that serve as the developmental precursors of oligodendrocytes. The Schwann cells are the PNS equivalent of oligodendrocytes, they help maintain axons and form myelin sheaths in the PNS.

Experiments with oligodendrocyte cells together with data reporting role of synthetic RXR agonists in control of remyelination support role and utility of compounds described in the patent for control of oligodendrogenesis and myelin sheath function in the CNs and PNS as well as to NG2 glia.

Thus, the diseases of the CNS and PNS which can be treated, prevented or alleviated by an RXR agonist, can be treated with the esters of 9CDHRA, 9CDHROL and esters of the latter.

RAR/RXR, RXR/PPAR or RXR/LXR signaling in the brain is reviewed or evoked for example in the following publications: (van Neerven 2008; Shudo K et al. 2009; Skerrett R et al. 2015)

There are three retinoic X receptors (RXR): RXR-alpha, RXR-beta, and RXR-gamma, encoded by the RXRA, RXRB, RXRG genes, respectively. RXR-ligand binding is a highly important mechanism responsible for vitamin A-mediated effects. RXR occupies a central position in nuclear receptor (NR) signaling, because it serves as the common heterodimerization partner of multiple NRs. These heterodimers are called permissive or nonper-missive depending on whether the role of the RXR is as an active or a silent transcription partner, respectively. Permissive RXR-NR heterodimers are activated by the RXR ligand or by the partner's ligand, and synergistic effects are usually observed if both partners of the heterodimer are bound to their ligands. One of the heterodimers is the RXR-RAR heterodimer.

The present inventors demonstrated that 9CDHRA as an RXR agonist coordinates the transcriptional activities of several nuclear receptor-signaling pathways, possibly through the corresponding permissive heterodimers (Riihl et al., 2015).

The compounds of the invention are particularly useful as RXR agonist and in conditions wherein agonists of RXR, in particular via RXR-RAR, but also RXR-PPAR or RXR-LXR is useful to maintain or restore health.

In a preferred embodiment the compounds can be used for the treatment of impaired cognitive functions and/or impaired learning.

The compounds and/or the pharmaceutical compositions according to the invention may be used in a therapeutic method, in particular for the treatment of memory impairment (memory loss), in particular working memory impairment, e.g. defects and loss, or short term memory impairment, e.g. defects and loss, like amnesia, etc. Memory impairment, impaired learning abilities and impaired cognitive functions may be associated with a mental disorder, such as mild cognitive disorder, amnestic syndrome, memory and cognitive deficiencies in schizophrenia and mood disorders, such as bipolar affective disorder and depression, stress related and anxiety disorders, such as partial and complete amnesia, dissociative amnesia.

Several psychoactive substances and medications are known to cause memory loss, impaired learning abilities and impaired cognitive functions, such as tricyclic antidepressants, dopamine agonists, antihistamines, benzodiazepines, statins, beta blockers, barbiturates, opioids, THC, alcohol, etc.

The compounds and/or the pharmaceutical compositions according to the invention are especially useful in reversing such memory impairment effected by a disease or a medication mentioned above.

The compounds and/or the pharmaceutical compositions according to the invention are especially useful in the prevention and/or treatment of short term memory impairment. The compounds and/or the pharmaceutical compositions according to the invention are particularly useful in improving working memory performance or reducing working memory loss.

The compounds and/or the pharmaceutical compositions according to the invention are useful in the treatment of depression, such as mild, moderate and severe depressive episodes as well as the depressive phase of bipolar disease, cyclothymia or dysthymia, mixed anxiety-depression disorder, depression or depressive episode associated with other diseases, such as schizophrenia, cancer, metabolic diseases, etc.

The compounds and/or the pharmaceutical compositions according to the invention are useful in preventing and/or treating neurodegenerative disorders. Preferably a neurodegenerative disorder is selected from Alzheimer's disease, Parkinson's disease, Mild Cognitive Impairment (MCI), Parkinson's disease with MCI, Alzheimer's disease, Huntington's disease, Dementia with Lewy bodies (DLB), Amyotrophic lateral sclerosis (ALS), and other neurodegenerative related dementias due to changes in the brain caused by ageing, disease or trauma; or spinal cord injury and ataxias, disseminated sclerosis and multiple sclerosis or other neurological conditions.

According to a specific embodiment, the invention also relates to a kit that is suitable for the treatment by the methods described above.

In an embodiment these kits comprise a composition containing the compound of the invention in appropriate dosages and a second composition containing a mood stabilizer, an antidepressant an anxiolytic etc.

In a further embodiment the kit comprises a composition containing the compound of the invention in appropriate dosages and a second composition containing an active agent against a neurodegenerative disorder as defined or listed herein.

The invention also relates to a combination comprising components of the kits as listed above.

Testing the compounds of the invention is possible in animal models (and non-animal models), e.g. chronic social defeat stress model of depressive behaviors, animal models of amyloidosis orAlzheimer disease and Parkinson's disease.

### Nutritional uses

The compounds of the invention might be used as a nutritional or dietary supplement, in a functional food composition, in a dietary supplement composition or in a nutraceutical composition. Such nutritional or dietary supplement, functional food composition, dietary supplement compositions or nutraceutical compositions have the benefit of preventing, reversing and/or alleviating a condition as disclosed herein. In a preferred embodiment the condition is memory loss, in particular working or short term memory impairment, particularly memory loss without the administration of a pharmaceutical, i.e. in a non-medical way and/or have the benefit of preventing, reversing and/or alleviating learning impairment and/or decline of cognitive functions. By non-medical a treatment is meant for the purposes of the application wherein the normal body functions are to be maintained wherein the condition is defined herein is non-pathological or has not reached a pathological level. Preferably, in non-medical treatment a nutritional or dietary supplement, a functional food composition, a dietary supplement composition or a nutraceutical composition is used to prevent, reverse and/or alleviate memory loss, learning impairment and/or decline of cognitive functions in a condition which is still not or cannot be considered as an illness.

Nutraceutical compositions may comprise a "nutraceutically acceptable carrier" means that the carrier is acceptable for human consumption and retain or improve the biological properties of the active agent.

In analogy to other dietary vitamin A compositions based on the invention amounts of retinoids of the vitamin A5 pathway can be measured in 9CDROL equivalents.

The composition of the invention are suitable for use in nutraceutical compositions, e.g. dietary dietary supplement, a functional food, a medical food or a food with a health claim.

As shown herein fat soluble forms would be stored in the liver and supply of the body could be fulfilled therefrom.

### Formulations

Compositions comprise, besides the active agent at least one carrier.

The carrier may be e.g. a diluent, adjuvant, excipient, stabilizer, or vehicle with which the agent is formulated for administration. Pharmaceutical carriers can be sterile liquids, such as water and oils, including those of petroleum, animal, vegetable or synthetic origin, such as peanut oil, soybean oil, mineral oil, sesame oil, and the like. Water is a typical carrier when the pharmaceutical composition is administered intravenously. Saline solutions and aqueous dextrose and glycerol solutions can also be employed as liquid carriers, particularly for injectable solutions.

In one embodiment, the agent is formulated in accordance with routine procedures as a pharmaceutical composition adapted for intravenous administration to mammalian or human beings. Typically, compositions for intravenous administration are solutions in sterile isotonic aqueous buffer. Where necessary, the composition can also include a solubilizing agent.

In solid formulations suitable pharmaceutical excipients include starch, glucose, lactose, sucrose, gelatin, malt, rice, flour, chalk, silica gel, sodium stearate, glycerol monostearate, talc, sodium chloride; dried skim milk, glycerol, propylene, glycol, water, ethanol, and the like. The composition, if desired, can also contain minor amounts of wetting or emulsifying agents, like lipids.

Pharmaceutical or nutraceutical compositions can take the form of solutions, suspensions, emulsion, tablets, pills, capsules, powders, sustained-release formulations, and the like.

In particular in case of longer alkyl chain esters a lipid vehicle is advantageous. The compounds of the invention are light and oxygen sensitive. Therefore selection of an appropriate vehicle may depend on its ability to stabilize the compound of the invention. Such formulations are described e.g. in WO2011034551A2 (Boch et al., Pharmaceutical formulations comprising 9-cis-retinyl esters in a lipid vehicle). Exemplary formulations and dosages are described in WO2011034551A2 and in WO2013134867A1.

Suitable oral dosage forms include, for example, tablets, pills, sachets, or capsules of hard or soft gelatin, methylcellulose or of another suitable material easily dissolved in the digestive tract. Suitable nontoxic solid carriers can be used which include, for example, pharmaceutical grades of mannitol, lactose, starch, magnesium stearate, sodium saccharin, talcum, cellulose, glucose, sucrose, magnesium carbonate, and the like. (See, e.g., Remington "Pharmaceutical Sciences", 17 Ed., Gennaro (ed.), Mack Publishing Co., Easton, Pennsylvania, 1985.)

Methods of introduction include but are not limited to intradermal, intramuscular, intraperitoneal, intravenous, subcutaneous, intranasal, intraocular, epidural and oral routes. The agents can be administered by any convenient route such as, for example, by infusion or bolus injection, by absorption through epithelial or mucocutaneous linings (e.g., oral mucosa, rectal and intestinal mucosa), and the like, and can be administered together with other functionally active agents.

### Dosages

The doses of the agents can be carefully selected depending on the clinical status, condition and age of the subject, dosage form and the like.

For example the human effective dose can be calculated from the animal dose as described for example in Chapter V. (STEP 2: Human equivalent dose calculation) and in Table 1 of a 2005 July US FDA Guidelines. This indicates that, to give a value of a dose to start with in humans, animal doses shall be divided by a factor calculated based on body surface area, e.g. by a factor of 6.2 in body weight kilogramm doses between rats and humans, and a factor of 12.3 in body weight kilogramm doses between mice and humans.

Thus, at least an upper limit can be safely given based on animal models depending species.

Side effects depend on dosages and the compounds of the invention are contemplated to be favorable in this regard.

Just to give an illustrative example, and subject to trials, in the case of eye drops, an agent can be administered, for example, from about 0.01 mg, about 0.1 mg, or about 1 mg, to about 25 mg, to about 50 mg, to about 90 mg per single dose. Eye drops can be administered one or more times per day, as needed. In the case of injections, suitable doses can be, for example, about 0.0001 mg, about 0.001 mg, about 0.01 mg, or about 0.1 mg to about 10 mg, to about 25 mg, to about 50 mg, or to about 90 mg of the agent, one to four times per week. In other embodiments, about 1.0 to about 30 mg of agent can be administered one to three times per week or more in case of need more often, e.g. daily. Oral dosages may fall into the same order of magnitude, e.g. from about 1 mg or 5 mg to about 10 mg, to about 25 mg, to about 50 mg, or to about 100 mg of the agent in a subject, daily or one to 6 times per week.

### EXAMPLES

### Example 1: Chemical synthesis - synthesis of dihydro retinoids:

To confirm whether relative MS-MS signal detected at 303>207 m/z corresponds to 9CDHRA, the stereoselective synthesis of both enantiomers of 9-*cis*-13,14-dihydroretinoic acid was carried out following the previously described strategy based on a palladium-catalyzed Csp2-Csp2 Suzuki coupling (Pazos Y et al. 2001). Details of the stereocontrolled synthesis, purification and characterization of the (*R*)- and (*S*)-enantiomers of 9-*cis-*13,14-dihydroretinoic acid are provided below.

The preparation of the ethyl ester of (*R*)-9-cis-13,14-dihydroretinoic acid (*R*)-**4** was based on the Suzuki coupling of enantiopure trienyliodide **3** and boronic acid **2** (Scheme 1). The synthesis of **3** started with (*Z*)-stannyldienol **5** (Domínguez B. et al. 2000, Pazos Y et al. 1999) which was transformed into the benzothiazolyl allyl sulfide **6** by Mitsunobu reaction with the corresponding thiol and subsequently oxidized to sulfone **7** with H₂O₂ and a peroxymolybdate (VI) reagent (Schultz HS et al. 1963) at -10 °C. The Julia-Kocienski olefination (Blakemore PR. 2002; Aïssa C 2009) was performed using a slight excess of base (NaHMDS, 1.15 equiv.) and 1.7 equivalents of enantiopure aldehyde (*R*)-**8**. (Moise AR et al. 2008, Leonard J. et al. 1995) As anticipated from previous findings on the stereochemical outcome of the reactions of allylsulfones and aldehydes (Sorg A et al. 2005, Vaz B et al., 2005), the newly formed olefin of trienyl ester (*R*)-**9** is of *Z*-geometry (which was confirmed by NOE experiments). Treatment of the precursor stannane with a solution of iodine in CH₂Cl₂ produced the iodide (*R*)*-***3** via Sn-I exchange and iodine-promoted isomerization of the 9*Z*,11*Z* diene to the desired 9*Z*,11*E* geometric isomer (as confirmed by NOE experiments). 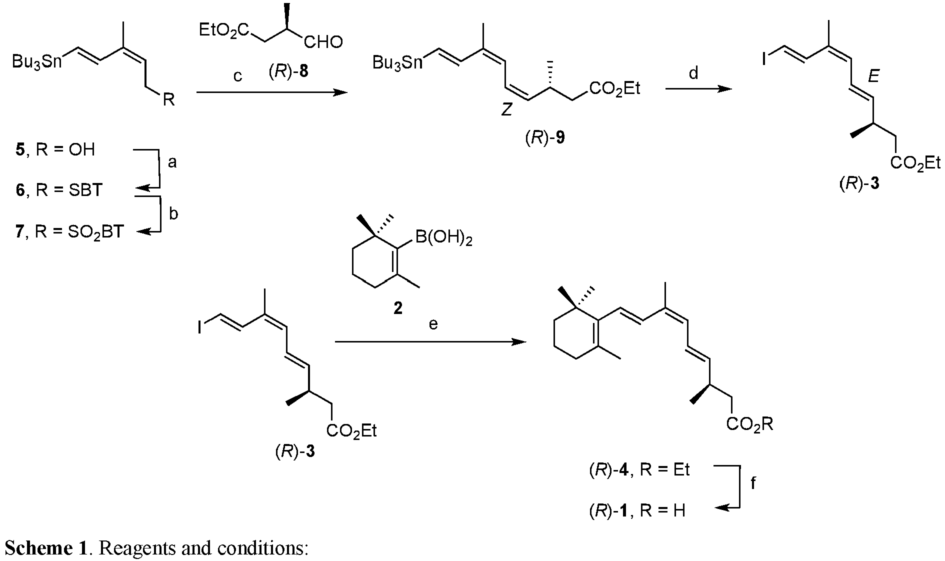
(a) PPh₃, BTSH, DIAD, CH₂Cl₂, 2h (98%).
(b) (NH₄)₆MO₇O₂₄·4H₂O, 30% H₂O₂, EtOH, -10 °C, 17h (66%).
(c) NaHMDS, THF, -78 °C, 30 min (93%).
(d) I₂, CH₂Cl₂, 25 °C, 30 min.
(e) Pd(PPh₃)₄, 10% aq. TlOH, THF, 25 °C, 3h (42%).
(f) 2M KOH, MeOH, 80 °C, 45 min (84%).

The Suzuki reaction of freshly prepared boronic acid **2** and dienyl iodide (*R*)-**3** using Pd(PPh₃)₄ as catalyst and 10% aq. TlOH as base in THF at ambient temperature, followed by immediate work-up afforded ethyl (*R*)-9-*cis*-13,14-dihydroretinoate (*R*)-**4** in 78% yield.

(From (*R*)-**4** the corresponding carboxylic acid (*R*)-**1** can be provided in 84% yield without detectable loss of stereochemical integrity.

Following the general scheme, enantiomer (*S*)-**4** can also be prepared with similar efficiency.

### Preparation and Characterization of Compounds - General procedures

Solvents were dried according to published methods and distilled before use. All other reagents were commercial compounds of the highest purity available. Unless otherwise indicated all reactions were carried out under argon atmosphere, and those not involving aqueous reagents were carried out in oven-dried glassware. Analytical thin layer chromatography (TLC) was performed on aluminum plates with Merck Kieselgel 60F254 and visualized by UV irradiation (254 nm) or by staining with an ethanolic solution of phosphomolibdic acid. Flash column chromatography was carried out using Merck Kieselgel 60 (230-400 mesh) under pressure. Electron impact (EI) mass spectra were obtained on a Hewlett-Packard HP59970 instrument operating at 70 eV. Alternatively an APEX III FT-ICR MS (Bruker Daltonics), equipped with a 7T actively shielded magnet was used and ions were generated using an Apollo API electrospray ionization (ESI) source, with a voltage between 1800 and 2200 V (to optimize ionization efficiency) applied to the needle, and a counter voltage of 450 V applied to the capillary. For ESI spectra samples were prepared by adding a spray solution of 70:29.9:0.1 (v/v/v) CH₃OH/water/formic acid to a solution of the sample at a v/v ratio of 1 to 5% to give the best signal-to-noise ratio. High Resolution mass spectra were taken on a VG Autospec instrument. ¹H NMR spectra were recorded in C₆D₆ and acetone-d₆ at ambient temperature on a Bruker AMX-400 spectrometer at 400 MHz with residual protic solvent as the internal reference.

### (2Z,4E)-1-(Benzothiazol-2-yl)sulfanyl-5-(tri-n-butylstannyl)-3-methylpenta-2,4-diene (6).

A solution of (2*Z*,4*E*)-3-methyl-5-(tributylstannyl)penta-2,4-dien-1-ol (1.0 g, 2.58 mmol), 2-mercaptoben-zothiazol (0.65 g, 3.87 mmol) and PPh₃ (1.10 g, 4.21 mmol) in THF (14 mL) was stirred for 5 min at 0 °C. A solution of DIAD (0.77 mL, 3.87 mmol) in THF (5 mL) was added dropwise and the mixture was stirred for 30 min at 25 °C. The solvent was removed and the residue was purified by column chromatography (C18-silicagel, CH₃CN) to afford 1.11 g (78%) of a colorless oil identified as (2*Z*,4*E*)-1-(Benzothiazol-2-yl)sulfanyl-5-(tri-*n*-bu-tylstannyl)-3-methylpenta-2,4-diene **6**. **¹H NMR** (400 MHz, C₆D₆) δ 8.04 (d, *J* = 8.2 Hz, 1H, CH), 7.46 (d, *J* = 19.2 Hz, ³*J*_{SnH} = 71.2 Hz, 1H, CH), 7.34 (d, *J* = 8.0 Hz, 1H, CH), 7.21 (ddd, *J* = 8.3, 7.3, 1.2 Hz, 1H, CH), 7.01 (ddd, *J* = 8.3, 7.4, 1.1 Hz, 1H, CH), 6.66 (d, *J* = 19.2 Hz, ²*J*_{SnH} = 71.2 Hz, 1H, CH), 5.65 (t, *J* = 8.1 Hz, 1H, CH), 4.37 (d, *J* = 8.2 Hz, 2H, CH₂), 1.86 (s, 3H, CH₃), 1.8-1.6 (m, 6H, CH₂), 1.5-1.4 (m, 6H, CH₂), 1.1-1.0 (m, 15H, CH₃ + CH₂) ppm. **¹³C NMR** (100 MHz, C₆D₆) δ 166.4 (s), 153.7 (s), 142.6 (d), 138.6 (s), 135.7 (s), 132.0 (d), 125.9 (d), 124.1 (d), 122.1 (d), 121.7 (d), 121.0 (d), 30.2 (t), 29.3 (t, 3x), 27.6 (t, 3x), 19.8 (q), 13.8 (q, 3x), 9.7 (t, 3x) ppm; **IR** (NaCl) ν 2955 (s, C-H), 2923 (s, C-H), 2870 (m, C-H), 2850 (m, C-H), 1460 (s), 1427 (s) cm⁻¹. **HRMS** (ESI⁺) *m*/*z* calcd for C₂₅H₄₀NS₂¹²⁰Sn: 538.1620; found: 538.1621.

### (2Z,4E)-1-(Benzothiazol-2-yl)sulfonyl-5-(tri-n-butylstannyl)-3-methylpenta-2,4-diene (7).

To a solution of (2*Z*,4*E*)-1-(benzothiazol-2-yl)sulfanyl-5-(tri-*n*-butylstannyl)-3-methylpenta-2,4-diene **6** (0.48 g, 0.89 mmol) in EtOH (9 mL), at -10 °C, was added a solution of (NH₄)₆Mo₇O₂₄·4H₂O, (0.44 g, 0.36 mmol) in aqueous hydrogen peroxide (35%, 7.7 mL, 89.1 mmol). After stirring for 17 h at -10 °C, the mixture was quenched with H₂O and extracted with Et₂O (3x). The combined organic layers were washed with brine (3x) and dried (Na₂SO₄), and the solvent was removed. The residue was purified by chromatography (C18-silicagel, MeOH) to afford 0.33 g (66%) of a colorless oil identified as (2*Z*,4*E*)-1-(benzothiazol-2-yl)sulfonyl-5-(tri-n-butylstannyl)-3-methylpenta-2,4-diene **7**.**¹H NMR** (400 MHz, C₆D₆) δ 8.10 (d, *J* = 8.2 Hz, 1H, CH), 7.18 (d, *J* = 19.2 Hz, 1H, CH), 7.16 (t, *J* = 7.5 Hz,1H, CH), 7.12 (t, *J* = 8.1 Hz, 1H, CH), 6.98 (t, *J* = 7.7 Hz, 1H, CH), 6.59 (d, *J* = 19.2 Hz, ²*J*_{SnH} = 68.2 Hz, 1H, CH), 5.43 (t, *J* = 8.0 Hz, 1H, CH), 4.32 (d, *J* = 8.1 Hz, 2H, CH₂), 1.8-1.6 (m, 9H, CH₃ + CH₂), 1.6-1.4 (m, 6H, CH₂), 1.1-1.0 (m, 15H, CH₃ + CH₂) ppm. **¹³C NMR**(100 MHz, C₆D₆) δ 167.1 (s), 152.9 (s), 143.1 (s), 141.7 (d), 136.9 (s), 134.4 (d), 127.3 (d), 127.1 (d), 125.0 (d), 122.1 (d), 112.0 (d), 53.3 (t), 29.3 (t, 3x), 27.5 (t, 3x), 20.0 (q), 13.8 (q, 3x), 9.6 (t, 3x) ppm; **IR** (NaCl) v 2955 (s, C-H), 2923 (s, C-H), 2850 (m, C-H), 1467 (m), 1333 (s), 1151 (s) cm⁻¹. **HRMS** (ESI⁺) *m*/*z* calcd for C₂₅H₃₉NNaO₂S₂¹²⁰Sn: 592.1338; found: 592.1334. **UV** (MeOH) λₘₐₓ 239 nm.

### (3S,4Z,6Z,8E)-Ethyl 3,7-Dimethyl-9-(tri-n-butylstannyl)nona-4,6,8-trienoate ((S)-9).

A cooled (-78 °C) solution of (2*Z*,4*E*)-1-(benzothiazol-2-yl)sulfonyl-5-(tri-*n*-butylstannyl)-3-methylpenta-2,4-diene (0.115 g, 0.20 mmol) in THF (9 mL) was treated with NaHMDS (0.23 mL, 1M in THF, 0.23 mmol). After stirring for 30 min at this temperature, a solution of (*S*)-ethyl 3-methyl-4-oxobutanoate (0.044 g, 0.30 mmol) in THF (4.5 mL) was added and the resulting mixture was stirred for 1h at -78 °C and 3h letting the temperature reach to rt. Et₂O and water were added at low temperature and the mixture was warmed up to room temperature. It was then diluted with Et₂O and the layers were separated. The aqueous layer was extracted with Et₂O (3x), the combined organic layers were dried (Na₂SO₄) and the solvent was removed. The residue was purified by column chromatography (C-18 silica gel, MeOH) to afford 0.94 g (93%) of a pale yellow oil identified as (3*S*,4*Z*,6*Z*,8*E*)-ethyl 3,7-dimethyl-9-(tri-*n*-butylstannyl)nona-4,6,8-trienoate (S)-9. **[α]²⁸_{D}** +11.5° (c 1.22, MeOH). **¹H NMR** (400 MHz, C₆D₆) δ 7.60 (d, *J* = 19.1 Hz, ³*J*_{SnH} = 65.1 Hz, 1H, CH), 6.85 (t, *J* = 11.4 Hz, 1H, CH), 6.64 (d, *J* = 19.2 Hz, ²*J*_{SnH} = 71.9 Hz 1H, CH), 6.58 (d, *J* = 11.9 Hz, 1H, CH), 5.31 (t, *J* = 10.4 Hz, 1H, CH), 4.03 (q, *J* = 7.1 Hz, 2H, CH₂), 3.5-3.3 (m, 1H, CH), 2.4-2.2 (m, 2H, CH₂), 2.02 (s, 3H, CH₃), 1.8-1.6 (m, 6H, CH₂), 1.6-1.4 (m, 6H, CH₂), 1.2-0.9 (m, 18H, CH₃ + CH₂) ppm. **¹³C NMR** (100 MHz, C₆D₆) δ 171.3 (s), 143.2(d), 135.8 (d), 135.4 (s), 130.7 (d), 123.8 (d), 122.9 (d), 59.8 (t), 41.8 (t), 29.3 (t, 3x), 29.2 (d), 27.5 (t, 3x), 20.7 (q), 20.3 (q), 14.0 (q), 13.7 (q, 3x), 9.6 (t, 3x) ppm. **IR** (NaCl) ν 2957 (s, C-H), 2924 (s, C-H), 2871 (m, C-H), 2851 (m, C-H), 1737 (s, C=O), 1459 (m), 1160 (m) cm⁻¹. **HRMS** (ESI⁺) *m*/*z* calcd for C₂₅H₄₆NaO₂¹²⁰Sn: 521.2416; found: 521.2408. **UV** (MeOH) λₘₐₓ 285 nm.

**(3*R*,4*Z*,6*Z*,8*E*)-Ethyl 3,7-Dimethyl-9-(tri-n-butylstannyl)nona-4,6,8-trienoate ((*R*)-9). [α]²⁹_{D}** -10.3° (c 1.25, MeOH).

### (3R,4E,6Z,8E)-Ethyl 9-Iodo-3,7-dimethylnona-4,6,8-trienoate ((R)-3).

To a solution of (3*R*,4*Z*,6*Z*,8*E*)-ethyl 3,7-dimethyl-9-(tri-*n*-butylstannyl)nona-4,6,8-trienoate (**R**)-**9** (0.060 g, 0.121 mmol) in CH₂Cl₂ (5.3 mL) was added dropwise iodine (0.046 g, 0.182 mmol) in CH₂Cl₂ (2.8 mL) and the resulting mixture was stirred for 30 min at 25 °C. A saturated Na₂S₂O₃ solution was added and the reaction mixture was extracted with Et₂O (3x), the combined organic layers were dried (Na₂SO₄) and the solvent was removed. The residue was purified by column chromatography (silica gel, 97:3 hexane/Et₃N) to afford 0.037 g (92%) of a pale yellow oil identified as (3*R*,4*E*,6*Z*,8*E*)-ethyl 9-iodo-3,7-dimethylnona-4,6,8-trienoate (*R*)-3. **[α]²⁴_{D}** +14.8° (*c* 0.74, MeOH). **¹H NMR** (400 MHz, C₆D₆) δ 7.65 (d, *J* = 14.5 Hz, 1H, CH), 6.28 (dd, *J* = 14.9, 11.3 Hz, 1H, CH), 6.05 (d, *J* = 14.5 Hz, 1H, CH), 5.64 (d, *J* = 11.1 Hz, 1H, CH), 5.44 (dd, *J* = 15.0, 7.8 Hz, 1H, CH), 3.95 (q, *J* = 7.1 Hz, 2H, CH₂), 2.64 (dt, *J* = 14.1, 7.0 Hz, 1H, CH), 2.15 (dd, *J* = 14.9, 7.1 Hz, 1H, CH), 2.06 (dd, *J* = 14.9, 7.3 Hz, 1H, CH), 0.97 (t, *J* = 7.2 Hz, 3H, CH₃), 0.88 (d, *J* = 6.8 Hz, 3H, CH₃) ppm. **¹³C NMR** (100 MHz, C₆D₆) δ 171.9 (s), 142.7 (d), 140.4 (d), 132.7 (s), 130.9 (d), 124.6 (d), 78.5 (d), 60.5 (t), 41.8 (t), 34.5 (d), 20.4 (q), 19.9 (q), 14.7 (q) ppm. **IR** (NaCl) ν 2972 (m, C-H), 2932 (m, C-H), 1731 (s, C=O), 1666 (m), 1180 (m) cm⁻¹. **HRMS** (ESI⁺) *m*/*z* calcd for C₁₃H₁₉INaO₂: 357.0322; found: 357.0316. **UV** (MeOH) λₘₐₓ 275 nm.

### (3S,4F,6Z,8E)-Ethyl 9-Iodo-3,7-dimethylnona-4,6,8-trienoate ((S)-3). [α]²³_{D} -16.7° (c 0.72, MeOH).

**(9*Z*,13*R*)-Ethyl 13,14-Dihydroretinoate ((*R*)-4).** To a solution of (3*R*,4*E*,6*Z*,8*E*)-ethyl 9-iodo-3,7-dime-thylnona-4,6,8-trienoate (*R*)-**3** (0.036 g, 0.107 mmol) in THF (2.3 mL) was added Pd(PPh₃)₄ (0.013 g, 0.011 mmol). After 5 min at room temperature, 2,6,6-trimethylcyclohex-1-enylboronic acid **2** (0.027 g, 0.161 mmol) was added in one portion followed by TlOH (10% aqueous solution, 0.75 mL, 0.407 mmol). After stirring for 3 h at 25 °C, Et₂O was added and the reaction mixture was filtered through a short pad of Celite^{®}. The filtrate was washed with NaHCO₃ (sat) and the organic layer was dried (Na₂SO₄) and the solvent was removed. The residue was purified by column chromatography (silica gel, 97:3 hexane/Et₃N) to afford 0.028 g (78%) of a pale yellow oil identified as (9*Z*,13*R*)-ethyl 13,14-dihydroretinoate (*R*)-**4. [α]²³_{D}** +14.2° (c 0.48, MeOH). **¹H NMR** (400 MHz, C₆D₆) δ 6.90 (d, *J* = 16.0 Hz, 1H, CH), 6.71 (dd, *J* = 14.9, 11.2 Hz, 1H, CH), 6.28 (d, *J* = 16.0 Hz, 1H, CH), 5.96 (d, *J* = 11.1 Hz, 1H, CH), 5.51 (dd, *J* = 15.0, 7.8 Hz, 1H, CH), 3.94 (q, *J* = 7.2 Hz, 2H, CH₂), 2.77 (dt, *J* = 14.1, 7.1 Hz, 1H, CH), 2.21 (dd, *J* = 14.8, 7.3 Hz, 1H, CH), 2.11 (dd, *J* = 14.8, 7.2 Hz, 1H, CH), 1.95 (t, *J* = 6.1 Hz, 2H, CH₂), 1.90 (s, 3H), 1.79 (s, 3H), 1.66 - 1.52 (m, 2H), 1.52 - 1.41 (m, 2H), 1.11 (s, 6H), 0.95 (t, *J* = 7.1 Hz, 3H, CH₃), 0.94 (d, *J* = 6.8 Hz, 3H, CH₃) ppm. **¹³C NMR** (100 MHz, C₆D₆) 171.4 (s), 138.3 (s), 137.6 (d), 132.7 (s), 130.6 (d), 129.1 (d), 129.0 (s), 127.9 (d), 124.8 (d), 59.8 (t), 41.6 (t), 39.6 (t), 34.2 (s), 34.1 (d), 33.0 (t), 28.9 (q, 2x), 21.8 (q), 20.4 (q), 20.1 (q), 19.5 (t), 14.1 (q) ppm. **IR** (NaCl) v 2961 (s, C-H), 2929 (s, C-H), 2866 (m, C-H), 1737 (s, C=O), 1455 (m), 1372 (m), 1167 (m) cm⁻¹. **HRMS** (ESI⁺) *m*/*z* calcd for C₂₂H₃₅O₂: 331.2632; found: 331.2625. **UV** (MeOH) λₘₐₓ 287 nm (ε = 20000 L·mol⁻¹·cm⁻¹).

### (9Z,13S)-Ethyl 13,14-dihydroretinoate ((S)-4). [α]²²_{D} -15.5° (c 0.51, MeOH).

**(9Z,13R)-13,14-Dihydroretinoic Acid ((R)-1).** To a solution of (9*Z*,13*R*)-ethyl 13,14-dihydroretinoate (*R*)-**4** (0.023 g, 0.069 mmol) in MeOH (4.7 mL) was added KOH (2M aqueous solution, 1.1 mL, 2.27 mmol) and the reaction mixture was stirred for 45 min at 80 °C. After letting the reaction cool down to room temperature, CH₂Cl₂ and brine were added and the layers were separated. The aqueous layer was washed with H₂O (3x). The combined aqueous layers were acidified with HCl 10% and extracted with CH₂Cl₂ (3x). The combined organic layers were dried (Na₂SO₄) and the solvent was removed. The residue was purified by column chromatography (silica gel, gradient from 95:5 to 90:10 CH₂Cl₂/MeOH) to afford 0.017 g (84%) of a pale yellow oil identified as (9*Z*,13*R*)-13,14-dihydroretinoic acid (*R*)-**1**. [**α]²²_{D}** +7.1° (*c* 0.67, MeOH). **¹H NMR** (400 MHz, acetone-d₆): δ= 6.66 (d, *J* = 16.0 Hz, 1H), 6.60 (dd, *J* = 15.0, 11.2 Hz, 1H), 6.18 (d, *J* = 16.0 Hz, 1H), 5.93 (d, *J* = 11.1 Hz, 1H), 5.65 (dd, *J* = 15.0, 7.5 Hz, 1H), 2.73 (dt, *J* = 13.9, 7.0 Hz, 1H), 2.4 - 22 (m, 2H), 2.1 - 2.0 (m, 1H), 1.91 (s, 3H), 1.72 (s, 3H), 1.7 - 1.6 (m, 2H), 1.5 - 1.4 (m, 2H), 1.08 (d, *J* = 6.8 Hz, 3H), 1.03 (s, 6H) ppm. **¹³C NMR** (100 MHz, acetone-d₆) □?? 173.5 (s), 138.9 (s), 138.8 (d), 133.4 (s), 131.1 (d), 129.8 (s), 129.7 (d), 128.5 (d), 125.4 (d), 41.8 (t), 40.3 (t), 34.9 (s), 34.6 (d), 33.6 (t), 29.4 (q), 22.1 (q), 20.7 (q), 20.6 (q), 20.0 (t) ppm. **IR** (NaCl) v 2957 (s, C-H), 2923 (s, C-H), 2855 (m, C-H), 1709 (s, C=O), 1446 (m), 1290 (m) cm⁻¹. **HRMS** (ESI⁺) *m*/*z* calcd for C₂₀H₃₁O₂: 303.2319; found: 303.2313. **UV** (MeOH) λₘₐₓ 289 nm (ε = 17600 L·mol⁻¹·cm⁻¹).

### (9Z,13S)-13,14-Dihydroretinoic Acid ((S)-1). [α]²⁴_{D} -6.9° (c 0.26, MeOH).

### Example 2.1: Synthesis of (R)-9-cis-13,14-dihydroretinol and (R)-9-cis-13,14-dihydroretinol acetate

(*R*)-9-*cis*-13,14-**dihydroretinol** was synthesized by DIBAL-H reduction of **(9Z,13R)-Ethyl 13,14-Dihydroretinoate ((*R*)-4)** in THF at -78 °C in 95% yield (Scheme 1).
(*R*)-9-*cis*-13,14-dihydroretinyl acetate was prepared in 86% yield by acetylation of (*R*)-9-*cis*-13,14-dihydroretinol with acetic anhydride and pyridine in the presence of dimethylaminopyridine (DMAP).

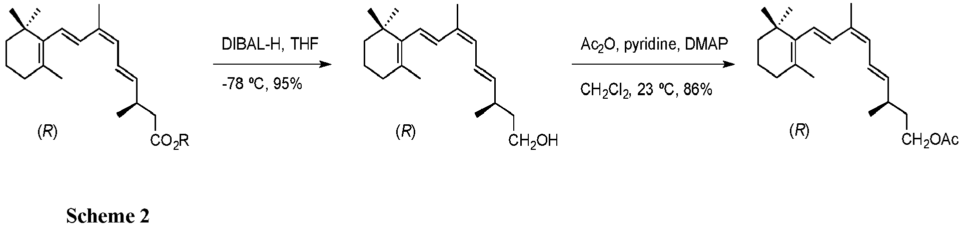

### (3R,4E,6Z,8E)-3,7-Dimethyl-9-(2,6,6-trimethylcyclohex-1-en-1-yl)nona-4,6,8-trien-1-ol ((R)-10)

To a cooled (-78 °C) solution of ethyl (3*R*,4*E*,6*Z*,8*E*)-3,7-dimethyl-9-(2,6,6-trimethylcyclohex-1-en-1-yl)nona-4,6,8-trienoate [ethyl (*R*)-9-*cis*-13,14-dihydroretinoate, (R)-4] (32.0 mg, 0.097 mmol) in THF (1.0 mL), DIBAL-H (0.242 mL, 0.242 mmol, 1.0 M in hexanes) was added and the resulting mixture was stirred for 30 min at -78 °C. The mixture was allowed to warm up to -20 °C in 2.5 h. H₂O was added and the mixture was extracted with Et₂O (3x). The combined organic layers were dried (Na₂SO₄) and the solvent removed. Flash chromatography of the residue (silica gel, first neutralized with 98:2 hexane/Et₃N, then gradient from 95:5 hexane/EtOAc to EtOAc) afforded (3*R*,4*E*,6*Z*,8*E*)-3,7-dimethyl-9-(2,6,6-trimethylcyclohex-1-en-1-yl)nona-4,6,8-trien-1-ol (26.5 mg, 95%) as a colorless oil. **¹H-NMR** (400.13 MHz, C₆D₆): δ 6.93 (d, *J* = 16.0 Hz, 1H), 6.69 (dd, *J* = 14.9, 11.1 Hz, 1H), 6.29 (d, *J* = 16.0 Hz, 1H), 6.01 (d, *J* = 11.1 Hz, 1H), 5.46 (dd, *J* = 15.0, 8.3 Hz, 1H), 3.35 (t, *J* = 6.6 Hz, 2H), 2.27 (dt, *J* = 14.0, 6.7 Hz, 1H), 1.98 - 1.92 (m, 2H), 1.93 (s, 3H), 1.79 (s, 3H), 1.63 - 1.52 (m, 2H), 1.51 - 1.43 (m, 2H), 1.36 (q, *J* = 6.7 Hz, 2H), 1.11 (s, 6H), 0.91 (d, *J* = 6.7 Hz, 3H) ppm. **¹³C-NMR** (100.62 MHz, C₆D₆): δ 140.0 (d), 138.6 (s), 132.5 (s), 130.9 (d), 129.6 (d), 129.2 (s), 128.1 (d), 124.9 (d), 60.9 (t), 40.2 (t), 39.9 (t), 34.5 (s), 34.3 (d), 33.2 (t), 29.21 (q), 29.18 (q), 22.0 (q), 20.9 (q), 20.7 (q), 19.7 (t) ppm **HRMS** (ESI⁺): Calcd. for C₂₀H₃₃O ([M+H]⁺), 289.2526; found, 289.2526. **IR** (NaCl): v 3338 (m, O-H), 2957 (s, C-H), 2926 (s, C-H), 2861 (m, C-H), 1454 (m), 1375 (m), 965 (m) cm⁻¹.

### (3R,4E,6Z,8E)-3,7-Dimethyl-9-(2,6,6-trimethylcyclohex-1-en-1-yl)nona-4,6,8-trien-1-yl Acetate ((R)-11)

To a solution of (3*R*,4*E*,6*Z*,8*E*)-3,7-dimethyl-9-(2,6,6-trimethylcyclohex-1-en-1-yl)nona-4,6,8-trien-1-ol (5.7 mg, 0.020 mmol) in CH₂Cl₂ (0.5 mL) were sequentially added Ac₂O (0.009 mL, 0.099 mmol), pyridine (0.008 mL, 0.099 mmol) and DMAP (0.5 mg, 0.004 mmol). The resulting mixture was stirred for 1 h at 25 °C. Then, Et₂O was added and the resulting solution was washed with a saturated aqueous solution of CuSO₄ (2x). The organic layer was dried (Na₂SO₄) and evaporated. Flash chromatography of the residue (silica gel, gradient from hexane to 85:15 hexane/EtOAc) afforded (3*R*,4*E*,6*Z*,8*E*)-3,7-dimethyl-9-(2,6,6-trimethylcyclohex-1-en-1-yl)nona-4,6,8-trien-1-yl acetate (5.6 mg, 86%) as a colorless oil. **¹H-NMR** (400.13 MHz, C₆D₆): δ 6.92 (d, *J* = 16.0 Hz, 1H), 6.67 (dd, *J* = 15.0, 10.8 Hz, 1H), 6.29 (d, *J* = 16.0 Hz, 1H), 5.98 (d, *J* = 11.1 Hz, 1H), 5.39 (dd, *J* = 15.0, 8.2 Hz, 1H), 4.11 - 3.92 (m, 2H), 2.16 (dq, *J* = 14.4, 7.2 Hz, 1H), 1.99 - 1.90 (m, 2H), 1.93 (s, 3H), 1.79 (d, *J* = 1.0 Hz, 3H), 1.67 (s, 3H), 1.62 - 1.53 (m, 2H), 1.50 - 1.41 (m, 4H), 1.11 (s, 6H), 0.86 (d, J = 6.8 Hz, 3H) ppm. **¹³C-NMR** (100.62 MHz, C₆D₆): δ 170.0 (s), 138.9 (d), 138.6 (s), 132.8 (s), 130.9 (d), 129.4 (d), 129.3 (s), 128.2 (d), 125.2 (d), 62.7 (t), 39.9 (t), 36.0 (t), 34.5 (s), 34.4 (d), 33.2 (t), 29.19 (q), 29.18 (q), 22.0 (q), 20.74 (q), 20.70 (q), 20.6 (q), 19.7 (t) ppm. **HRMS** (ESI⁺): Calcd. for C₂₂H₃₅O₂ ([M+H]⁺), 331.2632; found, 331.2621. **IR** (NaCl): v 2957 (s, C-H), 2926 (s, C-H), 2860 (m, C-H), 1741 (s, C=O), 1455 (m), 1365 (m), 1238 (s), 966 (m) cm⁻¹.

### Example 2.2: Synthesis of 9-cis-β,β-carotene (Scheme 3, including added numeration)

9-cis-Ethylretinoate (III). To a cold (0 °C) solution of ethyl (E)-4-(diethoxyphosphoryl)-3-methylbut-2-en-oate (II) (0.227 g, 1.10 mmol) in THF (2.0 mL) was added nBuLi (0.63 mL, 1.00 mmol, 1.6 M in hexane) and DMPU (0.15 mL, 1.24 mmol). After stirring for 1 h, the reaction was cooled down to -78 °C and a solution of (2Z,4E)-3-methyl-5-(2,6,6-trimethylcyclohex-1-en-1-yl)penta-2,4-dienal (I) (0.100 g, 0.46 mmol) in THF (2.5 mL) was added and stirred for 2 h. Water was added and the mixture was extracted with Et2O (3x). The combined organic layers were dried (Na2SO4) and the solvent was evaporated. The crude was purified by column chromatography (silica gel, 95:5 hexane/EtOAc) to afford 0.144 g (96%) of a yellow solid identified as ethyl 9-cis-retinoate (III).

**9-cis-Retinol** (IV). To a cold (-78 °C) solution of ethyl 9-cis-retinoate (III) (0.154 g, 0.47 mmol) in THF (2.3 mL) was added DIBAL-H (1.08 mL, 1.08 mmol, 1.0 M in toluene) and the reaction was stirred for 2 h. Then water was added and the mixture was extracted with EtOAc (3x). The combined organic layers were dried (Na2SO4) and the solvent was evaporated. The crude was purified by column chromatography (silica gel, gradient from 95:5 to 80:20 hexane/EtOAc) to afford 0.080 g (60%) of a yellow oil identified as 9-cis-retinol (IV).

9-cis-retinal (V). To a solution of 9-cis-retinol (IV) (65 mg, 0.23 mmol) in CH2Cl2 (9.1 mL) were added MnO2 (197 mg, 2.27 mmol) and Na2CO3 (240 mg, 2.27 mmol) and the reaction was stirred for 1.5 h at 25 °C. The mixture was filtered through a pad of Celite^{®} washing with CH2Cl2. The solvent was evaporated, affording 38 mg (58%) of a yellow oil identified as 9-cis-retinal (V).

(2E,4E,6E,8E)-Bromo-(3,7-dimethyl-9-(2,6,6-trimethylcyclohex-1-en-1-yl)nona-2,4,6,8-tetraen-1-yl)triphenyl-phosphane (VII). To a solution of Vitamin A / all-trans-retinol (VI) (1.370 g, 4.78 mmol) in MeOH (2.7 mL) was added PPh3 (1.443 g, 5.5 mmol) and a solution of HCl (1.4 mL, 4 M in dioxane) and the reaction was stirred for 2 h at 25 °C. Then, the mixture was poured into water and extracted with Et2O (2x). The aqueous layer was extracted with AcOEt (3x) and the combined organic layers were dried (Na2SO4) and the solvent was evaporated. The light orange residue was triturated three times with EtOAc to afford 1.390 g (52%) of a yellow foam identified as (2E,4E,6E,8E)-bromo-(3,7-dimethyl-9-(2,6,6-trimethylcyclohex-1-en-1-yl)nona-2,4,6,8-tet-raen-1-yl)triphenyl-phosphane VII. 1H NMR (400.16 MHz, CDCl3): δ 7.88 (m, 6H), 7.78 (m, 3H), 7.69 (m, 6H), 6.56 - 6.42 (m, 1H), 6.24 - 5.94 (m, 4H), 5.39 (d, J = 10.6 Hz, 1H), 4.99 (m, 2H), 2.01 (m, 2H), 1.92 (s, 3H), 1.69 (s, 6H), 1.55 - 1.40 (m, 4H), 1.01 (s, 6H) ppm. 13C NMR (101.63 MHz, CDCl3): δ 143.7 (s), 137.7 (s), 137.5 (s), 137.4 (d), 134.9 (d), 134.8 (d), 132.0 (d), 129.5 (d), 128.45 (d) 127.5 (d), 126.4 (d), 118.7 (s), 117.8 (s), 114.1 (d), 39.6 (t), 34.2 (s), 33.0 (t), 28.9 (q, 2x), 25.4 (t), 24.9 (t), 19.2 (q), 13.1 (q), 12.8 (q) ppm. HRMS (ESI⁺): Cald. for C38H44P+ ([M-Cl]+), 531.3175; found, 531.3166.

9-cis-β,β-carotene (VIII). To a solution of phosphonium salt (VII) (24.9 mg, 0.044 mmol) in THF (0.20 mL) at -78 °C was added nBuLi (0.027 mL, 0.044 mmol, 1.6 M in hexane) and the mixture was stirred for 30 min. Then a solution of 9-cis-retinal V (9.0 mg, 0.031 mmol) in THF (0.13 mL) was added and the mixture was stirred for 1 h at -78 °C and 30 min at 25 °C. Water was added and the mixture was extracted with Et2O (3x). The combined organic layers were washed with brine (2x) and dried (Na2SO4) and the solvent was evaporated. The residue was purified by column chromatography (C18-silica gel, CH3CN) to afford 11.3 mg (67%) of a red foam identified as 9-cis-β,β-carotene (VIII). The spectroscopic data are identical to those previously published (LIT).

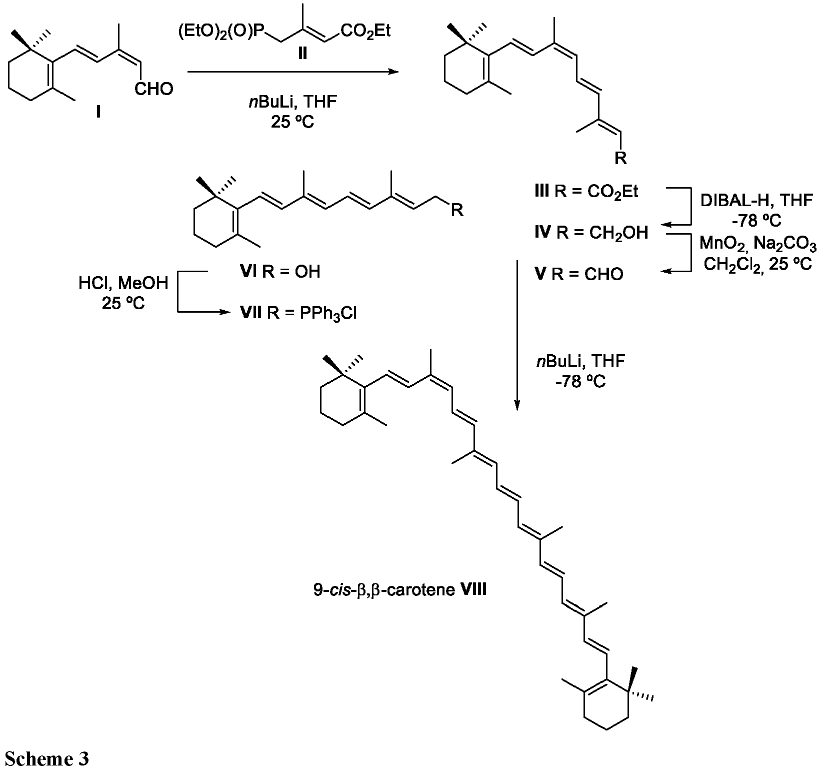

### Example 2.3: Synthesis of (R)-9-cis-13,14-dihydro-β,β-carotene (Scheme 4 including numeration)

(Z)-3-Iodo-3-methylprop-2-en-1-ol (X). CuI (0.34 g, 1.78 mmol) was added to a solution of propargylic alcohol (IX) (1.00 g, 11.84 mmol) in Et2O (13.2 mL). A solution of MeMgBr (17.8 mL, 53.51 mmol, 3 M in Et2O) was added at -20 °C. After stirring at this temperature for 2 h, the solution was allowed to reach 25 °C and further stirred for 12 h. A solution of I2 (9.10 g, 35.67 mmol) in Et2O (40.0 mL) was added at 0 °C and the cooling bath was removed. After stirring at 25 °C for 24 h, the resulting mixture was cooled down to 0 °C and ice water was added. The organic layer was washed with a saturated aqueous solution of Na2 S2O4 (3x) and then filtered through a pad of Celite^{®}. The residue was purified by distillation (0.2 mmHg, 60 °C) to afford 2.10 g (60%) of a yellow oil identified as (Z)-3-iodo-3-methylprop-2-en-1-ol (X). 1H-NMR (400.16 MHz, C6D6): δ 5.49 (s, 1H), 3.90 (s, 2H), 1.55 (s, 3H) ppm. 13C-NMR (101.63 MHz, C6D6): δ 146.6 (s), 74.1 (d), 67.5 (t), 20.9 (q) ppm. HRMS (ESI⁺): Cald. for C4H8IO ([M+H]⁺), 198.9609; found, 198.9614.

(Z)-3-Iodo-2-methylacrylaldehyde (XI). To a solution of (Z)-3-iodo-3-methylprop-2-en-1-ol (IIX) (0.21 g, 1.06 mmol) in CH2Cl2 (53.0 mL) was added MnO2 (0.93 g, 10.65 mmol) and Na2CO3 (1.13 g, 10.65 mmol) and the reaction was stirred for 1 h at 25 °C. Then, the mixture was filtered through a pad of Celite^{®} washing with CH2Cl2. The solvent was evaporated to afford 0.15 g (73%) of a yellow oil identified as (Z)-3-iodo-2-methylacrylaldehyde (XI).

2-(1E,3Z)-4-Iodo-3-methylbuta-1,3-dien-1-yl)-1,3,3-trimethylcyclohex-1-ene (XIII). To a cold (-30 °C) solution of phosphonium salt (XII) (150 mg, 0.31 mmol) in THF (2.5 mL), nBuLi (2.5 mL, 0.36 mmol, 2.36 M in hexanes) was added and the mixture was stirred at 0 °C for 45 min. After cooling down to -30 °C, a solution of (Z)-3-iodo-2-methylacrylaldehyde (XI) (70 mg, 0.36 mmol) in THF (2.5 mL) was added and the reaction mixture was stirred for 1.5 h. Water (5 mL) was added and the mixture was extracted with hexane (3x). The organic extracts were dried (Na2SO4) and the solvent was evaporated. The residue was purified by column chromatography (C18-silica gel, CH3CN) to afford 65 mg (64%) of a yellow oil identified as 2-(1E,3Z)-4-iodo-3-methylbuta-1,3-dien-1-yl)-1,3,3-trimethylcyclohex-1-ene (XIII). 1H-NMR (400 MHz, C6D6): δ 6.80 (d, J = 16.1 Hz, 1H), 6.32 (d, J = 16.1 Hz, 1H), 5.80 (s, 1H), 1.88 (m, 2H), 1.78 (s, 3H), 1.66 (s, 3H), 1.57 - 1.48 (m, 2H), 1.44 - 1.39 (m, 2H), 1.07 (s, 6H) ppm. 13C-NMR (101 MHz, C6D6): δ 142.3 (s), 137.5 (s), 135.1 (d), 132.1 (d), 130.8 (s), 78.6 (d), 39.7 (t) 34.0 (s), 33.1 (t), 29.1 (q, 2x), 22.0 (q), 20.9 (q), 19.3 (t) ppm. UV (MeOH): λmax 266 nm. IR (NaCl): v 2925 (m, C-H), 2862 (m, C-H), 962 (m), 771 (s) cm-1. HRMS (ESI⁺): Cald. for C14H21I ([M+H]⁺), 316.0688; found, 316.0684.

Silylether (XV). To a solution of 2-(1E,3Z)-4-iodo-3-methylbuta-1,3-dien-1-yl)-1,3,3-trimethylcyclohex-1-ene XIII (161 mg, 0.510 mmol) in THF (0.2 mL) was added Pd(PPh3)4 (45 mg, 0.039 mmol). After stirring for 5 min at 25 °C, a solution of (R)-borolane XIV (150 mg, 0.392 mmol) in THF (1.0 mL) and 10% aqueous solution of TlOH (4.26 mL) were added and the reaction mixture was stirred for 2 h. The mixture was extracted with Et2O (3x) and the combined organic layers were washed with brine (3x) and dried (Na2SO4) and the solvent was evaporated. The residue was purified by column chromatography (silica gel, 95:5 hexane/EtOAc) to afford 120 mg (71%) of a colorless oil identified as (3R,4E,6Z,8E)-{[3,7-dimethyl-9-(2,6,6-trimethylcyclohex-1-en-1-yl)nona-4,6,8-trien-1-yl]oxy}triisopropylsilane (XV). 1H NMR (400.16 MHz, C6D6): δ 6.93 (d, J = 16.0 Hz, 1H), 6.69 (dd, J = 14.9, 11.1 Hz, 1H), 6.29 (d, J = 16.0 Hz, 1H), 6.01 (d, J = 11.1 Hz, 1H), 5.46 (dd, J = 15.0, 8.3 Hz, 1H), 3.35 (t, J = 6.6 Hz, 2H), 2.34 - 2.19 (m, 1H), 1.99 - 1.89 (m, 2H), 1.93 (s, 3H), 1.79 (s, 3H), 1.62 - 1.53 (m, 2H), 1.50 - 1.44 (m, 2H), 1.36 (q, J = 6.7 Hz, 2H), 1.11 (s, 6H), 0.91 (d, J = 6.7 Hz, 3H) ppm. HRMS (ESI⁺): Cald. for C29H53OSi ([M+H]⁺), 445.3848; found, 445.3806.

(R)-9-cis-13,14-Dihydro-retinol XVI. To a cold (0 °C) solution of silylether (XV) (91.2 mg, 0.212 mmol) in THF (3.5 mL) was added Bu4NF (0.32 mL, 0.32 mmol) and the mixture was stirred for 0.5 h at 25 °C. A saturated aqueous solution of NaHCO3 was added and the mixture was extracted with Et2O (3x). The combined organic layers were washed with brine (3x) and dried (Na2SO4) and the solvent was evaporated. The residue was purified by column chromatography (gradient from 95:5 to 70:30 hexane/EtOAc) to afford 41 mg (67%) of a colorless oil identified as (R)-9-cis-13,14-dihydro-retinol (XVI). 1H NMR (400 MHz, C6D6): δ 6.93 (d, J = 16.0 Hz, 1H), 6.69 (dd, J = 14.9, 11.1 Hz, 1H), 6.29 (d, J = 16.0 Hz, 1H), 6.01 (d, J = 11.1 Hz, 1H), 5.46 (dd, J = 15.0, 8.3 Hz, 1H), 3.35 (t, J = 6.6 Hz, 2H), 2.34 - 2.19 (m, 1H), 1.99 - 1.89 (m, 2H), 1.93 (s, 3H), 1.79 (s, 3H), 1.62 - 1.53 (m, 2H), 1.50 - 1.44 (m, 2H), 1.36 (q, J = 6.7 Hz, 2H), 1.11 (s, 6H), 0.91 (d, J = 6.7 Hz, 3H) ppm. 13C NMR (101 MHz, C6D6): δ 140.0 (d), 138.6 (s), 132.5 (s), 130.9 (d), 129.6 (d), 129.2 (s), 128.0 (d), 124.9 (d), 60.9 (t), 40.2 (t), 39.9 (t), 34.5 (s), 34.3 (d), 33.2 (t), 29.21 (q), 29.19 (q), 22.0 (q), 20.9 (q), 20.7 (q), 19.7 (t) ppm. IR (NaCl): ν 3326 (br, O-H), 2966 (m, C-H), 2865 (m, C-H), 965 (s) cm-1. HRMS (ESI⁺): Cald. for C20H33O ([M+H]⁺), 289.2584; found, 289.2525.

(R)-9-cis-13,14-Dihydro-β,β-carotene (XIX). To a solution of alcohol (XVI) (25 mg, 0.087 mmol) in CH2Cl2 (3.7 mL) at 0 °C were added Dess-Martin periodinane (73.5 mg, 0.173 mmol) and pyridine (0.014 mL, 0.173 mmol) and the reaction was stirred for 30 min at 0 °C and 2 h at 25 °C. Then, Et2O (5 mL), a saturated aqueous solution of NaHCO3 (3 mL) and a saturated aqueous solution of Na2S2O3 (3 mL) were added. The layers were separated and the aqueous layer was extracted with Et2O (3x) and the combined organic layers were washed with a saturated aqueous solution of NaHCO3 (2x) and brine (2x), dried (Na2SO4) and the solvent was evaporated. The residue was purified by column chromatography (silica gel, gradient from 95:5 to 70:30 hexane/EtOAc) to afford 14 mg (57%) of a colorless oil identified as (R)-9-cis-13,14-dihydro-retinal (XVII). It was observed that this product was very unstable and easily isomerized. IR (NaCl): v 2927 (m, C-H), 2864 (m, C-H), 1461 (s), 1105. HRMS (ESI⁺): Cald. for C20H31O ([M+H]⁺), 287.2362; found, 287.2369. To a solution of phosphonium salt VII (24.9 mg, 0.044 mmol) in THF (0.2 mL) at -78 °C was added nBuLi (0.027 mL, 0.044 mmol, 1.6 M in hexanes) and the reaction was stirred for 30 min. Then a solution of (R)-9-cis-13,14-dihydro-retinal XVII (9.0 mg, 0.031 mmol) in THF (0.13 mL) was added and the mixture was stirred for 1 h at -78 °C and 30 min at 25 °C. Water was added and the mixture was extracted with Et2O (3x). The combined organic layers were washed with a saturated aqueous solution of NaCl (2x) and dried (Na2SO4). The solvent was evaporated and the residue was purified by column chromatography (C18-silica gel, CH3CN) to afford 11.3 mg (67%) of a red foam identified as (R)-9-cis-13,14-dihydro-β,β-carotene (XIX). 1H-NMR (400 MHz, C6D6) δ 6.80-6.63 (m, 2H), 6.57-5.90 (m, 7H), 5.80-5.35 (m, 4H), 2.32-2.10 (m, 3H), 1.94 (s, 3H), 1.91 (s, 3H), 1.89-1.82 (m, 4H), 1.78 (s, 9H), 1.67-1.43 (m, 8H), 1.33 (s, 6H), 1.10 (s, 12H), 0.96 (m, 3H) ppm. HRMS (ESI⁺): Cald. for C40H59 ([M+H]⁺), 539.2528; found, 539.4611.

References used in the synthesis procedures which are incorporated herein:
Okitsu, T. Chem. Comm. 2008, 47, 6330-6332.
Englert, G. Helv. Chim. Acta 1975, 58(8), 2367-2390.
Zanoun, A. J. Mol. Struct.: THEOCHEM 2006, 777(1-3), 113-120.
Koyama, Y.; Hosomi, M.; Hashimoto, H. Shimamura, T. J. Mol. Struct. 1989, 193, 185-201.
Koukal, P.; Ulc, J.; Necas, D.; Kotora, M. Eur. J. Org. Chem. 2016, 2110-2114.
(a) Isler, 0.; Gutmann, H.; Lindlar, H.; Montaron, M.; Riiegg, R.; Ryser, G.; Zeller, P. Helv. Chim. Acta 1956, 39, 463. (b) Broek, A. D.; Muradin-Szweykowska, M.; Courtin, J. M. L.; Lugtenburg, J. Red. Trav. Chim. Pays-Bas 1983, 102, 46.

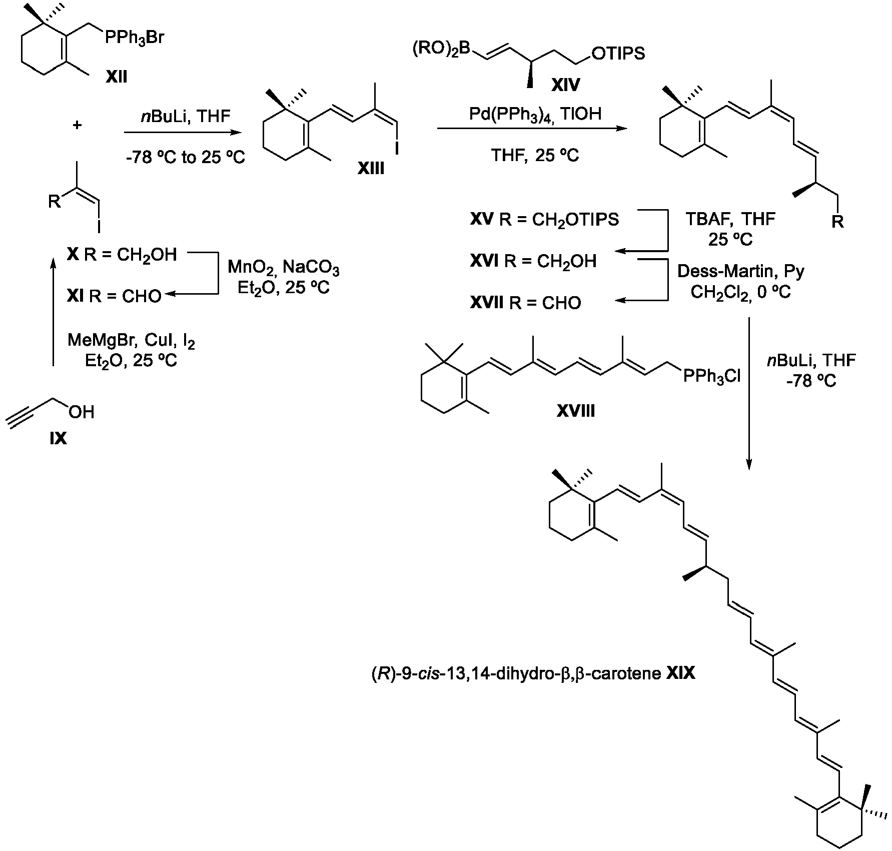

### Example 3: Animal experiments

### Animals:

Male mice (Charles River, France) were housed in groups of 3 mice per cage in a 7am-7pm light/dark cycle in individually ventilated cages (Techniplast, Italy). Food (standard chow diet, D04 from SAFE, France) and water were freely available. All experiments were carried out in accordance with the European Community Council Directives of 24 November 1986 (86/609/EEC) and in compliance with the guidelines of CNRS and the French Agricultural and Forestry Ministry (decree 87848).

For social defeat stress protocol we used C57BL/6N mice (Charles River, France) at the age of 6-7 weeks. All mice were housed in groups of 4-5 mice per cage in a 7am-7pm light/dark cycle in individually ventilated cages (Techniplast, Italy) until stress protocol which was carried out in MICE cages adapted for social defeat stress (see below). CD1 males mice purchased from Charles River (France) were used as aggressors in this test (see below).

Food and water were freely available. All experiments were carried out in accordance with the European Community Council Directives of 24 November 1986 (86/609/EEC) and in compliance with the guidelines of CNRS and the French Agricultural and Forestry Ministry (decree 87848) and authorisation of French Ministry of Research.

### Behavioral procedures and tissue sample collection:

Behaviourally naive groups of mice were tested in the DNMTP in the T-maze according to a protocol previously described (Wietrzych et al., 2005), with modifications to facilitate pharmacological tests (Wietrzych-Schindler et al., 2011). Specifically, in the present protocol, animals were first trained for 10 consecutive days with minimal inter-trial intervals (ITI) of about 15 sec, which was necessary to attain the criterion of 90% or more of correct choices during four consecutive days (days 9-10). After this period ITI was increased semi-randomly by blocks of 6 min during 5 consecutive days (days 11-15 indicated) to determine "test ITI" for each animal, which was defined as the shortest ITI at which mice perform at chance level. From day 16 mice were tested using corresponding "test ITI" delays following vehicle injection on the day 16 (to test for the effect of vehicle alone) and following 9CDHROL (40 mg/kg) injection on day 17. Three mice were excluded from testing since their latency to choose the arm during the retention phase exceeded 3 min in more than one trial/day on three consecutive days (exclusion criterion). On the day 18 mice were again injected with 9CDHROL (40 mg/kg) and 9 hours later sacrificed for chemical analyses. Mice which received vehicle injections on three consecutive days were used as vehicle-treated controls for analyses of 9CDHROL metabolism. An additional group of 3 naive mice was also injected with 9CDHROL (40 mg/kg) to test for 9CDHROL metabolism following acute treatment.

**Forced swim test:** The forced swim paradigm was carried out between 1pm and 4pm in a 3-litre glass beaker half-filled with water at 22-23°C (the water depth was 17 cm). All mice were tested only once in this task. To this end, each mouse was lowered gently into the water and the time of immobility was scored during a 6-minute testing period. The mouse was judged immobile when it floated in an upright position and made only small movements to keep its head above the water. After 6 min, the mouse was taken out of the water, left to dry under a red light lamp and returned to its home cage. The immobility scores of each animal were used as an index of despair behaviour.

**Sucrose preference test:** This task, designed to measure hedonic behaviours in mice is based on the palatable nature of sucrose observed in a number of mouse strains. On the first day of the test, sucrose-naive mice were placed in individual cages at 11am and left there with water and food for habituation period. At 5pm one water bottle was replaced with two bottles: one containing water and another 0.8% sucrose solution. Three hours later (8pm) the bottles were weighed to measure liquid consumption and were replaced in cages until morning. The measures of an overnight consumption were then carried out for additional day to evaluate sucrose preference. Mice were not water deprived at any moment, in order to measure spontaneous sucrose preference and exclude any potential emotional confounds induced by stress of water deprivation. The sucrose preference was expressed as the percent of sucrose solution consumed with respect to total liquid consumption.

**Social Defeat Stress:** Social defeat stress procedure was a modified version of the protocol previously described (Berton, McClung et al. 2006). C57BL/6N mice were defeated chronically for 10 consecutive days. Every day they were exposed to the physical contact with an unfamiliar CD1 aggressor in its home cage for maximal interaction time of 5 minutes. After each session of physical stress, C57BL/6N and CD1 mice were separated by a perforated wall and maintained in sensory contact for 24h. After the last session of stress, mice were transferred into new cages and housed separately throughout the behavioral tests period. C57BL/6N control mice, similarly to the experimental animals, were housed two per cage separated by a metal perforated wall. Every day, they were exposed to the physical contact for 5 minutes.

### Animal treatments

For behavioral analysis 9CDHROL and 9CDHBC were dissolved in ethanol, and then mixed with sunflower oil, so that the final solution contained 3% ethanol. Vehicle treatments consisted of 3% ethanol solution in sunflower oil. Treatments were administered by intraperitoneal injections at volume/weight ratio 3 ml/kg between at 7am and 7 h before the beginning of testing. Treatments were administered per os at 10mg/kg for each substance and volume/weight ratio 3 ml/kg between 5-6pm every second day of stress protocol immediately after the stress session starting from day 1of the stress protocol.

For metabolic analyses ATROL, 9CDHROL, 9CDHBC and control solutions were prepared as described above. Single per os application of the dose of 40mg/kg of each substance was used to treat mice in the evening and samples were collected 11h later in the light protected conditions, weighted, frozen in liquid nitrogen and stored at -80° until analyses. Serum was prepared and immediately stored in brown vials at -80C, till further analysis.

### Statistical analysis

The comparisons of behavioral performance during learning phase was carried out using one-way ANOVA on repeated measures with time as dependent parameter and percent of correct choices as an independent parameter. The pharmacological data were analyzed by comparing animal performance after vehicle and 9CDHROL treatment using one-way ANOVA on repeated measures. Post-hoc statistical analyses were performed to compare animal performance to 50% chance level using one-group student t-test. Significant differences are indicated in the corresponding figure.

### Results from animal supplementation studies

To test the possibility that 9CDHROL can act as substrate for production of 9CDHRA in vivo, we have tested working memory in delayed non-match to place (DNMTP) as a behavioral paradigm sensitive to detect activity of 9CDHRA (Riihl et al., 2015). To this end we have trained a cohort of C57BL6J male mice in DNMTP task to attain the criterion of maximal performance (above 90%) over 4 consecutive days (Fig. 1a). Wild type C57BL6N male mice acquired working memory task as indicated by significant effect of the day of training (F[9,39]=8,28; p<0.001, one-way ANOVA on repeated measures) when trained with 15 sec inter-trial intervals (ITI). Memory performance was decreased when mice were tested at longer ITI of 3min. However, when ITI reached on average 13min (the mean for the whole group indicated in the graph as gr13) mice displayed complete memory loss in DNMTP task as their performance was not different from the chance level of 50% (p<0.05, one group t-test). The ITI at which each animal displayed complete forgetting were identified as "test ITI" for each individual animal and was used when testing pro-mnemonic activity of 9CDHROL (Fig. 1b). During such tests vehicle application did not improve memory as percent of correct choices was comparable to chance level of 50% (p=0.2, ns; one group t-test), whereas 9CDHROL treatment significantly enhanced memory performance (F[1,4] 16; p<0.05, one-way ANOVA on repeated measures for the effect of treatment). 9CDHROL treated mice performed significantly better than the chance level of 50% (p<0.05; one group t-test).

### Example 4: Analytical procedures and HPLC and LC-MS analysis

### LC-MS analysis of tissue samples

**A. Just retinoid analysis:** Analytical procedures: High performance liquid chromatography mass spectrometry (Agilent 1260 Infinity LC system; Madrid, Spain) -mass spectrometry (SCIEX Triple Quad 3500 System; Sciex, Madrid, Spain) analyses were performed under dark yellow/amber light using previously validated protocol (Riihl, 2006; Rühl et al., 2015). For the detection of 13,14-dihydroretinoic acid MS-MS setting were 303 -> 207 m/z and for the detection of 13,14-dihydroretinol MS-MS settings were 290 -> 69 m/z using the same dwell time and collision energy comparable to MS-MS specific settings of retinoic acids. Accordingly, for sample preparation 100 mg of the material (if samples were under 100 mg, water was added up to the used standard weight: 100 mg) or 100 µl serum was diluted with a threefold volume of isopropanol, the tissues were minced by scissors, vortexed for 10 seconds, put in a ultra sonic bath for 5 minutes, shaken for 6 minutes and centrifuged at 13000 rpm in a Heraeus BIOFUGE Fresco at +4°C. After centrifugation, the supernatants were dried in an Eppendorf concentrator 5301 (Eppendorf, Germany) at 30°C. The dried extracts were resuspended with 60 µl of methanol, diluted with 40 µl of 60 mM aqueous ammonium acetate solution and transferred into the autosampler and subsequently analysed.

**B. Combined retinoid and carotenoid analysis:** Analytical procedures: High performance liquid chromatography mass spectrometry (Agilent 1260 Infinity LC system; Madrid, Spain)-mass spectrometry (SCIEX Triple Quad 3500 System; Sciex, Madrid, Spain) plus and additional online diode array detector (Waters 966 DAD, Waters, Santiago de Compostella, ES) analyses were performed under dark yellow/amber light using previously validated protocol (LIT) plus adding a third and 4th eluent after 20 min elution time. Linear gradient from 20 min 20% (isopropanol: methanol :methyl-tert-butyl-ether (MTBE) / 30:30:40) - 80% (isopropanol:methanol / 50:50), 25 min 40% (isopropanol:methanol :methyl-tert-butyl-ether (MTBE) / 30:30:40) - 60% (isopropanol:methanol / 50:50), 29 min 70% (isopropanol:methanol:methyl-tert-butyl-ether (MTBE) / 30:30:40) - 30% (isopropanol:methanol / 50:50), 30 min 0% (isopropanol: methanol :methyl-tert-butyl-ether (MTBE) / 30:30:40) - 100% (isopropanol:methanol / 50:50), 30,1 min 20% (isopropanol : methanol :methyl-tert-butyl-ether (MTBE) / 30:30:40) - 80% (isopropanol:methanol / 50:50). For the detection of 13,14-dihydroretinoic acid MS-MS setting were 303 -> 207 m/z, for the detection of 13,14-dihydroretinol MS-MS settings were 290 -> 69 m/z and for the detection of 9CDHBC 405 - > 405 / 405 -> 95 m/z. Accordingly, for sample preparation 100 mg of the material (if samples were under 100 mg, water was added up to the used standard weight: 100 mg) or 100 µl serum was diluted with a threefold volume of isopropanol, the tissues were minced by scissors, vortexed for 10 seconds, put in a ultrasonic bath for 5 minutes, shaken for 6 minutes and centrifuged at 13000 rpm in a Heraeus BIOFUGE Fresco at +4°C. After centrifugation, the supernatants were dried dried in a GYROZEN centrifugal vaccum concentrator equipped with a ILMAC MPC 301-Z vacuum pump (CONTROLTECNICA, Madrid, ES) at 30°C. The dried extracts were resuspended with 30 µl of methanol - MTBE (50 - 50) and transferred into the autosampler and 10 ul subsequently analysed.

### Statistical analysis

Statistical analyses for behavioral analysis and retinoid / carotenoid analysis were performed using student t-test for each of the two-group comparisons. Significant differences are indicated in the corresponding figures.

### Results from HPLC and LC-MS analysis

### Initial identification of endogenous 9CDHROL and identification of 9CDHROL after treatment of 9CDHROL to mice

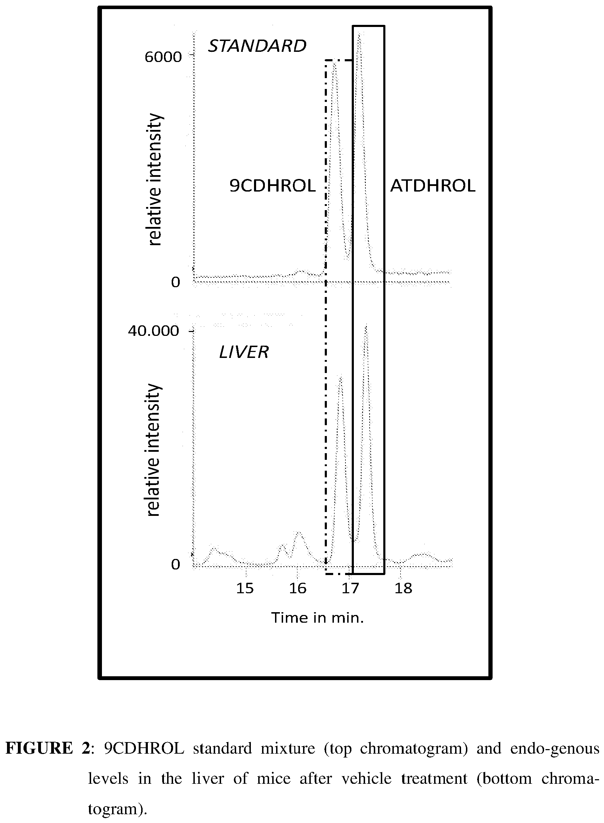

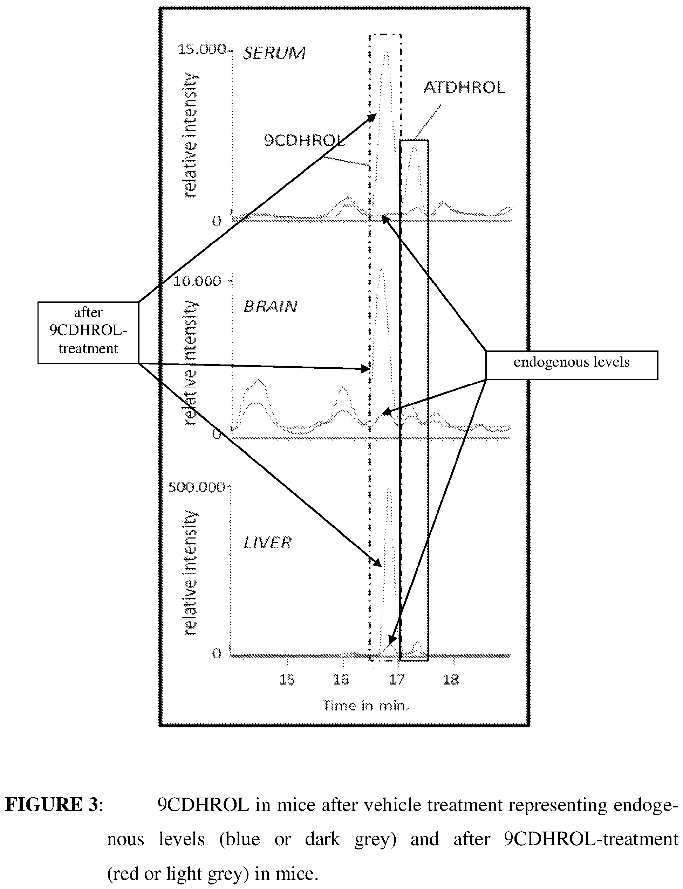

Initially we analyzed a mixed standard of 9-cis-13,14-dihydroretinol (9CDHROL) and all-trans-dihydroretinol (ATDHROL) using our LC-MS analytics (top Figure of Figure 2) and after we analysed a liver sample from vehicle treated mice (bottom figure of Figure 2). Both peaks from the standards 9CDHROL and ATDHROL are co-eluting with the peaks identified in the liver samples using the same MS-MS fragmentation channels in our LC-MS system. Based on the co-elution and using the same LC-MS parameters and the same fragmentation pattern channels (290 -> 69 m/z) used, we claim and identify 9CDHROL and ATDHROL as endogenous retinoids in the mammalian organism. In addition, these co-eluting peaks can also be observed in lower levels in serum and brain of mice and 9CDHROL is strongly increased after 9CDHROL-treatment (Figure 3).

### Identification of endogenous 9CDHROL in mouse, human and the human food matrix:

**Mouse brain** were prepared according to procedure described above. Briefly, after single per os application of the dose of 40mg/kg of 9CDHROL (or other cited substances) in the evening mice were sacrificed after 11 hours and tissues samples including brain were dissected in the light protected conditions, weighted, frozen in liquid nitrogen and stored at -80° until analyses. Serum was prepared and immediately stored in brown vials at -80C, till further analysis.

**Human serum samples** Were obtained from the blood of healthy volunteers with all the subjects' written informed consent.

**Food samples:** Beef liver was bought at a local butcher in Vigo, Spain. Conserves of peaches in can (Metades, Pessago em calda, AuchanlAlcampo-home-brand / 420g can) were purchased at Alcampo, Vigo, Spain.

Initially we analyzed a standard of 9-cis-13,14-dihydroretinol (9CDHROL, top chromatogram of Figure 14A) using our LC-MS analytics and after we analysed a control brain sample from control treated mice (middle chromatogram of Figure 14A). The peak from the 9CDHROL standard is co-eluting with the peak identified in the brain samples using the same MS-MS fragmentation channels in our LC-MS system.

In addition we identified 9CDHROL in human serum (Figure 14B) as well as in relevant human food / beef liver (Figure 14C). Based on the co-elution and using the same LC-MS parameters and the same fragmentation pattern channels (290 -> 69 m/z) used, we claim and identify 9CDHROL as a novel endogenous retinoid in the mammalian organism mouse and humans as well as being present in human food represented by beef liver, which is also a mammalian organism.

### Identification of 9CDHBC in the human food matrix (Figure 15):

9CDHBC was identified using co-elution and comparable UV/VIS spectra in peaches in a can (conserve).

### Example 5: Identification of compounds after treatment in mice

### Identification of 9CDHRA after 9CDHROL treatment in mice

Recently we identified that 9CDHRA is an endogenous derivative in mice (Rühl et al., 2015) and in humans (still unpublished data). The next step is to identify precursors of 9CDHRA in *in vivo* and *in vitro* models.

Animal treatment, administration of 9CDHROL and sample analysis was carried out as described in Examples 3 and 4.

Using mouse supplementation experiments we gavaged n=6 mice orally with 100 mg/kg bw 9CDHROL i.p. and 9 h after the treatment the mice were killed and blood, brain and liver was taken. After centrifugation of the drawn blood, serum was obtained.

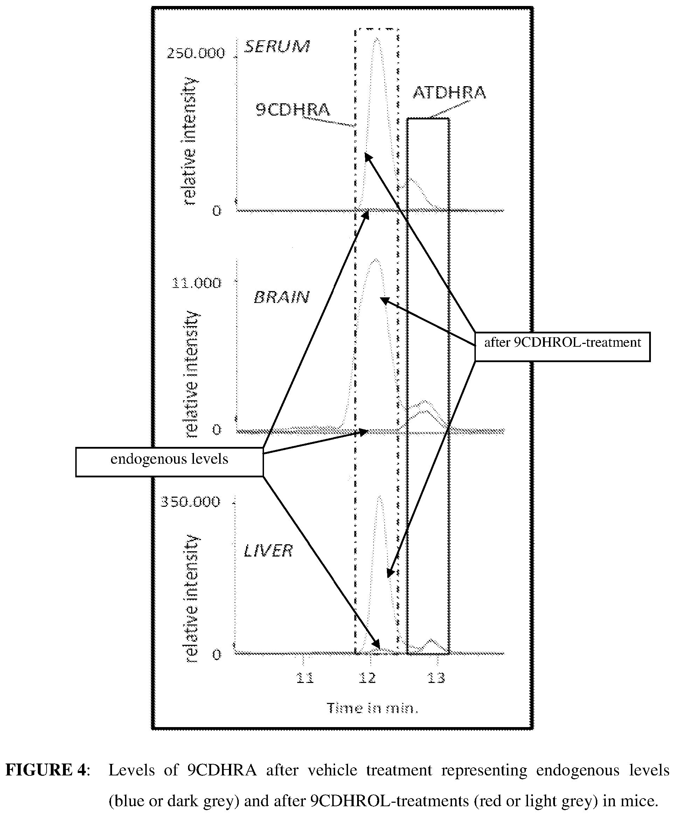

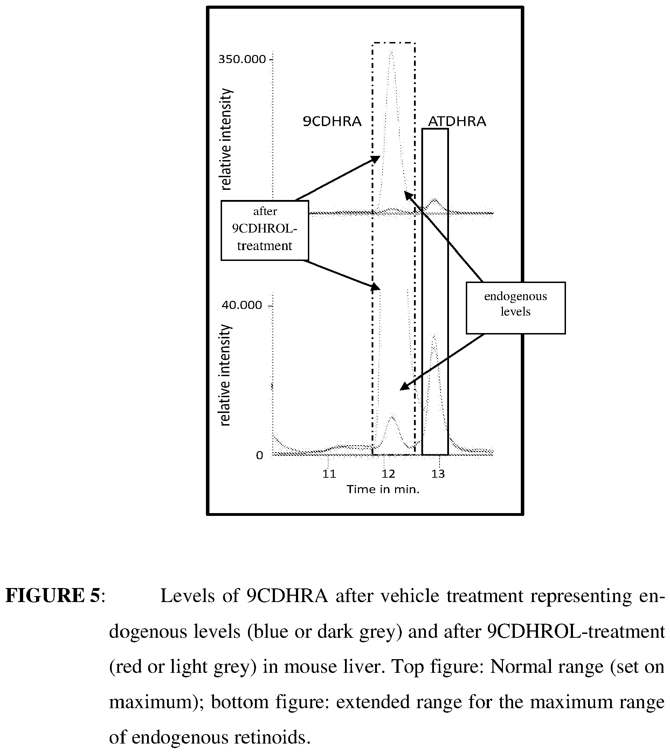

In serum, brain and liver 9CDHRA and ATDHRA was found endogenously in low levels. In addition after 9CDHROL-treatment a strong increase of 9CDHRA levels were identified in serum, brain and liver analysed samples (Figure 4). As a comparison we displayed the increase levels of 9CDHRA in liver samples in Figure 5.

### Identification of 9CDHROL in mice

**9CDHROL is accumulated in mice 9CDHROL-treated mice and a metabolite from 9CDHBC *in vitro* oligodendrocyte cell culture:** Treatment with 9CDHROL in orally supplemented mice increases 9CDHROL in mouse brain (Figure 14A, bottom chromatogram) compared to control-treated brain (middle chromatogram of Figure 14A). We note herein that administration of 9CDHBC and 9CDHROL increases 9CDHROL levels in human oligodendrocytes cell culture, while ATBC did not display any increase. 9CBC (and ATROL) display no or just a weak and non-isomer selective increase of 9CDHROL in oligodendrocytes cell culture (Figure 14D). **In summary, 9CDHBC is an excellent precursor substrate of 9CDHROL in human oligodendrocyte cell culture *in vitro.***

### Example 6: Cell culture experiments

### Cell culture experiments

Normal immortalized oligodendrocyte line 158N was cultured as previously reported (Feutz et al., 2001). When cells attained 70% of confluency they were treated with 10-2M ethanol solution of one of the following compounds: 9CDHROL, 9CDHROL-acetate, 9CDHRA-ethyl ester. 9CDHRA and ethanol as control, vehicle treatment. 18 h after treatment cells were collected in amber Eppendorf tubes and stored at -80°C until analyses. Treatments and cell collection were carried out in light limited conditions to avoid photolysis of retinoids.

High performance liquid chromatography mass spectrometry (LC-MS) was carried out as described in Example 4.

### Conversion of ATROL and 9CROL

In analogous experiments we applied the following retinol species, mutatis mutandis on the same cell culture: ATROL and 9CROL. As a standard normal ROL standard was applied (black line). The species are also indicated by arrows (Figure 11).

Conversion of the retinol species were also followed. On Figure 12 measurement of 13,14 dihydro-retinoic acid (DH-RA) species 9CDHRA and ATDHRA is shown after administration of the same retinol species ATROL and 9CROL (blue line and red line, respectively). It is clearly shown that at most a very small amount of dihydro-acids or no amount at all (9CROL) occurs in the cells.

In analogous experiments, measurement of retinoic acid species 13-cis retinoic acid (13CRA) as well as 9CRA and ATRA is shown after administration of the same retinol species ATROL and 9CROL (blue line and red line, respectively). It is clearly shown that at most a very small amount of these acid forms if any occur in the cells which are barely can be seen against a noisy background. Thus, conversion of the alcohols into the corresponding acid forms is very weak and the alcoholic forms are certainly inappropriate as precursors.

Analytical procedures were carried out as described above.

### Identification of 9CDHRA after addition of 9CDHROL, 9CDHRET-acetate or 9CDHRA-ethyl-ester to oligodendrocytes

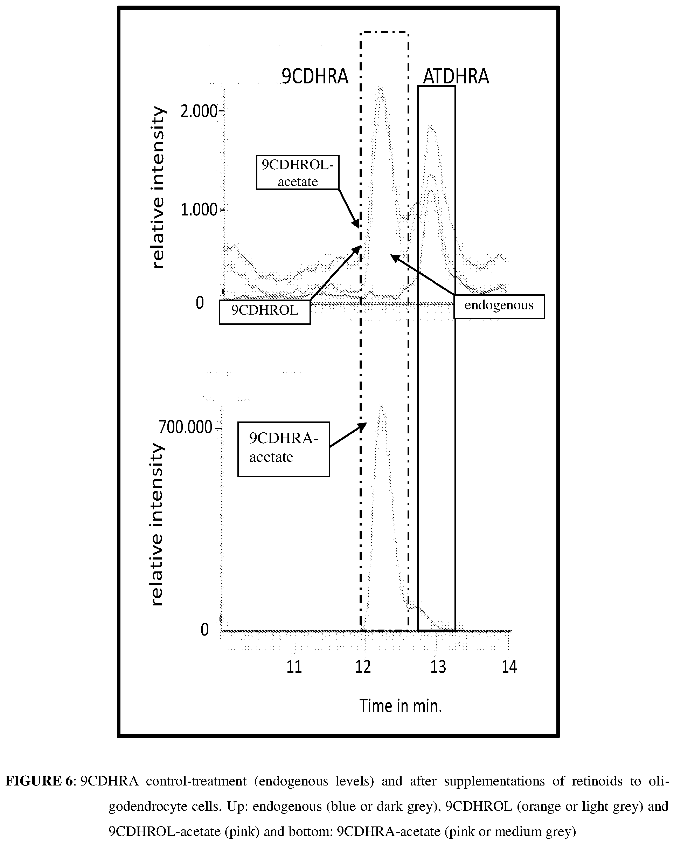

We identified that 9CDHRA is a metabolite of 9CDHROL supplementation in serum, brain and liver of mice. In addition we treated oligodendrocyte cells lines with 10-5 M of 9CDHROL, 9CDHRET-acetate or 9CDHRA-ethyl-ester for 18 h. We observed that after administration of 9CDHROL, 9CDHRET-acetate a strong increase of 9CDHRA-levels was found (top Figure 6). In addition we found that after 9CDHRA-ethyl-ester application a very strong increase was identified (bottom Figure 6).

### Identification of 9CDHROL after administration of 9CDHBC and 9CDHROL

Administration of 9CDHBC and 9CDHROL increases 9CDHROL levels in human oligodendrocytes cell culture, while ATBC did not display any increase. 9CBC (and ATROL) display no or just a weak and non-isomer selective increase of 9CDHROL in oligodendrocytes cell culture (Figure 14D). In summary, 9CDHBC is an excellent precursor substrate of 9CDHROL in human oligodendrocyte cell culture *in vitro.*

### Identification of 9CDHBC after 9CDHBC, 9CBC and ATBC administration

**Identification of 9CDHBC after 9CDHBC and 9CBC administration, but not after ATBC administration to oligodendrocyte cell culture in vitro (Figure 16A and 16B):** In oligodendrocytes cell culture when administering 9CBC we can detect 9CBC with a retention time of 26,1 min at 411 nm detection wavelength in these treated cells (Figure 16A, 411 nm detection), while no 9CBC could be detected in control treatments as well as either after ATBC or 9CDHBC-treatments. The existence has also been confirmed by a UV/Vis spectra taken by the diode array detector (data not shown here).

Focusing on 9CDHBC with a UV detection at 366nm (Figure 16B), we can detect a very small peak of 9CDHBC after control treatments or ATBC treatments, while after 9CBC-treatments a somewhat higher peak and an even more higher peak can be detected after 9CDHBC-treatment (at 25,1 min retention time) and was confirmed by a comparable UV spectra taken by the diode array detector (data not shown here).

### Identification of 9CDHRA after 9CDHROL, 9CDHROL-ester, 9CDHRA and 9CDHRA-ester administration to oligodendrocyte cell culture

In treated oligodendrocyte cells lines with 10⁻⁵ M of 9CDHROL, 9CDHROL-acetate, 9CDHRA or 9CDHRA-ethyl-ester for 18 h. We observed that after 9CDHROL, 9CDHROL-acetate a strong increase of 9CDHRA-levels was found (Figure 17A). In addition, we found that after 9CDHRA-ethyl-ester (and 9CDHRA, data not shown) applications a very strong increase was identified (bottom chromatogram of Figure 17A). While 9CDHROL and 9CDHROL-esters are excellent precursor of 9CDHRA, we also determined that ATROL, 9CDHBC, 9CBC and ATBC are not converted to 9CDHRA (data not shown). In summary, 9CDHROL, 9CDHROL-esters and 9CDHRA-esters are excellent, selective and isomer-specific RXR-ligand precursors in human oligodendrocyte cell culture *in vitro.*

### Example 7 Identification of 9CDHRA after 9CDHROL, 9CDHBC supplementation in mice

The experiments were analogous to those described in Example 5.

In addition to the *in vitro* experiments we also performed *in vivo* supplementation experiments with orally supplemented mice with 9CDHBC and 9CDHROL (**Figure 17B** **and** **17C**). A moderate but significant increase of 9CDHRA was observed after 9CDHBC-supplementation (Figure 17B) in comparison with control. Low levels of 9CDHRA and its isomer ATDHRA can also be observed in serum, liver and brain of control-mice (top chromatograms of Figure 17C) of mice and especially 9CDHRA is strongly increased after 9CDHROL-supplementation (bottom chromatograms of Figure 17C).

### Example 8: Experimental procedure - testing (R)-9CDHRA in animal tests

### Example 8.1: Methods

### Animals:

Rbp1-/- and Rxrγ-/- mutants as well as their wild type (WT) control mice were raised on a mixed genetic background (60% C57BL/6J and 40% 129SvEms/j) from heterozygous crosses as described (Ghyselinck NB 1999; Krezel et al., 1996), and tested at the age of 3-6 months. All mice were housed in groups of 4-5 mice per cage in a 7am-7pm light/dark cycle in individually ventilated cages (Techniplast, Italy). Food and water were freely available. All experiments were carried out in accordance with the European Community Council Directives of 24 November 1986 (86/609/EEC) and in compliance with the guidelines of CNRS and the French Agricultural and Forestry Ministry (decree 87848).

For social defeat stress protocol we used C57BL/6N mice which were transferred from Taconic (France) at the age of 6 weeks and housed in groups of 4 per cage. After 1 week of habituation period they were subjected to the social defeat stress. CD 1 purchased from Charles River (France), were used as aggressors in this test.

*Behavioural procedures:* All behavioural tests were carried out in the Institute Clinique de la Souris (http://www.ics-mci.frl) according to standard operating procedures.

### Forced swim test:

The forced swim paradigm (Dalvi and Lucki, 1999) was carried out between 1pm and 4pm in a 2-litre glass beaker half-filled with water at 22-23°C (the water depth was 17 cm). All mice were tested only once in this task. To this end, each mouse was lowered gently into the water and the time of immobility was scored during a 6-minute testing period. The mouse was judged immobile when it floated in an upright position and made only small movements to keep its head above the water. After 6 min, the mouse was taken out of the water, left to dry under a red light lamp and returned to its home cage. The immobility scores of each animal were used as an index of despair behaviour.

*Sucrose preference test:* This task, designed to measure hedonic behaviours in mice (Moreau, 1997), is based on the palatable nature of sucrose observed in a number of mouse strains. On the first day of the test, sucrose-naive mice were placed in individual cages at 11am and left there with water and food for habituation period. At 5pm one water bottle was replaced with two bottles: one containing water and another 0.8% sucrose solution. Three hours later (8pm) the bottles were weighed to measure liquid consumption and were replaced in cages until morning. The measures of an overnight consumption were then carried out for additional day to evaluate sucrose preference. Mice were not water deprived at any moment, in order to measure spontaneous sucrose preference and exclude any potential emotional confounds induced by stress of water deprivation. The sucrose preference was expressed as the percent of sucrose solution consumed with respect to total liquid consumption.

*Social defeat stress:* Social defeat stress procedure was a modified version of the protocol previously described (Berton et al. 2006). C57BL/6N mice were defeated chronically for 10 consecutive days. Every day they were exposed to the physical contact with an unfamiliar CD1 aggressor in its home cage for maximal interaction time of 5 minutes. After each session of physical stress, C57BL/6N and CD1 mice were separated by a perforated wall and maintained in sensory contact for 24h. After the last session of stress, mice were transferred into new cages and housed separately throughout the behavioral tests period. C57BL/6N control mice, similarly to the experimental animals, were housed two per cage separated by a metal perforated wall. Every day, they were exposed to the physical contact for 5 minutes.

Animals were then tested in the forced swim and sucrose tests.

### Example 7.2: R-9CDHRA supplementation reverses behavioural changes in Rbp1-/- mice

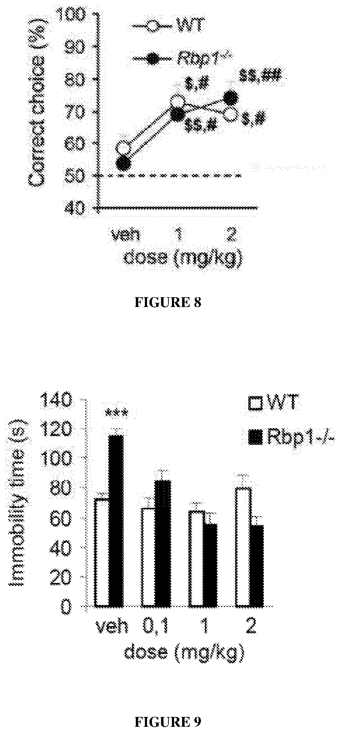

In order to address relevance of 9CDHRA in modulation of RXR functions *in vivo* inventors tested whether R-9CDHRA can reverse behavioural deficits in Rbp1-/- mice. Acute treatment with R-9CDHRA reduced in dose dependent manner immobility of knockout mice in the forced swim test attaining maximal effect already at 1 mg/kg, which was comparable to antidespair effect of synthetic RXR agonist UVI2108 at the same dose or 5 mg/kg treatment with ATRA (Figure 9). Effects of treatments were not evident in WT mice, which may be related to the low baseline immobility in this strain and in consequence floor effect. Such activities were mediated by RXRγ as 2 mg treatment with R-9CDHRA did not improve performance of Rxrγ-/- mice, which remained immobile for 120 ± 25 sec as compared to 119 ± 19 sec in vehicle treated Rxrγ-/- animals.

Similarly to antidespair effects in the forced swim test, 1 or 2 mg/kg of R-9CDHRA also improved performance of Rbp1-/- mice in memory tests. Such treatments increased the rate of successful choices of Rbp1-/-, which performed significantly better than 50% of chance level when tested at inter-trial interval of 6 min in DNMTP test (Figure 8). Treatment with 2 mg/kg of R-9CDHRA raised also performance of WT mice to about 70% of correct choices when tested at long inter-trial intervals of 12 or 18 min at which the same WT mice performed at chance level (50% of correct choices) if treated with vehicle. Such treatment did not improve performance of Rxrγ-/- mice which performed at 57 ± 7% of correct choices, providing further evidence that compromised RXRγ function associated with reduced levels of 9CDHRA is at the origin of the deficits observed in Rbp1-/- animals.

### Example 7.3: R-9CDHRA displays antidepressant effects in chronic social defeat stress model.

The present inventors have found that R-9CDHRA supplementation treats depressive behaviours in chronic stress model of depression. Stress is an important environmental factor in the etiology of depression. To test efficiency of 9cDHRA for treatment of depressive behaviours induced by stress, we used social defeat stress animal model (Berton et al., 2006, Hollis and Kabbaj, 2014). Ten days of short daily physical contacts with resident dominant CD1 male followed by subsequent sensory contact efficiently induced despair in the forced swim task (Figure 10.a). Treatment with 1 or 3 mg/kg of 9cDHRA during the stress protocol efficiently normalised immobility time which was comparable with control non-stressed mice. In contrast to the lower dose, treatment with 3mg/kg of 9cDHRA displayed anti-despair effects as it was significantly lower than immobility time observed in stressed, non- treated mice indicating thus that 9cDHRA displays dose effect in controlling this behavioural parameter. The effect of 9cDHRA was comparable with activity of synthetic panRXR agonist UVI2108, suggesting critical role of RXR activation in attaining anti-despair activity of by 9cDHRA. In addition to despair behaviors chronic stress induced also anhedonia reflected by absence of preference of sweetened drink which was consumed at the level not significantly different from 50% reflecting random choice (Figure 10.b). Anhedonia was abolished in stressed mice treated already with low dose of 9cDHRA (1mg/kg) as illustrated by sucrose preference significantly exceeding a chance level of 50%, although this preference was even more marked after treatment with higher dose of 3mg/kg of 9cDHRA. A panRXR agonist UVI2108 displayed activities similar to 9cDHRA supporting the involvement of RXR activation by 9cDHRA in antidepressant activities.

In a previous patent use of RXRg-/- was a negative control to show that 9cDHRA acts at RXRg to improve memory - in consequence in absence of RXRg it cannot improve memory. Rbp1-/- were used beacuse they show the same type of deficits as RXRg-/- suggetsing that RXRg signlaing is down. By showing that 9cDHRA can nomalise their memory was a proof of concept that these mice are missing RXR ligand and not the receptor itself (or receptor functionality). Even in previous experiments we showed that 9cDHRA can also improve memory of WT mice suggesting that it could work as memory enhancer in healthy subjects, but also could be used as memory enhancer in AD. However 9cDHRA may have teratogenic effect so we test precursors whch are most probably derived of such teratogenic activities.

### Example 7.4: 9CDHROL or 9CDHBC prevent depressive behaviours in chronic stress model of depression (Figure 18):

Stress is an important environmental factor in the aetiology of depression. To test efficiency of 9cDHROL and 9cDHBC for treatment of depressive behaviours induced by stress, we used social defeat stress animal model (Berton et al., 2006, Hollis and Kabbaj, 2014). Ten days of short daily physical contacts with resident dominant CD1 male followed by subsequent sensory contact efficiently induced despair in the forced swim task (fig. 18.A). Treatment with 10mg/kg of 9CDHROL or 10mg/kg of 9CDHBC during the stress protocol efficiently normalised immobility time which was comparable with control non-stressed mice. In addition to despair behaviour's chronic stress induced also anhedonia reflected by absence of preference of sweetened drink which was consumed at the level not significantly different from 50% reflecting random choice (fig. 18.B). Anhedonia was abolished in stressed mice treated with 10mg/kg of 9CDHROL or 10mg/kg of 9CDHBC during the stress protocol as illustrated by sucrose preference significantly exceeding a chance level of 50%.

### SUMMARY AND INDUSTRIAL APPLICABILITY

Vitamin A is a cluster of derivatives which can be converted in the body to visual pigments and ligands for nuclear hormone receptors. The vitamin A1 (retinol) is well known to be a precursor for the RAR ligand all-trans retinoic acid (ATRA), while the identity of the precursor of 9-cis-13,14 dihydroretinoic acid (9CDHRA), the recently discovered endogenous ligand of the retinoid X receptor (RXR) is unknown. In this study we show that nutritional precursors of 9cDHRA are 9-cis-13,14-dihydroretinol (9CDHROL) and 9-cis-13,14-dihydro-beta carotene (9CDHBC), which are respectively novel types of retinoid and carotenoid never described up to date, but present at high levels in food matrix and endogenously in mammalian organisms, including human. Using in vitro and in vivo experiments we demonstrate that such precursors might be directly or indirectly metabolized to 9CDHRA. In contrast, well know endogenous retinoids / carotenoids like all-trans-retinol, 9-cis-retinol and all-trans-β-carotene are only weak and non-selective precursors of 9CDHRA. We also demonstrate that known endogenous carotenoid 9-cis-β-carotene (9CBC) only weakly transformed to 9CDHBC via dehydrogenation. However, 9CDHBC is readily turned into 9cDHRA, and as such can be proposed as an indirect precursor of RXR ligand - 9cDHRA. Through our metabolic screen we established for the first time that 9CDHRA-esters, 9CDHROL, 9CDHROL-ester, 9CDHBC are excellent to fair nutritional and physiologically relevant selective precursors of the endogenous RXR-ligand 9CDHRA to induce RXR-mediated signaling. We further described this new class of substances as a new independent and selective new type of vitamin A, named hereafter Vitamin A5 for 9cDHROL or pro-vitamin A5 for 9CDHBC. The proof of concept for preventive / pharmaceutical usage of those compounds is shown in treatment of depressive-like behaviors in chronic stress animal model of depression. Similar use of these compounds can be envisaged for various diseases where RXR-mediated signalling is affected or was proposed as therapeutic target. Such diseases may include neurodegenerative and metabolic diseases, skin- and immunological dysfunctions (including inflammation) as well as cardio-vascular diseases, but may also concern life-style applications like memory enhancing effects.

### REFERENCES

| |
|---|
| Gennaro (ed.) Remington "Pharmaceutical Sciences", 17 Ed., Mack Publishing Co., Easton, Pennsylvania, 1985. |
| van Neerven S. 2008 RAR/RYR and PPAR/RXR signaling in neurological and psychiatric diseases. Prog Neu-robiol. 85(4):433-51. |
| Shudo K et al. 2009 Towards Retinoid Therapy for Alzheimer's Disease Curr Alzheimer Res.6(3): 302-311 |
| Skerrett R, Pellegrino MP, Casali BT, Taraboanta L, Landreth GE. J Biol Chem. 2015 Combined Liver X Receptor/Peroxisome Proliferator-activated Receptor γ Agonist Treatment Reduces Amyloid β Levels and Improves Behavior in Amyloid Precursor Protein/Presenilin 1 Mice. 290(35) 21591-602 |
| Ching Kuang Chow 2007 Fatty Acids in Foods and their Health Implications.,Third Edition Series: Food Science and Technology by CRC Press ISBN 9780849372612 |
| Arild C RustaN, and Christian A Drevon, Fatty Acids 2005 Structures and Properties ENCYCLOPEDIA OF LIFE SCIENCES. John Wiley & Sons, doi: 10.1038/npg.els.0003894 |
| Moise AR, Domínguez M, Alvarez S, Alvarez R, Schupp M, et al. (2008) Stereospecificity of Retinol Saturase: Absolute Configuration, Synthesis, and Biological Evaluation of Dihydroretinoids. J Am Chem Soc 130: 1154-1155. |
| Leonard J, Mohialdin S, Reed D, Ryan G, Swain PA (1995) Stereoselective conjugate addition of organolithium and organocopper reagents to [delta]-oxygenated [alpha],[beta]-unsaturated carbonyl systems derived from glyceraldehyde acetonide. Tetrahedron 51: 12843-12858. |
| Aissa C (2009) Mechanistic Manifold and New Development of the Julia-Kocienski Reaction. Eur J Org Chem: 1831-1844. |
| Schultz HS, Freyermuth HB, Buc SR (1963) New Catalysts for the Oxidation of Sulfides to Sulfones with Hydrogen Peroxide. J Org Chem 28: 1140-1142. |
| Blakemore PR (2002) The modified Julia olefination: alkene synthesis via the condensation of metallated het-eroarylalkylsulfones with carbonyl compounds. J Chem Soc Perkin Trans 1: 2563-2585. |
| Babino, D., M. Golczak, P.D. Kiser, A. Wyss, K. Palczewski, and J. von Lintig. 2016. The Biochemical basis of vitamin A3 production in athopod vision. ACS Chem. Biol. (in press) |
| Berton O. et al. 2006 Essential Role of BDNF in the Mesolimbic Dopamine Pathway in Social Defeat Stress. Science 311(5762) 864-868 |
| Cama, H.R., P.D. Dalvi, R.A. Morton, and M.K. Salah. 1952. Studies in vitamin A. XXI. Retinene2 and vitamin A2. Biochem J 52:542-7. |
| Cama, H.R., P.D. Dalvi, R.A. Morton, and M.K. Salah. 1952. Studies in vitamin A. XXI. Retinene2 and vitamin A2. Biochem J 52:542-7. |
| Chawla, A., J.J. Repa, R.M. Evans, and D.J. Mangelsdorf. 2001. Nuclear receptors and lipid physiology: opening the X-files. Science 294:1866-70. |
| Dalvi A, Lucki I (1999) Murine models of depression. Psychopharmacology 147:14-16 |
| de Lera AR, Krezel W, Riihl R. 2016. An Endogenous Mammalian Retinoid X Receptor Ligand, At Last! ChemMedChem 11(10):1027-1037. |
| de Lera, A.R., W. Krezel, and R. Rühl. 2016. An endogenous mammalian RXR ligand, at last ! ChemMedChem 2016, 11, 1027 - 1037 |
| Desvergne B. 2007. RXR: from partnership to leadership in metabolic regulations. Vitam Horm 75:1-32. |
| Diagnostic and Statistical Manual of Mental Disorders, Fourth Edition, (DSM IV) American Psychiatric Association. |
| Domínguez B, Pazos Y, de Lera AR (2000) Stereocontrolled Synthesis of 6-s-cis and 6-s-trans-Locked 9Z-Retinoids by Hydroxyl-Accelerated Stille Coupling of (Z)-Tri-n-Butylstannylbut-2-en-1-ol and Bicyclic Dienyl Triflates. J Org Chem 65: 5917-5925. |
| Edisbury, J.R., M. R.A., and S. G.W. 1937. A possible Vitam A2. Nature 140:234-234. |
| Evans, R. M. and Mangelsdorf, D. J. 2014 Cell, 157, 255. |
| Evans, R.M., and D.J. Mangelsdorf. 2014. Nuclear Receptors, RXR, and the Big Bang. Cell 157:255-66. |
| Feutz AC, Pham-Dinh D, Allinquant B, Miehe M, Ghandour MS. 2001. An immortalized jimpy oligodendrocyte cell line: defects in cell cycle and cAMP pathway. Glia 34(4):241-252. |
| Ghyselinck NB, Bavik C, Sapin V, Mark M, Bonnier D, et al. (1999) Cellular retinol-binding protein I is essential for vitamin A homeostasis. Embo J 18: 4903-4914. |
| Giguere, V., E.S. Ong, P. Segui, and R.M. Evans. 1987. Identification of a receptor for the morphogen retinoic acid. Nature 330:624-9. |
| Gillam, A.E., I.M. Heilbron, W.E. Jones, and E. Lederer. 1938. On theoccurrence and constitution of the 693 mu chromogen (Vitamn A2 ?) of fish liver oils. Biochemical Journal 32:405-416. |
| Goldstein, J.T., A. Dobrzyn, M. Clagett-Dame, J.W. Pike, and H.F. DeLuca. 2003. Isolation and characterization of unsaturated fatty acids as natural ligands for the retinoid-X receptor. Arch Biochem Biophys 420:185-93. |
| Heyman, R.A., D.J. Mangelsdorf, J.A. Dyck, R.B. Stein, G. Eichele, R.M. Evans, and C. Thaller. 1992. 9-cis retinoic acid is a high affinity ligand for the retinoid X receptor. Cell 68:397-406. |
| Hollis F, Kabbaj M. 2014 Social defeat as an animal model for depression. ILAR J. 55(2) 221-32. |
| Kai Z J 2011 Nanosci Nanotechnol. Synthesis of retinyl palmitate catalyzed by Candida sp.99-125 lipase immobilized on fiber-like SBA-15 mesoporous material. 11(9):7593-602. |
| Karrer, P., R. Morf, and K. Schöpp. 1931a. Zur Kenntnis des Vitamin A aus Fischtranen II. Helvetica Chimica Acta 14:1431-1436. |
| Kastner, P., M. Mark, N. Ghyselinck, W. Krezel, V. Dupe, J.M. Grondona, and P. Chambon. 1997a. Genetic evidence that the retinoid signal is transduced by heterodimeric RXR/RAR functional units during mouse development. Development 124:313-26. |
| Kastner, P., N. Messaddeq, M. Mark, O. Wendling, J.M. Grondona, S. Ward, N. Ghyselinck, and P. Chambon. 1997b. Vitamin A deficiency and mutations of RXRalpha, RXRbeta and RARalpha lead to early differentiation of embryonic ventricular cardiomyocytes. Development 124:4749-58. |
| Kitareewan, S., L.T. Burka, K.B. Tomer, C.E. Parker, L.J. Deterding, R.D. Stevens, B.M. Forman, D.E. Mais, R.A. Heyman, T. McMorris, and C. Weinberger. 1996. Phytol metabolites are circulating dietary factors that activate the nuclear receptor RXR. Mol Biol Cell 7:1153-66. |
| Kliewer, S.A., K. Umesono, D.J. Mangelsdorf, and R.M. Evans. 1992. Retinoid X receptor interacts with nuclear receptors in retinoic acid, thyroid hormone and vitamin D3 signalling. Nature 355:446-9. |
| Liu ZQ 2015 Appl Microbiol Biotechnol. Efficient two-step chemo-enzymatic synthesis of all-trans-retinyl palmitate with high substrate concentration and product yield. 99(21):8891-902 |
| Mangelsdorf, D.J., and R.M. Evans. 1995. The RXR heterodimers and orphan receptors. Cell 83:841-50. |
| Mangelsdorf, D.J., C. Thummel, M. Beato, P. Herrlich, G. Schutz, K. Umesono, B. Blumberg, P. Kastner, M. |
| Mark, P. Chambon, and R.M. Evans. 1995. The nuclear receptor superfamily: the second decade. Cell 83:835-9. |
| Moise, A.R., A. Isken, M. Dominguez, A.R. de Lera, J. von Lintig, and K. Palczewski. 2007. Specificity of zebrafish retinol saturase: formation of all-trans-13,14-dihydroretinol and all-trans-7,8- dihydroretinol. Biochemistry 46:1811-20. |
| Moise, A.R., A. Isken, M. Dominguez, A.R. de Lera, J. von Lintig, and K. Palczewski. 2007. Specificity of zebrafish retinol saturase: formation of all-trans-13,14-dihydroretinol and all-trans-7,8- dihydroretinol. Biochemistry 46:1811-20. |
| Moise, A.R., V. Kuksa, Y. Imanishi, and K. Palczewski. 2004. Identification of all-trans-retinol:all-trans-13,14-dihydroretinol saturase. J Biol Chem 279:50230-42. |
| Moise, A.R., V. Kuksa, W.S. Blaner, W. Baehr, and K. Palczewski. 2005. Metabolism and transactivation activity of 13,14-dihydroretinoic acid. J Biol Chem 280:27815-25. |
| Moise, A.R., S. Alvarez, M. Dominguez, R. Alvarez, M. Golczak, G.P. Lobo, J. von Lintig, A.R. de Lera, and K. Palczewski. 2009. Activation of retinoic acid receptors by dihydroretinoids. Mol Pharmacol 76:1228-37. |
| Moreau, J. L. 1997 Validation of an animal model of anhedonia, a core symptom of depression. Encephale, vol. 23, no. 4, pp. 280-289, |
| Nunez, V., D. Alameda, D. Rico, R. Mota, P. Gonzalo, M. Cedenilla, T. Fischer, L. Bosca, C.K. Glass, A.G. Arroyo, and M. Ricote. 2010. Retinoid X receptor alpha controls innate inflammatory responses through the up-regulation of chemokine expression. Proc Natl Acad Sci U S A 107:10626-31. |
| O'Byme, Sheila M. and Blaner, William S., 2013 The Journal of Lipid Research, Retinol and retinyl esters: biochemistry and physiology Thematic Review Series: Fat-Soluble Vitamins: Vitamin A 54, 1731-1743 |
| Pazos Y, de Lera AR (1999) Stereoselective Synthesis of 9-cis-Retinoic Acid Based on Stepwise or Convergent Suzuki Coupling Reactions. Tetrahedron Lett 40: 8287-8290. |
| Pazos Y, Iglesias B, de Lera AR (2001) The Suzuki coupling reaction in the stereocontrolled synthesis of 9-cis-retinoic acid and its ring-demethylated analogues. J Org Chem 66: 8483-8489. |
| Petkovich, M., N.J. Brand, A. Krust, and P. Chambon. 1987. A human retinoic acid receptor which belongs to the family of nuclear receptors. Nature 330:444-50. |
| Riihl R, Krzyzosiak A, Niewiadomska-Cimicka A, Rochel N, Szeles L, Vaz B, Wietrzych-Schindler M, Alvarez S, Szklenar M, Nagy L, de Lera AR, Krezel W. 2015. 9-cis-13,14-dihydroretinoic acid is an endogenous retinoid acting as RXR ligand in mice. PLoS Genet 11(6):e1005213. |
| Riihl R. 2006. Method to determine 4-oxo-retinoic acids, retinoic acids and retinol in serum and cell extracts by liquid chromatography/diode-array detection atmospheric pressure chemical ionisation tandem mass spectrometry. Rapid Commun Mass Spectrom 20(16):2497-2504. |
| Shirley M A et al. 1996 Oxidative and reductive metabolism of 9-cis-retinoic acid in the rat. Identification of 13,14-dihydro-9-cis-retinoic acid and its taurine conjugate. Drug Metab Dispos. 24(3):293-302 |
| Shulman, A.I., and D.J. Mangelsdorf. 2005. Retinoid x receptor heterodimers in the metabolic syndrome. N Engl J Med 353:604-15. |
| Sorg A, Brückner R (2005) Unexpected cis-Selectivity in (Sylvestre) Julia Olefinations with Bu3 Sn-Containing Allyl Benzothiazolyl Sulfones: Stereoselective Synthesis of 1,3-Butadienyl- and 1,3,5-Hexatrienylstannanes. Synlett 2005: 289,293. |
| Stephensen, C.B., A.D. Borowsky, and K.C. Lloyd. 2007. Disruption of Rxra gene in thymocytes and T |
| lymphocytes modestly alters lymphocyte frequencies, proliferation, survival and T helper type 1/type 2 balance. Immunology 121:484-98. |
| Szanto A, Narkar V, Shen Q, Uray IP, Davies PJ, Nagy L. 2004. Retinoid X receptors: X-ploring their (patho)physiological functions. Cell Death Differ 11 Suppl 2:S126-143. |
| Ulven, S.M., T.E. Gundersen, A.K. Sakhi, J.C. Glover, and R. Blomhoff. 2001. Quantitative axial profiles of retinoic acid in the embryonic mouse spinal cord: 9-cis retinoic acid only detected after all-trans-retinoic acid levels are super-elevated experimentally. Dev Dyn 222:341-53. |
| Vahlquist, A., J.B. Lee, G. Michaelsson, and O. Rollman. 1982. Vitamin A in human skin: II Concentrations of carotene, retinol and dehydroretinol in various components of normal skin. J Invest Dermatol 79:94-7. |
| Vaz B, Alvarez R, Souto JA, de Lera AR (2005) γ-Allenyl Allyl Benzothiazole Sulfonyl Anions Undergo cis-Selective (Sylvestre) Julia Olefinations. Synlett 2005: 294,298. |
| Wan, Y.J., G. Han, Y. Cai, T. Dai, T. Konishi, and A.S. Leng. 2003. Hepatocyte retinoid X receptor-alpha-deficient mice have reduced food intake, increased body weight, and improved glucose tolerance. Endocrinology 144:605-11. |
| Wietrzych M, Meziane H, Sutter A, Ghyselinck N, Chapman PF, Chambon P, Krezel W. 2005. Working memory deficits in retinoid X receptor gamma-deficient mice. Learn Mem 12(3):318-326. |
| Wietrzych-Schindler M, Szyszka-Niagolov M, Ohta K, Endo Y, Perez E, de Lera AR, Chambon P, Krezel W. 2011. Retinoid x receptor gamma is implicated in docosahexaenoic acid modulation of despair behaviors and working memory in mice. Biol Psychiatry 69(8):788-794. |
| Yin Chunhua 2006 Chinese J. Chem. Eng. Synthesis of Vitamin A Esters by Immobilized Candida sp. Lipase in Organic Media 14(1) 81-86 |
| |
| Patent publications: |
| WO 95/04018 A1 |
| WO 95/32946 A1 |
| WO2011/034551 A2 |
| WO2013/134867 A1 |

## Claims

1. A compound of general formula (I) 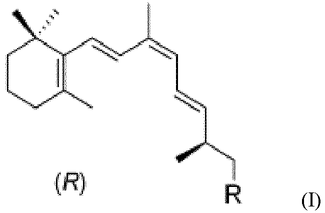 wherein R is -CH₂OR₂ wherein
R₂ is H or an acyl group -C(O)R₃ wherein -C(O)R₃ is a group which is removed by hydrolysis in a mammalian tissue or organ to result in (*R*)-9-cis-13,14-dihydroretinol and a biologically acceptable tolerable compound
said compound being converted into (*R*)-9-cis-13,14-dihydroretinoic acid in a mammalian tissue or organ or cells, once administered.

2. The compound of general formula (I) according to claim 1 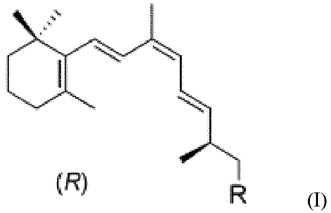 wherein R is -CH₂OR₂, wherein
R₂ is H or an acyl group -C(O)R₃ wherein R₃ is a C₁₋₂₅ alkyl or a C₂₋₂₅ alkenyl,
wherein said compound is converted into 9-*cis*-13,14-dihydroretinol in mammalian tissue or organ or cells, once administered
said compound being converted into (*R*)-9-*cis*-13,14-dihydroretinoic acid in a mammalian tissue or organ, once administered.

3. The compound according to claim 2 wherein said compound is
- a compound of general formula (3) 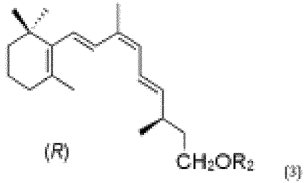 wherein R₂ is H or an acyl group -C(O)R₃ wherein R₃ is selected from a C₁₋₂₃ alkyl, preferably a C₁₋₆ alkyl and a C₉₋₂₃ alkyl, more preferably C₁₋₄ alkyl and a C₁₁₋₂₁ alkyl and a C₂₋₂₅ alkenyl, preferably a C₂₋₆ alkenyl and a C₁₋₂₃ alkenyl, more preferably a C₁₃₋₂₃ alkenyl.

4. The compound according to claim 3, wherein said compound is of general formula (4) 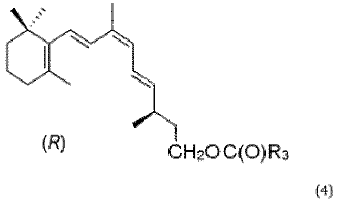 wherein R₃ is selected from
- a C₁₋₄ alkyl, preferably methyl, ethyl, propyl or isopropyl,
- a C₁₁₋₂₁ alkyl, preferably C₁₃₋₁₉ alkyl and
- a C₁₁₋₂₃ alkenyl, preferably a polyunsaturated C₁₃₋₂₃ alkenyl.

5. A compound of general formula (I) for use in therapy in a mammalian subject, 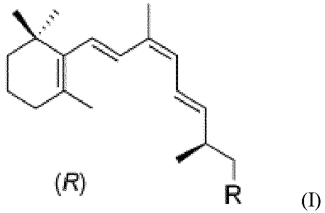 wherein R is selected from a group of general formula (A), -CH₂OR₂ and -COOR₁ wherein
R is a group of general formula (A) 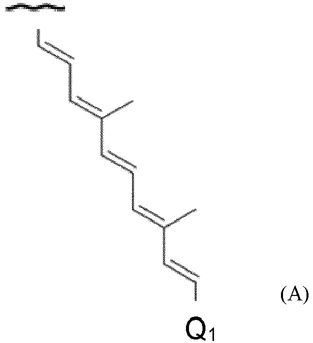
wherein Q₁ is a substituted or unsubstituted C₆₋₁₀ alkenyl or cycloalkenyl, preferably a substituted or unsubstituted trimethylcycloalkenyl or more preferably a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl, and/or
R₂ is H or an acyl group -C(O)R₃ wherein -C(O)R₃ is a group which is removed by hydrolysis in a mammalian tissue or organ to result in (*R*)-9-*cis*-13,14-dihydroretinol and a biologically acceptable tolerable compound and/or
R₁ is a group which is removed by hydrolysis in a mammalian tissue or organ to result in (*R*)-9-*cis*-13,14-dihydroretinoic acid and a biologically acceptable tolerable compound,
said compound being converted into RXR-ligand (*R*)-9-*cis*-13,14-dihydroretinoic acid in a mammalian tissue or organ or cells, once administered.

6. The compound for use according to claim 5, wherein the compound is of following general formula (I): 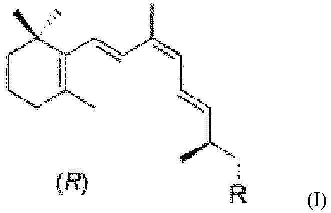 wherein R is selected from a group of general formula (A), -CH₂OR₂, and -COOR₁ wherein
R is a group of general formula (A): 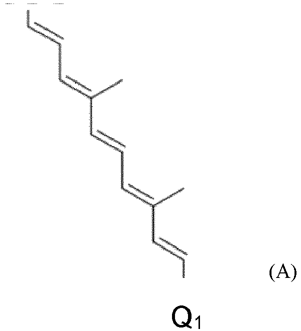
wherein Q₁ is a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl, wherein if the trimethylcyclohexenyl, e.g. 2,6,6-trimethylcyclohexenyl group is substituted it is preferably hydroxyl-substituted or oxosubstituted, preferably oxosubstituted;
wherein said compound is converted into (*R*)-9-*cis*-13,14-dihydroretinol in mammalian tissue or organ or cells, once administered
and/or
R₂ is H or an acyl group -C(O)R₃ wherein R₃ is a C₁₋₂₅ alkyl or a C₂₋₂₅ alkenyl,
wherein said compound is converted into (*R*)-9-*cis*-13,14-dihydroretinol in mammalian tissue or organ or cells, once administered
and/or
R₁ is a C₁₋₂₅ alkyl or a C₂₋₂₅ alkenyl,
said compound being converted into (*R*)-9-*cis*-13,14-dihydroretinoic acid in a mammalian tissue or organ, once administered.

7. The compound for use according to claim 6 wherein said compound is
- a compound of general formula (3) 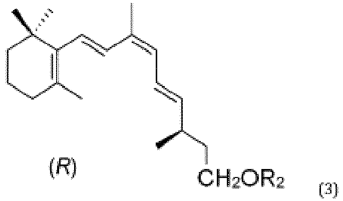 wherein R₂ is H or an acyl group -C(O)R₃ wherein R₃ is selected from a
C₁₋₂₃ alkyl, preferably a C₁₋₆ alkyl and a C₉₋₂₃ alkyl, more preferably C₁₋₄ alkyl and a C₁₁₋₂₁ alkyl and a C₂₋₂₅ alkenyl, preferably a C₂₋₆ alkenyl and a C₁₋₂₃ alkenyl, more preferably a C₁₃₋₂₃ alkenyl, and/or
- a compound of general formula (5) 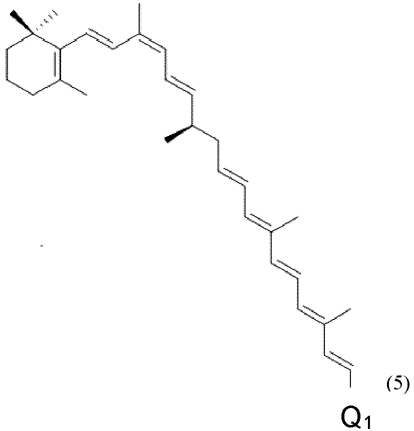
wherein Q₁ is a substituted or unsubstituted C₆₋₁₀ alkenyl or cycloalkenyl, preferably a substituted or unsubstituted trimethylcycloalkenyl or more preferably a substituted or unsubstituted 2,6,6-trimethylcyclohexenyl, even more preferably unsubstituted 2,6,6-trimethylcyclohex-1-en-1-yl or 2,6,6-trimethylcyclohex-2-en-1-yl, if the trimethylcyclohexenyl, e.g. 2,6,6-trimethylcyclohexenyl group is substituted it is preferably hydroxyl-substituted or oxosubstituted, preferably oxosubstituted;
wherein said compound is converted into (*R*)-9-*cis*-13,14-dihydroretinol in mammalian tissue or organ or cells, once administered.

8. The compound for use according to claim 6 wherein said compound is of general formula (2) 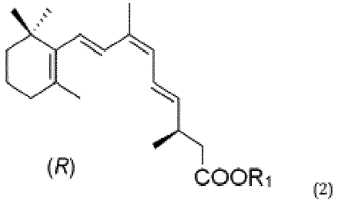 wherein R₁ is selected from a
C₁₋₂₃ alkyl, preferably a C₁₋₆ alkyl and a C₉₋₂₃ alkyl, more preferably C₁₋₄ alkyl and a C₁₁₋₂₁ alkyl and a C₂₋₂₄ alkenyl, preferably a C₂₋₆ alkenyl and a C₉₋₂₃ alkenyl, more preferably a C₁₃₋₂₃ alkenyl, wherein preferably R₁ is selected from methyl, ethyl, propyl or isopropyl optionally methyl, propyl or isopropyl.

9. The compound for use according to claim 7, wherein said compound is of general formula (4) 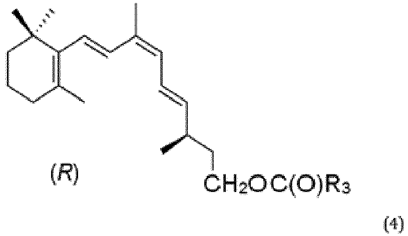 wherein R₃ is selected from
- a C₁₋₄ alkyl, preferably methyl, ethyl, propyl or isopropyl,
- a C₁₁₋₂₁ alkyl, preferably C₁₃₋₁₉ alkyl and
- a C₁₁₋₂₃ alkenyl, preferably a polyunsaturated C₁₃₋₂₃ alkenyl.

10. The compound for use according to claim 5 or 6 which is 9-cis-13,14-dihydro-beta,beta-carotene.

11. A pharmaceutical composition or a nutraceutical composition for use in therapy of a mammalian subject, preferably in the prevention or treatment of a retinoid X receptor (RXR) mediated signaling dysfunction in a mammalian subject, said composition comprising the compound as defined in any of claims 1 to 4 or the compound for use as defined in any of claims 5 to 9 or the 9-cis-carotenoid according to claim 10, said composition also comprising one or more pharmaceutically or nutraceutically acceptable additive(s) and/or excipient(s).

12. The compound or pharmaceutical or nutraceutical composition recited in any of claims 1 to 11 for use in therapy as a selective precursor of the endogenous RXR-ligand in the prevention or treatment of a retinoid X receptor (RXR) mediated signaling dysfunction in a mammalian subject; preferably for use in the prevention and/or treatment of a disease selected from central nervous system related diseases and peripheral nervous system related diseases, or
for use in the therapeutic prevention and/or treatment of
- learning and/or memory impairment, wherein preferably said memory is working memory,
- impaired cognitive functions or impaired learning and memory,
- depression, or
for use in the prevention and/or treatment of a neurodegenerative disorder, preferably a neurodegenerative disorder selected from Alzheimer's disease, Parkinson's disease, Mild Cognitive Impairment (MCI), Parkinson's disease with MCI, Huntington's disease, Dementia with Lewy bodies (DLB), Amyotrophic lateral sclerosis (ALS), and other neurodegenerative related dementias due to changes in the brain caused by ageing, disease or trauma; or spinal cord injury and ataxias, disseminated sclerosis and Multiple sclerosis (MS) or other neurological conditions.

13. A nutraceutical composition for use in the therapeutic prevention of retinoid X receptor (RXR) mediated signaling dysfunction depending on a ligand depending vitamin A5 deficiency in a mammalian subject, for general Vitamin AS-supplementation, said composition comprising 9-cis-beta,beta-carotene (9CBC) in an amount or concentration higher than it is present in the natural food matrix and/or comprises additional amount of the compound.

14. A non-medical use of a compound or pharmaceutical or nutraceutical composition as recited in any of claims 1-12, preferably for enhancing memory performance, wherein preferably said memory is working memory, or as a food ingredient or as a dietary supplement for maintaining health of a subject.

15. A 9-*cis*-beta-carotene (9CBC) or a 9CBC-containing functional food for use in therapy as a carotenoid precursor of 9-*cis*-13,14-dihydrobeta-carotene (9CDHBC), as a food ingredient or as a nutraceutical, in therapeutically preventing a ligand-depending Vitamin AS-deficiency and/or for general Vitamin AS-supplementation, or as a food supplement to activate RXR at physiological relevant level of 9CDHBC and/or 9CDHRA in the human body, wherein preferably 9CBC is used in the form of a composition comprising said compound in an amount or concentration higher than it is present in the natural food matrix and/or comprises additional amount of the compound.

16. A nutraceutical composition for use in the therapeutic prevention of retinoid X receptor (RXR) mediated signaling dysfunction depending on a ligand depending vitamin A5 deficiency in a mammalian subject, said composition comprising 9-cis-beta,beta-carotene (9CBC) in an amount or concentration higher than it is present in the natural food matrix and/or comprises additional amount of the compound.

## Patentansprüche

1. Eine Verbindung der allgemeinen Formel (I) 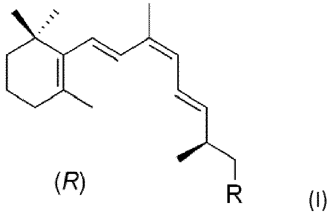 worin R -CH₂OR₂ ist, worin
R₂ H oder eine Acylgruppe -C(O)R₃ ist, wobei -C(O)R₃ eine Gruppe ist, die durch Hydrolyse in einem Säugetiergewebe oder -organ entfernt wird, um (R)-9-*cis*-13,14-Dihydroretinol und eine biologisch verträgliche Verbindung zu ergeben
wobei die Verbindung in (R)-9-*cis*-13,14-Dihydroretinsäure in einem Säugetiergewebe oder -organ oder in Zellen umgewandelt wird, sobald sie verabreicht wird.

2. Die Verbindung der allgemeinen Formel (I) nach Anspruch 1 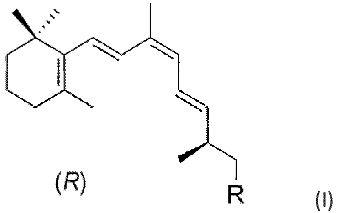 worin R CH₂OR₂ ist, worin
R₂ H oder eine Acylgruppe C(O)R₃ ist, worin R₃ ein C₁₋₂₅-Alkyl oder ein C₂₋₂₅-Alkenyl ist,
wobei die Verbindung in 9-*cis*-13,14-Dihydroretinol in Säugetiergewebe oder -organ oder -zellen umgewandelt wird, sobald sie verabreicht wird
wobei die Verbindung in (R)-9-*cis*-13,14-Dihydroretinsäure in einem Säugetiergewebe oder -organ umgewandelt wird, sobald sie verabreicht wird.

3. Verbindung nach Anspruch 2, wobei es sich bei der Verbindung um
- eine Verbindung der allgemeinen Formel (3) 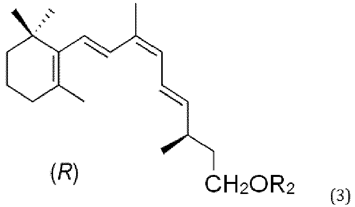
worin R₂ H oder eine Acylgruppe C(O)R₃ ist, worin R₃ ausgewählt ist aus einem
C₁₋₂₃-Alkyl, vorzugsweise einem C₁₋₆-Alkyl und einem C₉₋₂₃-Alkyl, besonders bevorzugt einem C₁₋₄-Alkyl und einem C₁₁₋₂₁-Alkyl und einem C₂₋₂₅-Alkenyl, vorzugsweise einem C₂₋₆-Alkenyl und einem C₁₋₂₃-Alkenyl, besonders bevorzugt einem C₁₃₋₂₃-Alkenyl.

4. Verbindung nach Anspruch 3, wobei die Verbindung die allgemeine Formel (4) hat 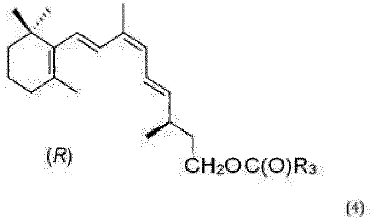 worin R₃ ausgewählt ist aus
- einem C₁₋₄-Alkyl, vorzugsweise Methyl, Ethyl, Propyl oder Isopropyl,
- einem C₁₁₋₂₁-Alkyl, vorzugsweise C₁₃₋₁₉-Alkyl und
- einem C₁₁₋₂₃-Alkenyl, vorzugsweise einem mehrfach ungesättigten C₁₃₋₂₃-Alkenyl.

5. Verbindung der allgemeinen Formel (I) zur Verwendung in der Therapie bei einem Säugetiersubjekt, 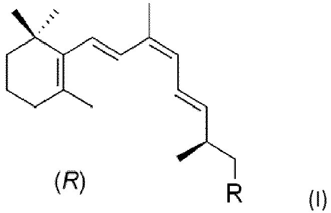 worin R aus einer Gruppe der allgemeinen Formel (A), -CH₂OR₂ und -COOR₁ ausgewählt ist, worin
R eine Gruppe der allgemeinen Formel A ist 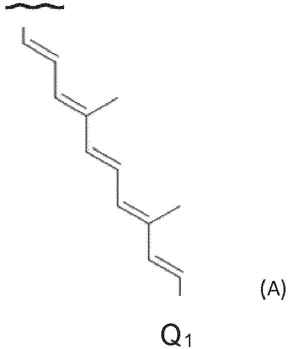
worin Q₁ ein substituiertes oder unsubstituiertes C₆₋₁₀-Alkenyl oder Cycloalkenyl, vorzugsweise ein substituiertes oder unsubstituiertes Trimethylcycloalkenyl oder noch bevorzugter ein substituiertes oder unsubstituiertes 2,6,6-Trimethylcyclohexenyl, noch bevorzugter ein unsubstituiertes 2,6,6-Trimethylcyclohex-1-en-1-yl oder 2,6,6-Trimethylcyclohex-2-en-1-yl ist, und/oder
R₂ H oder eine Acylgruppe -C(O)R₃ ist, wobei -C(O)R₃ eine Gruppe ist, die durch Hydrolyse in einem Säugetiergewebe oder -organ unter Bildung von (R)-9-*cis*-13,14-Dihydroretinol und einer biologisch verträglichen Verbindung entfernt wird, und/oder
R₁ eine Gruppe ist, die durch Hydrolyse in einem Säugetiergewebe oder -organ entfernt wird, um (R)-9-*cis*-13,14-Dihydroretinsäure und eine biologisch annehmbare verträgliche Verbindung zu ergeben, wobei diese Verbindung in einem Säugetiergewebe oder -organ oder in Zellen in den RXR-Liganden (R)-9-*cis*-13,14-Dihydroretinsäure umgewandelt wird, sobald sie verabreicht wird.

6. Verbindung zur Verwendung nach Anspruch 5, wobei die Verbindung die allgemeine Formel (I) hat 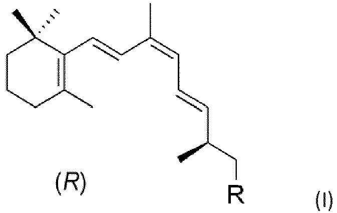 worin R aus einer Gruppe der allgemeinen Formel (A), CH₂OR₂ und COOR₁ ausgewählt ist, worin
R eine Gruppe der allgemeinen Formel A ist: 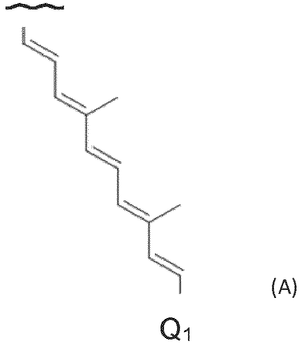
worin Q₁ ein substituiertes oder unsubstituiertes 2,6,6-Trimethylcyclohexenyl, noch bevorzugter ein unsubstituiertes 2,6,6-Trimethylcyclohex-1-en-1-yl oder 2,6,6-Trimethylcyclohex-2-en-1-yl ist, wobei, wenn das Trimethylcyclohexenyl, z. z. B. 2,6,6-Trimethylcyclohexenyl, substituiert ist, vorzugsweise hydroxylsubstituiert oder oxosubstituiert ist, vorzugsweise oxosubstituiert;
wobei die Verbindung in (R)-9-*cis*-13,14-Dihydroretinol in Säugetiergewebe oder -organ oder -zellen umgewandelt wird, sobald sie verabreicht wird
und/oder
R2 H oder eine Acylgruppe C(O)R₃ ist, worin R₃ ein C₁₋₂₅-Alkyl oder ein C₂₋₂₅-Alkenyl ist,
wobei die Verbindung in (R)-9-*cis*-13,14-Dihydroretinol in Säugetiergewebe oder -organen oder -zellen umgewandelt wird, sobald sie verabreicht wurde
und/oder
R₁ ein C₁₋₂₅-Alkyl oder ein C₂₋₂₅-Alkenyl ist,
wobei die Verbindung in einem Säugetiergewebe oder -organ nach Verabreichung in (R)-9-*cis*-13,14-Dihydroretinsäure umgewandelt wird.

7. Verbindung zur Verwendung nach Anspruch 6, wobei die Verbindung ist
- eine Verbindung der allgemeinen Formel (3) 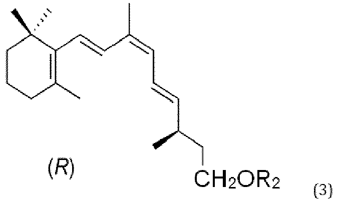 worin R₂ H oder eine Acylgruppe C(O)R₃ ist, worin R₃ ausgewählt ist aus einem
C₁₋₂₃-Alkyl, vorzugsweise einem C₁₋₆-Alkyl und einem C₉₋₂₃-Alkyl, besonders bevorzugt einem C₁₋₄-Alkyl und einem C₁₁₋₂₁-Alkyl und einem
C₂₋₂₅-Alkenyl, vorzugsweise einem C₂₋₆-Alkenyl und einem C₁₋₂₃-Alkenyl, besonders bevorzugt einem C₁₃₋₂₃-Alkenyl, und/oder
- eine Verbindung der allgemeinen Formel (5) 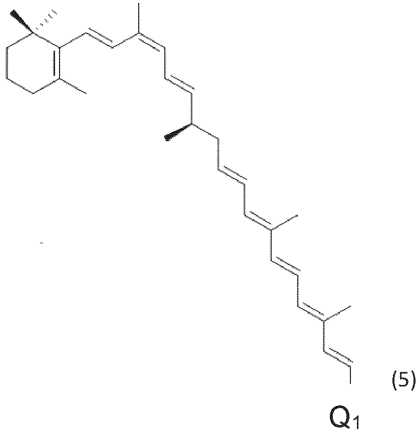
worin Q₁ ein substituiertes oder unsubstituiertes C₆₋₁₀-Alkenyl oder Cycloalkenyl, vorzugsweise ein substituiertes oder unsubstituiertes Trimethylcycloalkenyl oder noch bevorzugter ein substituiertes oder unsubstituiertes 2,6,6-Trimethylcyclohexenyl, noch bevorzugter ein unsubstituiertes 2,6,6-Trimethylcyclohex-1-en-1-yl oder 2,6,6-Trimethylcyclohex-2-en-1-yl ist. Wenn die Trimethylcyclohexenyl-, z.B. 2,6,6-Trimethylcyclohexenylgruppe substituiert ist, ist sie vorzugsweise hydroxylsubstituiert oder oxosubstituiert, vorzugsweise oxosubstituiert;
wobei die Verbindung in (R)-9-*cis*-13,14-Dihydroretinol in Säugetiergewebe oder -organ oder -zellen umgewandelt wird, sobald sie verabreicht wird.

8. Verbindung zur Verwendung nach Anspruch 6, wobei die Verbindung die allgemeine Formel (2) hat 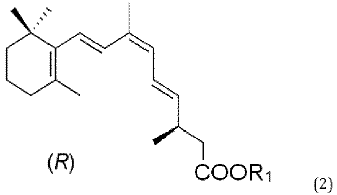 worin R₁ ausgewählt ist aus einem
C₁₋₂₃-Alkyl, vorzugsweise einem C₁₋₆-Alkyl und einem C₉₋₂₃-Alkyl, noch bevorzugter einem C₁₋₄-Alkyl und einem C₁₁₋₂₁-Alkyl und einem
C₂₋₂₄-Alkenyl, vorzugsweise einem C₂₋₆-Alkenyl und einem C₉₋₂₃-Alkenyl, besonders bevorzugt einem C₁₃₋₂₃-Alkenyl, ausgewählt ist, wobei R₁ vorzugsweise aus Methyl, Ethyl, Propyl oder Isopropyl, gegebenenfalls Methyl, Propyl oder Isopropyl ausgewählt ist.

9. Verbindung zur Verwendung nach Anspruch 8, wobei die Verbindung die allgemeine Formel (4) hat 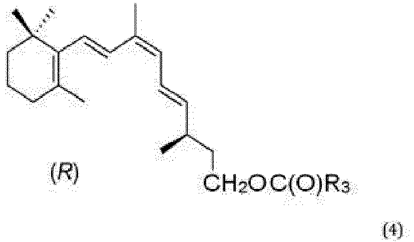 worin R₃ ausgewählt ist aus
- einem C₁₋₄-Alkyl, vorzugsweise Methyl, Ethyl, Propyl oder Isopropyl,
- einem C₁₁₋₂₁-Alkyl, vorzugsweise C₁₃₋₁₉-Alkyl und
- einem C₁₁₋₂₃-Alkenyl, vorzugsweise einem mehrfach ungesättigten C₁₃₋₂₃-Alkenyl.

10. Die Verbindung zur Verwendung nach Anspruch 5 oder 6, die 9-*cis*-13,14-Dihydro-beta,beta-Carotin ist.

11. Pharmazeutische Zusammensetzung oder nutrazeutische Zusammensetzung zur Verwendung in der Therapie eines Säugetieres, vorzugsweise zur Vorbeugung oder Behandlung einer durch den Retinoid-X-Rezeptor (RXR) vermittelten Signaldysfunktion in einem Säugetier, wobei die Zusammensetzung die in einem der Ansprüche 1 bis 5 definierte Verbindung oder die in einem der Ansprüche 6 bis 9 definierte Verbindung zur Verwendung oder das 9-cis-Carotinoid nach Anspruch 10 umfasst, wobei die Zusammensetzung auch einen oder mehrere pharmazeutisch oder nutrazeutisch annehmbare(n) Zusatzstoff(e) und/oder Trägerstoff(e) umfasst.

12. Verbindung oder pharmazeutische Zusammensetzung nach einem der Ansprüche 1 bis 11 zur therapeutischen Verwendung als selektiver Vorläufer des endogenen RXR-Liganden bei der Vorbeugung oder Behandlung einer Retinoid-X-Rezeptor (RXR)-vermittelten Signaldysfunktion in einem Säugetier; vorzugsweise zur Verwendung bei der Vorbeugung und/oder Behandlung einer Krankheit, ausgewählt aus mit dem zentralen Nervensystem zusammenhängenden Krankheiten und mit dem peripheren Nervensystem zusammenhängenden Krankheiten, oder
zur Verwendung bei der therapeutischen Prävention und/oder Behandlung von
- Lern- und/oder Gedächtnisstörungen, wobei das Gedächtnis vorzugsweise ein Arbeitsgedächtnis ist,
- gestörte kognitive Funktionen oder gestörtes Lernen und Gedächtnis,
- Depression, oder
zur Verwendung bei der Vorbeugung und/oder Behandlung einer neurodegenerativen Störung, vorzugsweise einer neurodegenerativen Störung, ausgewählt aus der Alzheimer-Krankheit, der Parkinson-Krankheit, der leichten kognitiven Beeinträchtigung (MCI), der Parkinson-Krankheit mit MCI, der Huntington-Krankheit, der Demenz mit Lewy-Körpern (DLB), der amyotrophen Lateralsklerose (ALS) und anderen neurodegenerativ bedingten Demenzen, die auf alters-, krankheits- oder traumabedingte Veränderungen im Gehirn zurückzuführen sind; oder Rückenmarksverletzungen und Ataxien, disseminierte Sklerose und Multiple Sklerose (MS) oder andere neurologische Erkrankungen.

13. Nutrazeutische Zusammensetzung zur Verwendung bei der therapeutischen Vorbeugung von Retinoid-X-Rezeptor (RXR)-vermittelter Signaldysfunktion in Abhängigkeit von einem ligandenabhängigen Vitamin-A5-Mangel bei einem Säugetier, zur allgemeinen Vitamin-A5-Supplementierung, wobei die Zusammensetzung 9-*cis*-beta,beta-Carotin (9CBC) in einer Menge oder Konzentration enthält, die höher ist als diejenige, die in der natürlichen Nahrungsmittelmatrix vorhanden ist, und/oder eine zusätzliche Menge der Verbindung umfasst.

14. Nicht-medizinische Verwendung einer Verbindung nach einem der Ansprüche 1 bis 5 oder der Verbindung, wie sie in einem der Ansprüche 6 bis 9 definiert ist, oder des 9-cis-Carotinoids zur Verwendung nach Anspruch 10 oder 11 oder der nutrazeutischen Zusammensetzung nach Anspruch 12, vorzugsweise zur Verbesserung der Gedächtnisleistung, wobei das Gedächtnis vorzugsweise das Arbeitsgedächtnis ist, oder als Nahrungsmittelbestandteil oder als Nahrungsergänzungsmittel zur Erhaltung der Gesundheit eines Subjekts.

15. 9-cis-Beta-Carotin (9CBC) oder ein 9CBC-haltiges funktionelles Lebensmittel zur therapeutischen Verwendung als Carotinoid-Vorläufer von 9-cis-13,14-Dihydro-Beta-Carotin (9CDHBC), als Lebensmittelzusatzstoff oder als Nutrazeutikum, zur therapeutischen Verhinderung eines ligandenabhängigen Vitamin-A5-Mangels und/oder zur allgemeinen Vitamin-A5-Supplementierung, oder als Nahrungsergänzungsmittel zur Aktivierung von RXR in physiologisch relevanter Höhe von 9CDHBC und/oder 9CDHRA im menschlichen Körper, wobei vorzugsweise 9CBC in Form einer Zusammensetzung verwendet wird, die die Verbindung in einer Menge oder Konzentration enthält, die höher ist als die in der natürlichen Nahrungsmittelmatrix vorhandene und/oder eine zusätzliche Menge der Verbindung umfasst.

16. Nutrazeutische Zusammensetzung zur Verwendung bei der therapeutischen Vorbeugung von Retinoid-X-Rezeptor (RXR)-Me-Signalisierungsdysfunktion in Abhängigkeit von einem ligandenabhängigen Vitamin-A5-Mangel in einem Säugetiersubjekt, wobei die Zusammensetzung 9-cis-beta,beta-Carotin (9CBC) in einer Menge oder Konzentration umfasst, die höher ist als diejenige, die in der natürlichen Nahrungsmittelmatrix vorhanden ist, und/oder eine zusätzliche Menge der Verbindung umfasst.

## Revendications

1. Composé de formule générale (I) 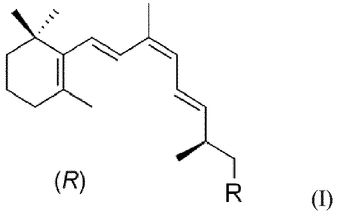 dans laquelle R est -CH₂OR₂ où
R₂ est H ou un groupe acyle -C(O)R₃ dans lequel -C(O)R₃ est un groupe qui est éliminé par hydrolyse dans un tissu ou un organe de mammifère pour donner du (*R*)-9-*cis*-13,14-dihydrorétinol et un composé tolérable biologiquement acceptable
ledit composé étant converti en acide (*R*)-9-*cis*-13,14-dihydrorétinoïque dans un tissu ou un organe ou des cellules de mammifère, une fois administré.

2. Composé de formule générale (I) selon la revendication 1 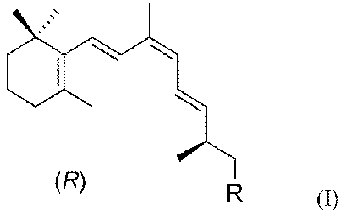 dans laquelle R est -CH₂OR₂, dans lequel
R₂ est H ou un groupe acyle -C(O)R₃ dans lequel R₃ est un alkyle en C₁₋₂₅ ou un alcényle en C₂₋₂₅, dans lequel ledit composé est converti en 9-cis-13,14-dihydrorétinol dans un tissu ou un organe ou des cellules de mammifère, une fois administré
ledit composé étant converti en acide (*R*)-9-*cis*-13,14-dihydrorétinoïque dans un tissu ou un organe de mammifère, une fois administré.

3. Composé selon la revendication 2, dans lequel ledit composé est
- un composé de formule générale (3) 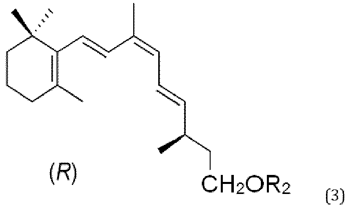
dans laquelle R₂ est H ou un groupe acyle -C(O)R₃ dans lequel R₃ est choisi parmi
un alkyle en C₁₋₂₃, de préférence un alkyle en C₁₋₆ et un alkyle en C₉₋₂₃, plus préférentiellement un alkyle en C₁₋₄ et un alkyle en C₁₁₋₂₁ et
un alcényle en C₂₋₂₅, de préférence un alcényle en C₂₋₆ et un alcényle en C₁₋₂₃, plus préférentiellement un alcényle en C₁₃₋₂₃.

4. Composé selon la revendication 3, dans lequel ledit composé est de formule générale (4) 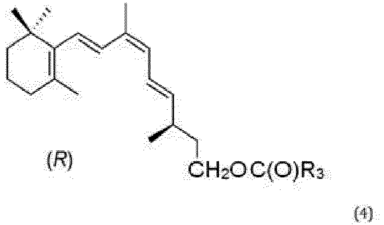 dans laquelle R₃ est choisi parmi
- un alkyle en C₁₋₄, de préférence un méthyle, un éthyle, un propyle ou un isopropyle,
- un alkyle en C₁₁₋₂₁, de préférence un alkyle en C₁₃₋₁₉, et
- un alcényle en C₁₁₋₂₃, de préférence un alcényle polyinsaturé en C₁₃₋₂₃.

5. Composé de formule générale (I) destiné à être utilisé dans la thérapie d'un sujet mammifère, 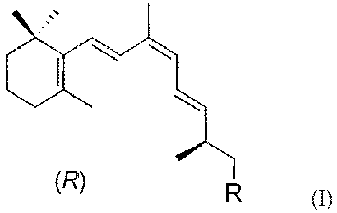 dans laquelle R est choisi parmi un groupe de formule générale (A), -CH₂OR₂ et -COOR₁ dans laquelle
R est un groupe de formule générale (A) 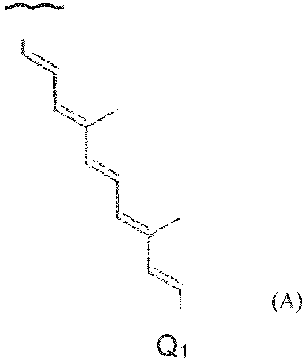
dans laquelle Q₁ est un alcényle ou cycloalcényle C₆₋₁₀ substitué ou non substitué, de préférence un triméthylcycloalcényle substitué ou non substitué ou plus préférentiellement un 2,6,6-triméthylcyclohexényle substitué ou non substitué, encore plus préférentiellement un 2,6,6-triméthylcyclohex-1-ène-1-yle ou un 2,6,6-triméthylcyclohex-2-ène-1-yle non substitué, et/ou
R₂ est H ou un groupe acyle -C(O)R₃ dans lequel -C(O)R₃ est un groupe qui est éliminé par hydrolyse dans un tissu ou un organe de mammifère pour donner du (*R*)-9-*cis*-13,14-dihydrorétinol et un composé tolérable biologiquement acceptable et/ou
R₁ est un groupe qui est éliminé par hydrolyse dans un tissu ou un organe de mammifère pour donner l'acide (*R*)-9-*cis*-13,14-dihydrorétinoïque et un composé tolérable biologiquement acceptable,
ledit composé étant converti en acide (R)-9-*cis*-13,14-dihydrorétinoïque, ligand du RXR dans un tissu ou un organe ou des cellules de mammifère, une fois administré.

6. Composé à utiliser selon la revendication 5, dans lequel le composé est de formule générale (I) : 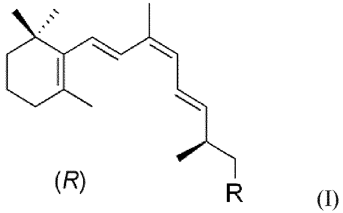 dans laquelle R est choisi parmi un groupe de formule générale (A), -CH₂OR₂, et -COOR₁ dans laquelle
R est un groupe de formule générale (A) : 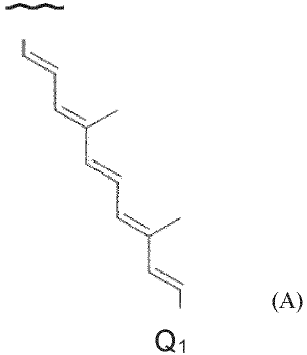
où Q₁ est un 2,6,6-triméthylcyclohexényle substitué ou non substitué, encore plus préférentiellement un 2,6,6-triméthylcyclohex-1-ène-1-yle ou un 2,6,6-triméthylcyclohex-2-ène-1-yle non substitué, où si le triméthylcyclohexényle, par exemple le groupe 2,6,6-triméthylcyclohexényle, est substitué, il est de préférence substitué par un hydroxyle ou par un oxosubstitut, de préférence par un oxosubstitut ;
dans lequel ledit composé est converti en (*R*)-9-*cis*-13,14-dihydrorétinol dans un tissu ou un organe ou des cellules de mammifère, une fois administré
et/ou
R₂ est H ou un groupe acyle -C(O)R₃ dans lequel R₃ est un alkyle en C₁₋₂₅ ou un alcényle en C₂₋₂₅, dans lequel ledit composé est converti en (*R*)-9-*cis*-13,14-dihydrorétinol dans un tissu, un organe ou des cellules de mammifère, une fois administré
et/ou
R₁ est un alkyle en C₁₋₂₅ ou un alcényle en C₂₋₂₅,
ledit composé étant converti en acide (*R*)-9-*cis*-13,14-dihydrorétinoïque dans un tissu ou un organe de mammifère, une fois administré.

7. Composé à utiliser selon la revendication 6, dans lequel ledit composé est
- un composé de formule générale (3) 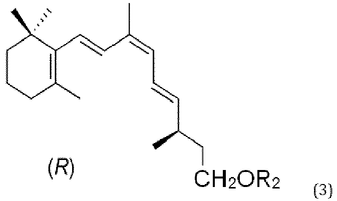
dans laquelle R₂ est H ou un groupe acyle -C(O)R₃ dans lequel R₃ est choisi parmi
un alkyle en C₁₋₂₃, de préférence un alkyle en C₁₋₆ et un alkyle en C₉₋₂₃, plus préférentiellement un alkyle en C₁₋₄ et un alkyle en C₁₁₋₂₁ et
un alcényle en C₂₋₂₅, de préférence un alcényle en C₂₋₆ et un alcényle en C₁₋₂₃, plus préférentiellement un alcényle en C₁₃₋₂₃, et/ou
- un composé de formule générale (5) 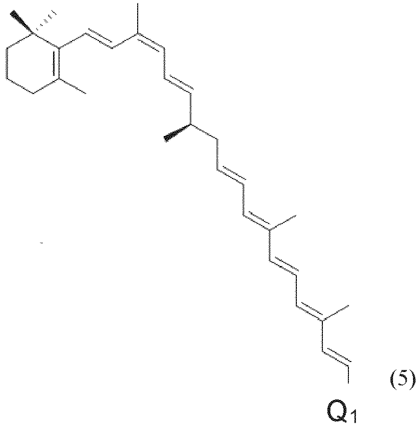
dans laquelle Q₁ est un alcényle ou un cycloalcényle en C₆₋₁₀ substitué ou non substitué, de préférence un triméthylcycloalcényle substitué ou non substitué ou plus préférentiellement un 2,6,6-triméthylcyclohexényle substitué ou non substitué, encore plus préférentiellement un 2,6,6-triméthylcyclohex-1-ène-1-yle ou un 2,6,6-triméthylcyclohex-2-ène-1-yle non substitué, si le triméthylcyclohexényle, par exemple le groupe 2,6,6-triméthylcyclohexényle, est substitué, il est de préférence hydroxyle-substitué ou oxo-substitué, de préférence oxo-substitué ;
dans lequel ledit composé est converti en (*R*)-9-*cis*-13,14-dihydrorétinol dans un tissu ou un organe ou des cellules de mammifère, une fois administré.

8. Composé à utiliser selon la revendication 6, dans lequel ledit composé est de formule générale (2) 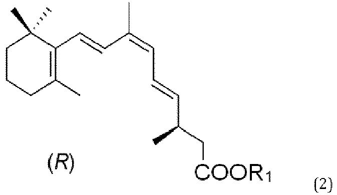 dans laquelle R₁ est choisi parmi
un alkyle en C₁₋₂₃, de préférence un alkyle en C₁₋₆ et un alkyle en C₉₋₂₃, plus préférentiellement un alkyle en C₁₋₄ et un alkyle en C₁₁₋₂₁, et
un alcényle en C₂₋₂₄, de préférence un alcényle en C₂₋₆ et un alcényle en C₉₋₂₃, plus préférentiellement un alcényle en C₁₃₋₂₃, dans laquelle R₁ est de préférence choisi parmi le méthyle, l'éthyle, le propyle ou l'isopropyle, éventuellement le méthyle, le propyle ou l'isopropyle.

9. Composé à utiliser selon la revendication 7, dans lequel ledit composé est de formule générale (4) 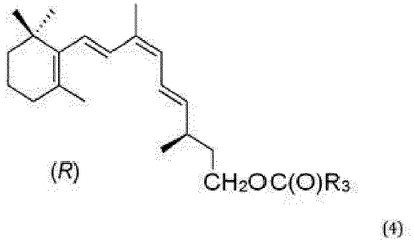 dans laquelle R₃ est choisi parmi
- un alkyle en C₁₋₄, de préférence un méthyle, un éthyle, un propyle ou un isopropyle,
- un alkyle en C₁₁₋₂₁, de préférence un alkyle en C₁₃₋₁₉, et
- un alcényle en C₁₁₋₂₃, de préférence un alcényle polyinsaturé en C₁₃₋₂₃.

10. Composé à utiliser selon la revendication 5 ou 6, qui est le 9-cis-13,14-dihydro-bêta,bêta-carotène.

11. Composition pharmaceutique ou composition nutraceutique destinée à être utilisée dans la thérapie d'un sujet mammifère, de préférence dans la prévention ou le traitement d'un dysfonctionnement de la signalisation médiée par le récepteur X de rétinoïdes (RXR) chez un sujet mammifère, ladite composition comprenant le composé défini dans l'une quelconque des revendications 1 à 4 ou le composé à utiliser tel que défini dans l'une quelconque des revendications 5 à 9 ou le 9-cis-caroténoïde selon la revendication 10, ladite composition comprenant également un ou plusieurs additif(s) et/ou excipient(s) pharmaceutiquement ou nutraceutiquement acceptable(s).

12. Composé ou composition pharmaceutique ou nutraceutique décrite selon l'une quelconque des revendications 1 à 11 pour une utilisation thérapeutique en tant que précurseur sélectif du ligand de RXR endogène dans la prévention ou le traitement d'un dysfonctionnement de la signalisation médiée par le récepteur X de rétinoïdes (RXR) chez un sujet mammifère ; de préférence pour une utilisation dans la prévention et/ou le traitement d'une maladie choisie parmi les maladies liées au système nerveux central et les maladies liées au système nerveux périphérique, ou
pour une utilisation dans la prévention thérapeutique et/ou le traitement
- des troubles de l'apprentissage et/ou de la mémoire, ladite mémoire étant de préférence la mémoire de travail,
- d'une altération des fonctions cognitives ou des troubles de l'apprentissage et de la mémoire,
- de la dépression, ou
pour une utilisation dans la prévention et/ou le traitement d'un trouble neurodégénératif, de préférence un trouble neurodégénératif choisi parmi la maladie d'Alzheimer, la maladie de Parkinson, le trouble cognitif léger (MCI), la maladie de Parkinson avec MCI, la maladie de Huntington, la démence à corps de Lewy (DCL), la sclérose latérale amyotrophique (SLA) et d'autres démences neurodégénératives dues à des changements dans le cerveau provoqués par le vieillissement, une maladie ou un traumatisme ; ou les lésions de la moelle épinière et les ataxies, la sclérose disséminée et la sclérose en plaques (SEP) ou d'autres affections neurologiques.

13. Composition nutraceutique à utiliser dans la prévention thérapeutique du dysfonctionnement de la signalisation médiée par le récepteur X de rétinoïdes (RXR) en fonction d'une carence en vitamine A5 dépendant d'un ligand chez un sujet mammifère, pour une supplémentation générale en vitamine A5, ladite composition comprenant du 9-cis-bêta,bêta-carotène (9CBC) dans une quantité ou une concentration supérieure à celle présente dans la matrice alimentaire naturelle et/ou comprenant une quantité supplémentaire du composé.

14. Utilisation non médicale d'un composé ou d'une composition pharmaceutique ou nutraceutique ainsi que décrit selon l'une quelconque des revendications 1 à 12, de préférence pour améliorer les performances de la mémoire, où de préférence ladite mémoire est la mémoire de travail, ou comme ingrédient alimentaire ou comme complément alimentaire pour maintenir la santé d'un sujet.

15. Un 9-*cis*-bêta-carotène (9CBC) ou un aliment fonctionnel contenant du 9CBC à utiliser en thérapie comme précurseur caroténoïde du 9-*cis*-13,14-dihydro-bêta-carotène (9CDHBC), comme ingrédient alimentaire ou comme nutraceutique, dans la prévention thérapeutique d'une carence en vitamine A5 dépendant d'un ligand et/ou pour une supplémentation générale en vitamine A5, ou comme complément alimentaire pour activer le RXR à un niveau physiologique pertinent de 9CDHBC et/ou de 9CDHRA dans le corps humain, où de préférence le 9CBC est utilisé sous la forme d'une composition comprenant ledit composé dans une quantité ou une concentration supérieure à celle présente dans la matrice alimentaire naturelle et/ou comprenant une quantité supplémentaire du composé.

16. Composition nutraceutique à utiliser dans la prévention thérapeutique du dysfonctionnement de la signalisation médiée par le récepteur X de rétinoïdes (RXR) dépendant d'une carence en vitamine A5 dépendant d'un ligand chez un sujet mammifère, ladite composition comprenant du 9-*cis*-bêta,bêta-carotène (9CBC) dans une quantité ou une concentration supérieure à celle présente dans la matrice alimentaire naturelle et/ou comprenant une quantité supplémentaire du composé.
